# EUROPEAN PATENT SPECIFICATION

(11) **EP 2 751 577 B1**
(45) Date of publication and mention of the grant of the patent: **17.04.2019**
(21) Application number: 12827797.7
(22) Date of filing: 28.08.2012
(51) Int. Cl.: G01N 35/08, A61B 5/00, A61B 5/145, G01N 33/66, A61B 5/1495, A61B 5/1459, A61B 5/1473

(54) **A method of manufacturing a sterilized optical analyte sensor**
Ein Verfahren zur Herstellung eines sterilisierten optischen Analytsensors
Procédé de fabrication d'un capteur optique d'analyte stérilisé

(30) Priority: 30.08.2011 US 201161528977 P
(43) Date of publication of application: 09.07.2014
(73) Proprietor: MEDTRONIC MINIMED, INC., Northridge, CA 91325-1219 (US)
(72) Inventor: NISSIMOV, Haim, Laguna Hills, CA 92653 (US); GALLANT, Stuart, L., Mission Viejo, CA 92692 (US)
(74) Representative: Madgwick, Paul Roland
(86) International application number: PCT/US2012/052631
(87) International publication number: WO 2013/033076

(56) References cited:
- WO-A1-2009/124054
- WO-A1-2009/124054
- US-A1- 2008 081 000
- US-A1- 2008 081 000
- US-A1- 2010 198 034
- US-A1- 2010 198 034
- US-A1- 2010 293 892
- US-A1- 2010 293 892
- US-A1- 2011 004 082
- US-A1- 2011 004 082
- US-B1- 6 292 697
- US-B1- 6 292 697

## Description

### BACKGROUND

Health care providers have long recognized the need to monitor patients' analyte levels, including e.g., glucose levels. Low blood glucose may lead to anxiety, weakness, and in extreme cases coma and death. Likewise, high blood glucose is associated with acidosis, glucose spilling into the urine, polyurea, hemoconcentration and related stresses on organ systems, including the renal and cardiovascular systems. Glycemic control may be particularly important in the critical care setting, where high or low blood glucose has been related to increased morbidity and mortality. The document US2011/004082 relates to a non-invasive method for measuring blood glucose using a LED source and a plurality of photo detectors arranged to measure attenuation by tissue of the subject.

Improved glycemic control requires continuous and accurate monitoring of a patient's blood glucose level. There is a need for methods of effectively manufacturing, packaging, and calibrating analyte sensors without compromising the sterility of the sensor or damaging the analyte sensors. There is also a need to be able to program or read-write information to a memory of an analyte sensor. As analyte sensors become more complicated in order to accurately monitor blood glucose level on a real-time basis, certain analyte sensors may require the programming or reading-writing of information to the memory after the analyte sensor has been processed, for example sterilization, such that it is no longer acceptable to expose the analyte sensor to the atmosphere. Further, certain sterilization processes, such as gamma irradiation or ethylene oxide, can affect certain analyte sensors that operate based on a chemical detection system, such as fluorophores, thus there is a need to compensate or correct for any affects the sterilization process has on the analyte sensor's operation.

### SUMMARY

### BRIEF DESCRIPTION OF THE DRAWINGS

**FIG. 1** illustrates the components of a continuous glucose monitoring system in accordance with one embodiment of the system.
**FIG. 2** is a schematic diagram showing RF and wire communication pathways among an external RFID programmer unit, a glucose sensor and a monitor in accordance with an embodiment of the system.
**FIG. 3** shows relative placement of the sensor plug housing in a sterilized package and an external RFID programmer unit.
**FIG. 4** is a flow chart showing a one-point calibration routine in accordance with an embodiment of the invention.
**FIG. 5** is a flow chart showing a pH calibration routine in accordance with an embodiment of the invention.
**FIGS. 6A****,** **6B****,** **6C****, and** **6D** is a flow chart showing a signal acquisition routine in accordance with an embodiment of the invention.
**FIG. 7** is a flow chart showing a pH calculation routine in accordance with an embodiment of the invention.
**FIGS. 8****,** **9****, and** **10** is a flow chart showing a calculation routine in accordance with an embodiment of the invention.
**FIG. 11** is a flow chart showing a temperature sensor control routine in accordance with an embodiment of the invention.
**FIGS. 12****,** **13****,** **14****, and** **15** is a flow chart showing an ex vivo calibration routine in accordance with an embodiment of the invention.
**FIG. 16** is a flow chart showing an in vivo state routine in accordance with an embodiment of the invention.
**FIG. 17** shows a glucose sensor having a series of holes that form a helical configuration.
**FIG. 18** shows a cross-sectional view of one embodiment of a glucose sensor having a cavity in the distal portion of the sensor and a temperature probe.
**FIG. 19** shows a cross-sectional view of another embodiment of a glucose sensor having a cavity in a distal portion of the sensor enclosed within a cage and an additional reference material.
**FIG. 20** shows a cross-sectional view of another embodiment of a glucose sensor having a cavity in a distal portion of the sensor enclosed within a cage and a reference material extending to the atraumatic tip.
**FIG. 21** shows a cross-sectional view of another embodiment of a glucose sensor having a cavity in a distal portion of the sensor enclosed within a cage and a reference material as a tab extending across the diameter of the cage.
**FIG. 22** shows a schematic view of another embodiment of a glucose sensor having a glucose sensing optical detector adjacent to a reference optical detector.
**FIG. 23** shows a glucose measurement system comprising one excitation light source, a single exciter-dual emitter fluorophore system, and a micro spectrometer and/or spectrometer.
**FIG. 24** is a flow chart of a shift detection routine in accordance with an embodiment of the invention.
**FIG. 25** is a flow chart of a trend prediction routine in accordance with an embodiment of the invention.
**FIG. 26** shows the glucose response of HPTS-CysMA/3,3'-oBBV in hydrogel.
**FIG. 27** displays four plots of fluorescent signal versus glucose concentration at four different temperatures generated by one embodiment of a measurement device disclosed herein.
**FIG. 28** displays the four plots of **FIG. 27** with each constant temperature plot normalized to the value of its fluorescent signal at 50 mg/dL.
**FIG. 29** displays essentially the same raw data as **FIG. 27****,** but instead displays four plots of fluorescent signal versus temperature at four different glucose concentrations.
**FIG. 30** displays the fluorescent signal generated by one embodiment of an analyte measuring device disclosed herein at four temperatures and four glucose concentrations.
**FIG. 31** displays a plot of fluorescent signal versus glucose concentration at four temperatures as generated by one embodiment of an analyte measuring device disclosed herein.
**FIG. 32** displays plots of the values of the three Michaelis-Menten parameters versus temperature.
**FIG. 33** displays plots of normalized values of the three Michaelis-Menten parameters versus temperature and displays a best fit line associated with each normalized parameter.
**FIG. 34** compares a plot of glucose concentration as determined by reference measurements with a plot of glucose concentration as determined by one embodiment of an analyte measuring device disclosed herein.
**FIG. 35** displays plots of the ratio of two fluorescent signals versus pH at four glucose concentrations.
**FIG. 36** displays plots of two fluorescent signals at four temperatures and four pH levels.
**FIG. 37** displays plots of two fluorescent signals at four temperatures and four pH levels.
**FIG. 38** displays plots of the ratio of two fluorescent signals versus pH at four temperatures, along with best fit lines corresponding to each temperature.
**FIG. 39** displays plots of the slopes and intercepts of the best fit lines of **FIG. 38** at each of the temperatures from that figure.
**FIG. 40** compares a plot of pH as determined by reference measurements with a plot of pH as determined by one embodiment of an analyte measuring device disclosed herein.

### DETAILED DESCRIPTION

In some embodiments, an analyte sensor is incorporated into a system whereby information (e.g., a fluorescent emission signal) about a patient's analyte concentration and/or activity can be converted to an electrical (analog or digital) signal and communicated to a user programmable (or pre-programmed) controller. The controller may comprise an integral visual and/or audio output device, such as a conventional computer monitor/display, alarm, speaker, and/or an integral or operably coupled printer. Preferably, the controller comprises a visual display that indicates the present analyte concentration (e.g., mg glucose/dL) and/or activity (e.g., millimoles glucose/kg water or mmol/L), the trend (steady, rising or falling), and the relative rate of change (e.g. mg/dL/min); in some embodiments, the user can select the desired output format and units.

The trending can be displayed, for example, as a percentage change or a range. In one embodiment, the controller displays a relative glucose rate of change, and does not measure an absolute glucose level. The trend can be displayed as a relative sensor signal change from the previous signal or several of the previous signals. The trend can be displayed as a graphical view of the glucose or signal level changing over time, or over a separate parameter, such as temperature or pH, or a combination thereof. The trend can be displayed as a relative change in the glucose concentration compared to a reference measurement that is one measurement interval prior, or in some embodiments multiple intervals prior to the latest measurement, or in some embodiments the reference measurement may be an average concentration over a preselected number of prior measurement intervals.

In some embodiments, where controller output of conventional blood glucose concentration (i.e., mg/dL) is desired, the glucose activity may be converted to blood glucose concentration (as long as the hematocrit (HCT) is known) by the equation: $glucose concentration = glucose actual \times \left(HCT\times .7029\right) + \left[\left(1-HCT\right)\times 0.939394\right]$

Thus, in some embodiments, the controller may comprise the necessary algorithms and program instructions to convert direct measurements of glucose activity, along with HCT input, into conventional glucose concentration values, and further comprise a user selectable switch or other actuator to allow toggling between the measured glucose activity and the calculated glucose concentration values (See e.g., co-pending PCT application PCT/US2010/061163 entitled: Glucose Sensor And Controller With User Selectable Output, filed on Dec 17, 2010).

Controller electronics are well known in the art. In one embodiment, the controller may comprise electronics and receiver/display unit configurations such as those described in US Patent Publication. No. 2009/0188054.

The controller may also be in communication with other devices, including e.g., data processing device(s), storage devices, and/or wired and wireless networks; in some embodiments these additional devices/functionalities may be integral with the controller while in other embodiments, these additional devices/functionalities may be remote and operably coupled to the controller. In some other embodiments, the patient's glucose activity level can be transmitted to a data network for viewing via a computer or other network interface device. Embodiments that transmit information in this manner are disclosed in U.S. Patent Numbers 6,024,699, 6,168,563, 6,645,142, 6,976,958 and 7,156,809. In yet other embodiments, information about the patient's glucose activity level can be transmitted through voice synthesizer or earcon. An example of a system that transmits information to the user via auditory output is disclosed in U.S. Patent Number 7,440,786.

In many embodiments, the controller can be configured to alert the user if the measured glucose activity level rises above or falls below a certain threshold. Examples of such devices that are capable of alerting the user of abnormal glucose levels are described in U.S. Patent Numbers 5,497,772 and 5,791,344, and US Patent Publication No. 2009/0177054. Such an alarm function may also desirably be initiated based on rates of glucose activity change, e.g., if the glucose activity is dropping rapidly, then an alarm may alert medical personnel that rapid intervention (infusion of glucose) may avert a critical condition before a programmed threshold is reached and thereby facilitate desired glycemic control. Of course, in some preferred embodiments discussed in greater detail below, the glucose activity level and/or rate of change thereof is communicated from the controller to a blood glucose modulating unit (e.g., an insulin and glucose infusion pump). Although medical practitioners do not necessarily agree at this time on the stringency of glycemic control (e.g., some studies suggest better clinical outcomes with tight glycemic control in the critically ill, whereas others suggest that moderate glycemic control may be preferred), embodiments of the invention provide systems and methods for facilitating any desired stringency of glycemic control.

Besides receiving information from the sensor, the controller preferably also controls the light interrogation of the sensor chemistry, e.g., by illuminating LED's to provide the excitation light and/or reference light. Of course other light sources, e.g., laser light, may be employed in some embodiments depending on the sensor optics and the indicator chemistry. In some preferred embodiments, the frequency of light interrogation can be controlled based on the blood glucose activity. For example, where the glucose activity level is steady, the interrogation rate may be relatively slow/infrequent, thereby conserving energy (battery life in portable controllers) and minimizing any photo-bleaching effect or photodegradation on the indicator chemistry. However, where the glucose activity level is increasing or decreasing the interrogation rate may be increased. This is especially desirable, where glucose activity is dropping and establishing real-time or near real-time glucose monitoring may have greater clinical relevance.

### Example of Controller/Monitor

In one preferred embodiment, the controller enables a number of functions to be described herein; primarily, however, its fundamental purpose is to "interrogate" (illuminate) the sensor chemistry and to respond to the resultant fluorescent signals which are proportional to the glucose concentration present, and to convert the signal into a glucose value for reporting, displaying, and trending. Preferably, it also measures the temperature of the sensor tip and uses the temperature to correct for the glucose concentration as a function of sensed temperature. Preferably, the glucose concentration is also corrected by an indirect measurement of pH which may be derived, e.g., from a ratio of the same fluorescent signals as used for the glucose concentration measurement (algorithmically as described for example in U.S. Patent No. 7,751,863).

In preferred embodiments, the controller connects to the optical sensor and makes the measurements of signal intensity and temperature to produce a glucose value based on the modulation of the fluorescent chemistry. Preferably, it includes a user interface which is comprised of a display, indicator, audible alarm, and keypad, which enables the user to input various parameters e.g., alarm limits, values for the in-vivo adjustment, set the display mode, confirmation of pre-patient insertion calibration of the sensor, etc. Likewise, in preferred embodiments, it will also produce error messages and advice the user via the display and built-in alarm when errant conditions or situations are detected.

The controller may communicate to an external device via an IrDA port or via RF communication, for the purpose of programming specific parameters prior to any use, and also for downloading data after use to the PC for further display, printing and report generation.

The major functional blocks of the controller are itemized in Table 1, although it is to be understood that these are illustrative of one preferred embodiment of a controller useful in the systems described herein. Any of the specific functional blocks may be substituted with art-recognized equivalents or alternatives. Likewise, in some embodiments of the controller, not all of these functional blocks may be included.

**TABLE 1**

| **Functional Block #** | **Description** |
|---|---|
| **1** | Microcontroller |
| **2** | 512 Kbyte Flash Memory |
| **3** | Real time clock |
| **4** | Heater controller |
| **5** | Graphical display |
| **6** | Audible alarm, buzzer |
| **7** | Optical block containing LEDs, detectors, lenses and filters |
| **8** | Temperature measurement circuit |
| **9** | User keypad |
| **10** | IrDA communications transceiver and circuitry |
| **11** | Interface to sensor ID memory component |
| **12** | Battery voltage monitor circuit |
| **13** | Batteries |
| **14** | Power supply circuitry |
| **15** | Optical and electrical cable to sensor |
| **16** | Mixed signal (optical-electrical) connector |
| **17** | Sensor containing optical fiber, thermocouple and memory |

The microcontroller is at the core of the controller. It and all other circuitry are powered by the Power Supply circuit, which obtains its input power from 4 x AA batteries or an external power source. Microcontroller monitors the Power Supply voltage via Battery Monitor Circuit.

Microcontroller controls all of the elements of the system including the user interface devices (keypad, display and the audible alarm) and provides all control for the Optical Subassembly. The Optical Subassembly includes LEDs for illuminating the Sensor and detectors for receiving the fluorescent and reference signals. The Optical Subassembly communicates with the Sensor and the Microcontroller. In a preferred embodiment, the Optical Subassembly is comprised of two LEDs of two different wavelengths, and two photodiode detectors each configured to detect at different wavelength. The LEDs may be controlled by signals from Microcontroller and an interface circuit, and via embedded software, can be set at several rates e.g., 1/min, 5/min, etc., and at any pulse width between 1 ms to 100 ms. The amplitude of the LED drive current is also adjustable by the Microcontroller up to about 50 ma from virtually 0 ma. In one embodiment, the maximum allowable amplitude of the LED drive current is between 20 and 40 ma. The number of measurements made or signals derived for each light pulse can be adjusted. In one embodiment the number of measurements made per pulse is changed by adjusting the pulse width. For example, by increasing the pulse width, more measurements can be acquired per pulse, depending upon the conversion time of the A/D converter. In one embodiment the pulse width is 10 ms, and the system is able to take 25+ measurements, which can have the advantage of smoothing the data or allowing for more statistical analysis and data processing. In other embodiments, the pulse width is 5 ms. In yet other embodiments, the pulse width is 20 to 30 ms.

Synchronous with the LED pulse, the two photodiode detectors receive the "green" and or "blue" signals; green being the result of the fluorescent response of the chemistry and blue being the "reflected" or *reference* signal. It should be noted that other sensor embodiments could utilize other referencing signals, and that "blue" is just an illustration for the current design embodiment.

The photodiode detectors are configured in a fairly typical, but highly optimized "transimpedance amplifier" configuration which converts the sensed photodiode current to a voltage. Signal currents are typically in the low picoampere range, from perhaps a few picoamps to a few hundred picoamps. The detected pulses - from each detector - are then amplified and the signals digitized via dual A/D convertors within the Dual Detector Circuit. The A/D convertors enable the voltage digital reading to be read and stored by the Microcontroller.

The Thermocouple Measurement circuitry measures the sensor temperature via thermocouple embedded within the sensor itself. The heater temperature of the separate Calibration Chamber heater is controlled by Heater Controller.

The graphical display which provides all of the controller's information including the calculated (and pH/temperature corrected) glucose value, trend information, rate of change, alarms and alerts, and in conjunction with Keypad enables the user to input and adjust parameters.

With reference to, an aspect of the optical signal path is the actual transmission of the optical signals from the monitor optical subassembly to the sensor (the efferent path) and from the sensor to the optical subassembly (the afferent path). The optical signal transmission is accomplished with the use of a bundled fiber optic cable, which contains a mix of fibers for the efferent and afferent signals. The Optical subassembly optionally comprises a lens or a filtering system. A lensing system may be used to collimate, focus, or otherwise modified to illuminate the fluorophore sensor at a desired emission pattern. Additionally the optical system may further comprise an optical element, such as an interference filter or a band pass filter, a mirror, an attenuator, an amino acid light transmitter, quartz light transmitter, or a diffuser to emit the light uniformly.

### System Overview

Various embodiments of optical systems and methods are disclosed herein for determining blood glucose concentrations. The various embodiments preferably share at least two features. First, they involve exciting a chemical indicator system with an excitation light signal and measuring the emission light signal of the indicator system, wherein the indicator system is in contact with the blood and comprises a fluorescent dye operably coupled to a glucose binding moiety-such that the emission light signal generated by the indicator system upon excitation is related to the blood glucose concentration. Second, they involve correcting the blood glucose concentration measurements from the indicator system for potential artifacts due to the optical system, which artifacts are unrelated to the blood glucose concentration. The correction is performed by ratiometric analysis. More particularly, the ratio of emission light signal to a second light signal that is propagated through the optical system, e.g., the excitation light signal or a separate reference light signal, is used for correcting any non-glucose related contributions of the optical system. Where the excitation light signal is used for the ratiometric correction, the sensor preferably includes a reflective surface, e.g., a mirror, located somewhere along the sensor, such that at least a portion of the excitation light which has passed through the optical system is reflected back to a detector. Where a separate reference light signal is used, the reference light signal may either be: (1) generated by a separate light source and reflected back to a detector, or (2) generated as a separate emission light signal from a separate dye disposed somewhere along the sensor. Thus, a glucose sensor in accordance with preferred embodiments will comprise either a reflective surface or a second dye adapted to emit a reference light signal. In one embodiment, the system comprises a second fluorescent dye, such as a red dye that is not sensitive to glucose as a reference signal.

Various structural configurations have been proposed for holding a chemical indicator system in a position, which is exposed to the blood and disposed within the excitation light path. For examples of exposing a chemical indicator system to the blood, exposing the indicator system to an excitation light signal, detecting an emission light signal from the indicator system, and enabling ratiometric correction of glucose determinations for artifacts to the system optics; see US Patent Publication No. 2008/0188725 and 2011/0105866; both of which are incorporated herein in their entireties by reference.

Optical glucose sensors, such as those described in U.S. Patent Nos. 5,512,246, 5,503,770, 6,627,177, 7,417,164 7,470,420, 7,751,863, 7,767,846, 7,824,918, 7,829,341 and 7,939,664, U.S. Patent Publication Nos. 2006/0083688, 2008/0188725, 2008/0187655, 2008/0305009, 2009/0018426, 2009/0018418, 2009/0061528, 2009/0081803, 2009/0264719, 2009/0177143, 2010/0312483, 2011/0077477, 2011/0105866, PCT Publication Nos. WO2008/001091, WO2009/019470, WO2009/087373, and WO2009/106805, co-pending U.S. Patent Appl. Nos. 12/972,385, 13/022,494 and 13/046,571, and co-pending PCT Appl. Nos. PCT/US2010/061163 and PCT/US2010/061173 typically employ a chemical indicator system disposed at the distal end of an optical fiber, wherein the indicator system is maintained in contact with the blood, such that an excitation light signal sent distally down the fiber causes the chemical indicator system to emit a light signal related to the concentration of glucose.

In certain embodiments, an optical glucose measurement system is disclosed for measuring glucose concentration in blood using one or more glucose-sensing chemical indicator systems. Such indicator systems preferably comprise a fluorophore operably coupled to a glucose binding moiety. Preferably, the glucose binding moiety acts as a quencher with respect to the fluorophore (e.g., suppresses the fluorescent emission signal of the fluorophore in response to excitation light when it associates with the fluorophore). In preferred embodiments, as the glucose binding moiety binds glucose (e.g., as glucose concentrations rise), it dissociates from the fluorophore, which then generates a fluorescent emission signal upon excitation. Accordingly, in such embodiments, the higher the glucose concentration, the more glucose bound by the binding moiety, the less quenching, and the higher the fluorescence intensity of the fluorophore upon excitation.

In certain embodiments, the optical glucose measurement system measures glucose concentrations intravascularly and in real-time through the use of such chemical indicator systems. In certain embodiments, the glucose-sensing chemical indicator systems are immobilized in a hydrogel. The hydrogel may be inserted into an optical fiber such that light may be transmitted through the hydrogel while at least a portion of the hydrogel is in contact with blood. The hydrogel is preferably permeable to blood and analytes, specifically glucose. In certain embodiments, the optical fiber together with the hydrogel comprises a glucose sensor that is placed in a mammalian (human or animal) blood vessel.

Light may be transmitted into an optical glucose sensor from a light source. In certain embodiments, the light source is a light emitting diode that emits an optical excitation signal. The optical excitation signal excites the fluorophore system(s), such that the fluorophores emit light at an emission wavelength. In certain embodiments, the fluorophore systems are configured to emit an optical emission signal at a first wavelength having an intensity related to the blood glucose concentration in the blood vessel. In certain embodiments, light is directed out of the glucose sensor such that the light is detected by at least one detector. The at least one detector preferably measures the intensity of the optical emission signal, which is related to the glucose concentration present in the blood. Various optical configurations for interrogating glucose-sensing chemical indicator systems with one or more excitation light signals and for detecting one or more emission light signals from the chemical indicator systems may be employed, see e.g., U.S. Patent No. 7,751,863, US Publication No. 2008/0188725 and US Patent Appl No. 12/612,602.

### Adjustable Excitation

It should be noted that the variable parameters at which the LEDs or laser diodes are excited (pulse rate, amplitude, and pulse width) can be adjusted in response to conditions of the patient. For example, as mentioned above, the rate could be adjusted to be more frequent at lower glucose levels, and/or when the glucose values are changing rapidly to provide a higher density of information which could be relevant for clinical decision making. Likewise, at higher glucose levels, the excitation values (pulse rate, pulse width) might be reduced in order to reduce the power consumption and extend the overall use time if needed. Other pulse characters may be adjusted, such as the wavelength or excitation spectra, or the timing and/or duration of the emission and detection, or the repetition rate of the emitters may be adjustable. In one embodiment, the repetition rate is dynamically adjusted based on a trend of a physiological character, such as the glucose, pH, or temperature. In one embodiment, the light source emits once every 10 seconds to 2 minutes when glucose levels are within a preset range, e.g., within "normal" levels. When the glucose level is below a certain threshold concentration, the flashing increases to once per second to 10 seconds. When the glucose level is above a certain threshold concentration, the flashing decreases to once every 2 minutes to 1 or more hours. In another example, the excitation values might be decreased during lower glucose levels.

In another embodiment, the light source will reduce the emission rate depending on the mode and the time of the day. In one embodiment, the monitoring system may be put into a pause or stop mode, wherein the flashing is temporarily ceased until un-paused or another initiation signal is received, for example the temperature of the patient or from an external monitoring system, such as a multi-parameter patient monitoring station. In one embodiment, the monitoring system is put into a sleep or night mode, wherein the flashing is significantly reduced or ceased during fixed periods of time where the patient is in stable condition and/or sleeping. The sleep mode can be tracked manually or automatically based on an internal clock or by a sleep detection system, such as a brain wave monitor, body temperature detector, or eye movement monitor.

In another embodiment, the rate of flashing may be adjusted automatically depending on the amount of the time the sensor has been in use. In one embodiment, the pulse rate gradually decreases as the sensor is in use longer. In one embodiment, the pulse rate decreases once it reaches a threshold use time, for example the pulse rate may change after usage for 2, 4, 10, 24, or 48 hours, or a combination thereof. In one embodiment, the pulse rate is adjusted by a combination of a gradual adjustment and threshold step adjustments. The pulse rate may also adjust according to other characteristics of the patient or blood being measured, for example, the pH, temperature, oxygen or carbon dioxide levels, or other physiological characteristic, or simply the signal level during calibration or the sensor intensity with reference to the glucose level.

The amplitude and pulse width of the light source may similarly be adjusted based on the glucose level, sensor use time, and other physiological characteristics. The adjustment may be dynamic or step-wise, or manually controlled. In one embodiment, the light source comprises several emitters, for example 2 or 3 LEDs coupled together for example by a bundled fiber optic cable, and the several emitters being operated are adjusted according to similar glucose level, sensor use time, or other physiological characteristics. In one embodiment, the monitoring system further comprises algorithms, adjustment mechanisms, and/or standard routines, such as for example, an *in vivo* calibration or *in vitro* calibration that allows recalibrating the sensors using a one or two point calibration method, which allows adjusting the sensor response based on the adjusted pulse character, emission strength, or patient character.

The ability to adjust the excitation characteristic, such as the pulse rate or amplitude of the LED or laser diode can be useful in adjusting the optical system to compensate for photodegradation or chemical oxidation, thus increasing the sensor life and/or improving the accuracy or signal-to-noise ratio. For example, the fluorescent dye used to detect the glucose concentration may degrade by time, by use, or by contamination of chemicals. In one embodiment, the excitation values are adjusted in order to compensate for photodegradation, for example the pulse amplitude is increased in order to increase the fluorescent emission signal level to meet the minimum signal-to-noise ratio. In one embodiment, the pulse amplitude and/or pulse rate is reduced to decrease the effect of photodegradation, thereby prolonging the sensor life.

In one embodiment, the sensor assembly comprises two or more fluorescent sensor portions with separate emission and detector systems. In one embodiment, the first fluorescent sensor is operating at a faster pulse rate compared to a second fluorescent sensor, thus the second fluorescent sensor may experience less photodegradation, thus prolonging the sensor use life longer by having a second sensor with less exposure. The controller may additionally compare the signal output of the first and second fluorescent sensor to detect deviation problems for accuracy or noise. A single sensor assembly comprising two or more fluorescent sensors has the benefit of being calibrated in vivo or in vitro in a single calibration process, because it uses the same sensor assembly being positioned to measure the same calibration or measurement medium, such as a patient blood.

In one embodiment, photobleaching and/or any oxidative degradation of the chemical indicator system may be reduced by incorporating an antioxidant, as described in co-pending US Application No. 13/022,494 filed February 7, 2011.

### Noise Reduction

Various other means and features may be combined to improve the signal either by reducing or preventing the noise or subtracting the noise from the signal. The noise can be introduced from electrical wiring and circuits, optical sources such as the fiber optics or optical couplers, or from chemical sources such as the fluorescent dye.

In one embodiment, the signal measurement in a light environment may be compared against a signal measurement in a dark environment. Contrasting the signal difference in the two environments can show the amount of light noise that was introduced from the surrounding environment. This may be useful, for example in determining the amount of environmental light noise during calibration of the sensor in a bright environment, which can help improve the signal response during glucose measurement in dark environment, such as the patient blood. Also, in some embodiments compensating the signal measurement in the light environment with the signal measurement in the dark environment can also compensate for an offset in the electrical circuit path.

In one embodiment, the signal level, for example the sensor fluorescence intensity corresponding to the glucose level, is compared against the prior signal or a collection of the prior signals or prior signal levels for any noise. For example, the signal levels is contrasted to the average value of the previous signal levels to determine whether it exceeds a threshold value, thereby indicating a variation in glucose levels that exceeds a normal or typical variation. The threshold value can be set in advance as a constant value or can be set dynamically and/or by the user. As the result of the above noise detection, the extracted section data containing the noise affected glucose signal is deleted, thereby removed from the adding and averaging process thereafter.

In one embodiment, the controller will track the signal during a known constant intensity phase, for example during a stable point of the calibration process wherein the calibration standard is constant and stable. In one embodiment, an optical reference signal is used, for example a reference channel separate or shared with the measuring channel that has an emitter and a detector that monitors the signal of the emitter by itself, unprocessed by the sensor. In one embodiment, a signal reference standard is used, for example a black or an opaque matter, or a material that can reflect the emission from the emitter, or a material with a known fluorescence emission character. The signal reference standard may be in the shape of the sensor, such that the controller or the cable comprising the optic fibers can couple with the signal reference standard.

During the known constant intensity phase, the controller can be configured to detect variations between the signals, for example by comparing the signal against the prior signal or a collection of the prior signal. A determination that the signal is not sufficiently stable can alert the user of any problems with the controller or the system. In one embodiment, the controller will view the sensor response intensity, such as the fluorescence emission, and determine whether there is sufficient sensor intensity in view of the glucose level, pH level, use time, *in vitro* or *in vivo* calibration conditions, or pulse characters.

The number of optical fibers that transmits the light between the emitters and chemical sensor and the number of optical fibers that transmits the light between the chemical sensor and detectors can be arranged such that it has a specific ratio. The optical fibers can be divided and mapped into a single cable in various ratios, for example, 4:1, 9:1, 19:1, or 40:1. In a preferred embodiment, the ratio is 37:1. In a more preferred embodiment, the ratio is 19:1. In one embodiment, the optical cable comprises between 15 and 50 optic fibers, each fiber being between 20-60 µm in diameter. In another embodiment, the optical cable comprises between 20 and 50 optic fibers, each fiber being between 20-50 µm in diameter. In a preferred embodiment, the optic cable comprises 37 optical fibers, each fiber being 35 µm in diameter. Optical fibers of different sizes and numbers can be utilized. In one embodiment, the fibers are coupled to a single sensor. In systems comprising multiple sensors, the fibers may be split systematically between the several sensors, for example 3 or 4 sensors. In one embodiment, the coupling of the fibers, for example a 19:1 or 37:1 ratio fiber is coupled to a single glass or plastic optical fiber. The coupling can comprise of various materials and methods known to one skilled in the art.

In one embodiment, the orientation of the fibers is such that specific fibers are mapped for the efferent blue signals and likewise a number of fibers mapped for the afferent green and blue signals. Mapping the different optical fibers can achieve signal separation. In one embodiment, this is accomplished by having the "launch" light fibers relative to detection fibers. Splitting the optical fibers between emitters and detectors and various wavelengths that are needed can improve signal-to-noise ratio by reducing the instances of optical interference, leakage, loss, or attenuation.

Systematically mapping the different fibers, a uniform excitation and emission is achieved to optimize the signals, for example by reducing signal variation and noise associated with illuminating and/or detecting non-uniform light. In other embodiments, the optical fibers are randomized. In other embodiments, an optical diffuser is used to emit a substantially uniform emission. Such optical adjustment elements can be also used in the emission from the sensor chemistry.

In one embodiment, the fibers are broken out at the proximal ends into four different ferrules, each ferrule which mates to either one of the two LEDs or one of the two detectors. In a preferred embodiment, a first LED uses 4 fibers, a second LED uses 5 fibers, whereas the signal is detected using 23 fibers coupled to a first detector and the reflected reference signal are coupled to a second detector using 5 fibers. In another preferred embodiment, the first LED emits a light with a wavelength of 470 nm and the second LED emits a light with a wavelength of 425 nm. This yields a total of 37 mapped fibers illuminating the sensor assembly. For systems employing more than two detectors and/or emitters, the number of fibers and their proportional arrangements can be adjusted. This particular fiber mapping is an example of one mapping format; other mappings may be used to match new sensor designs which require more or less light or different wavelengths of light for enhanced functionality.

### Calibration

The controller or monitor may further comprise certain calibration mechanisms, such as those described in U.S. Patent Publication No. 2010/0312483. For example, the sensors may be characterized by a modified version of the Michaelis-Menten equation from enzyme kinetics. The equation can be applied to various chemical and biological sensing systems, such as covalently or electrostatically reacting fluorophore sensors, enzymetic fluorophore sensors, or other polymer or macromolecule systems. Systems which follow the reaction mechanism described by Scheme I conform to what is commonly referred to as Michaelis-Menten kinetics and may be described by what is commonly referred to as the Michaelis-Menten equation. *See* Conners, K.A. *Binding Constants:* The Measurement of Molecular Complex Stability, John Wiley & Sons, Inc., New York, 1987.

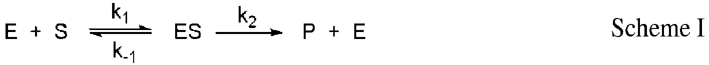

Certain embodiments of the analyte sensors disclosed herein, and in particular the glucose sensors disclosed herein, require calibration before they can be properly used to generate meaningful readings of analyte concentration. A calibration equation is useful to establish the mathematical relationship between the measured fluorescent intensity and the analyte concentration being estimated. For example, for the case of a glucose sensor, once a calibration equation has been determined, it may be used to measure a glucose concentration from a measured fluorescent intensity.

In particular, for the case of a glucose sensor chemistry, the fluorescent response (I) of the chemistry to glucose (G) can be described by a modified form of the Michaelis-Menten equation in which three parameters (a, b, and c) are determined: $I = a + b * G / \left(c+G\right)$
where "a" is the fluorescent signal intensity in the absence of glucose, "b" determines the asymptotic intensity at infinite glucose (minus the signal at zero concentration, "a"), and "c" gives the glucose concentration at which the intensity is one-half the difference between the asymptotic value and the background, i.e. a + b/2. The "c" parameter is thus analogous to the Michaelis-Menten constant, Km, in enzyme kinetic systems.

It should be noted that for certain embodiments of the glucose sensing chemistry, as with some other chemical systems conforming to Michaelis-Menten kinetics, the value of the Michaelis-Menten constant, *K*ₘ, or the "c" parameter is a substantially fixed property of the chemistry. In one embodiment, the "c" parameter is determined by the binding constant between glucose and the Receptor-Quencher.

In certain such methods, the Michaelis-Menten parameters are determined in reference to an analyte sensors based on lifetime chemistry. As in many chemical systems, the response of the sensor chemistry can be sensitive to other chemical parameters in addition to the analyte of interest.

In one embodiment, the Michaelis-Menten parameters are determined from a set of *in vitro* measurements of the fluorescent intensity using one or more solutions of known glucose concentrations. In certain such methods, it is advantageous that the solutions be held at or near physiological pH and temperature (7.4 and 37 degree centigrade, respectively), or calibrate the sensor with reference to the pH and temperature measurements. In one embodiment, the sensor is inserted into a test chamber and exposed to one glucose concentrations. The resulting data can be used to analytically determine the corresponding Michaelis-Menten parameters. Alternatively or in addition, the corresponding Michaelis-Menten parameters may be determined numerically from the resulting data using the method of least squares. This equation thus converts a measured fluorescent intensity, I, into a glucose value, G. It can be used in both *in vitro* and *in vivo* applications under the assumption that the fluorescent intensity does not change substantially (for a given glucose concentration) between the determination of the Michaelis-Menten parameters and the experimental application.

In one embodiment, an additional calibration step is used to compensate for changes in the fluorescent intensity that occur between the determination of the Michaelis-Menten parameters and the use of the sensor in an *in vitro* or *in vivo* application. This calibration could, in principle, be done at the bedside of a patient immediately before insertion of the sensor into a vein or artery. A temperature control feedback loop can be used to maintain the calibration solutions at an equivalent stable physiological temperature for the duration of the calibration procedure.

In another embodiment, the initial values of the Michaelis-Menten equation parameters are determined by the manufacturer or vendor, for example in a laboratory at the factory during manufacturing or prior to packaging, instead of by the end user or consumer, for example by a clinician at the patient's bedside. In this case, the Michaelis-Menten parameters can be determined from a multipoint *in situ* calibration in which, again, both the pH and temperature are carefully controlled. The initial determination of the Michaelis-Menten parameter values can thus be regarded as a factory calibration.

In some embodiments, Michaelis-Menten parameters determined for one manufactured sensor can be successfully used with another manufactured sensor to provide sufficiently accurate results. Accordingly, it may be efficient, cost effective, and sufficiently accurate to employ one set of Michaelis-Menten parameters for both sensors. Alternatively, a set of Michaelis-Menten parameters for an entire manufactured batch of sensors may be determined by calibrating a select few of the sensors, and averaging the resulting values to obtain a set of Michaelis-Menten parameters for the batch.

The monitor can utilize one or more values of the calibration parameters, including the Michaelis-Menten parameters (a, b and c), and the correction factors C_{A} and C_{F}, as described in U.S. Patent Publication Nos. 2010/0312483. In some embodiments, values of calibration parameters can be preloaded into the monitor, such as by storing one or more value in memory, whether by the user or another party. In some embodiments, one or more values of the calibration parameters can be entered after following a calibration procedure, either with the analyte sensor functionally connected to the monitor or with the analyte sensor connected to a different monitor or reading device. In some embodiments, when a different monitor or reading device is used for calibration, information relating to the calibration can be communicated directly or indirectly between the monitor and the different monitor or reading device functionally connected to the analyte sensor during calibration. In some embodiments, the monitor will receive information relating to the measurement of analyte concentrations as determined with a different device or by a different technique, and use the information during calibration. In some instances, the information can be sent to the monitor with manual entry, such as by keyboard or touchscreen or other manual methods; or by direct or indirect communication with a separate device determining the analyte concentration; or by reading values from an information storage medium such as scanning written or printed information, scanning barcodes, reading magnetic, optical, or computer storage medium including disks, strips, RAM, flash drives, etc.

In some embodiments, the monitoring system can be integrated into a network including other devices such as additional monitors, displays including remote displays, televisions, data entry locations, computers, PDAs, telephones, monitoring stations, doctor offices, hospitals, etc. Networking can be via the Internet, local area network, wide-area network, secure network, private network, wireless networds, etc.

Embodiments of the analyte measuring devices disclosed herein may configure light sources, detectors, and one or more sensors in a variety of ways, particularly when the analyte measuring device includes an indicator system utilizing multiple fluorophores. Various analyte sensors disclosed herein are configured to provide improved estimates of the analyte concentration of a particular solution by taking the temperature, pH, or other parameter of the particular solution into account. Accordingly, some embodiments of the analyte sensors disclosed herein may include a temperature sensing element configured to generate a signal indicative of a temperature of the sample.

### Methods of Estimating Analyte Concentration Incorporating Temperature Correction

Some embodiments of the devices disclosed herein generate a signal indicative of analyte concentration which exhibits a temperature dependence. For example, if two solutions of precisely the same analyte concentration are measured at two different temperatures with the same measurement device, in some embodiments, the measurement device may generate differing signals indicative of the two analyte concentrations. Thus, the accuracy of determining a solution's true analyte concentration based on such as signal may be improved by taking the temperature of the solution into account.

It has been discovered that for some embodiments of the measurement devices disclosed herein, and in particular, for glucose measurement devices employing a quencher binding moiety operably coupled to a fluorophore, the temperature dependence of the fluorescent signal approximately follows a modified version of the classic Michaelis-Menten equation from enzyme kinetics: $\left[Glu\right] = \frac{{c}_{T} ∗ \left[{G}_{i}-{a}_{T}\right]}{{a}_{T} + {b}_{T} - {G}_{i}}$ where
*[Glu]* is the calculated glucose concentration, at temperature T,
*a_{T}* is the first Michaelis-Menten parameter "*a*", at temperature T,
*b_{T}* is the second Michaelis-Menten parameter "*b*", at the same temperature T,
*c_{T}* is the third Michaelis-Menten parameter "*c*", at the same temperature T, and
*Gᵢ* is the fluorescent signal (*i* = 1,2), either referenced or unreferenced, where *G*₁ is the fluorescence emission at 550 nm or 583 nm when the fluorophore is excited at 470 nm (which is the absorption maximum of the fluorophore's base-form), and *G*₂ is the fluorescence emission at 550 nm or 583 nm when the fluorophore is excited at 420 nm or 430 nm (which is the absorption maximum of the fluorophore's acid-form). Note, however, that other combinations of excitation and emission wavelengths are also feasible for use in Equation 1. In the Examples below, *G*₂ has been used, unless indicated otherwise.

Various embodiments of the measurement devices disclosed herein employ a quencher-fluorophore indicator system which measures analyte concentration through the establishment of an equilibrium between the analyte of interest, the binding moiety (e.g. quencher), and the fluorophore. In such a system, analyte concentration is not measured by enzymatic consumption or conversion of the analyte. In contrast, the classic Michaelis-Menten equation specifically describes enzyme kinetics, a non-equilibrium phenomena involving the consumption/conversion of the enzyme's substrate by the enzyme. Therefore, it is not to be expected, indeed it is surprising, that an equation closely related to the classic Michaelis-Menten equation would effectively describe the temperature dependence of these types of quencher-fluorophore-based measurement devices and analyte sensing elements (or other measurement devices and analyte sensing elements functioning through analogous equilibrium mechanisms). In any event, knowledge that these devices (and similar devices) exhibit a temperature dependence which follows a modified Michaelis-Menten equation allows the use of temperature correction methods and algorithms to improve the accuracy of analyte concentration measurements. Such methods and algorithms are disclosed herein, along with measurement devices which implement such methods and algorithms.

Accordingly, some embodiment methods of estimating an analyte concentration include generating a signal indicative of analyte concentration and a signal indicative of temperature. Since, in some embodiments, the signal indicative of analyte concentration exhibits the temperature dependence just described, in some embodiments, the signal indicative of temperature may be used to adjust the signal indicative of analyte concentration to correct for temperature dependence. Thus, in certain such embodiments, the methods further include transforming the signal indicative of the analyte concentration utilizing an equation of the form of a modified Michaelis-Menten equation, such as Equation 1 above, depending on Michaelis-Menten parameters, such as the parameters "*a*", "*b*", and "*c*", as described above with reference to Equation 1.

The temperature dependence of Equation 1 is exhibited through the Michaelis-Menten parameters *a_{T}, b_{T},* and *c_{T},* as indicated by the subscript "*T*" labeling these parameters. In some embodiments, the temperature dependence may need to be determined through a temperature calibration. Thus, in certain embodiment methods, the values of one or more of the Michaelis-Menten parameters may be set based on data which includes temperature calibration data and the signal indicative of a temperature.

For example, in some embodiment methods, the temperature calibration data may be generated by a temperature calibration method. The temperature calibration method may include selecting a first test analyte sensing element, and creating and/or providing a set of at least three solutions of differing known analyte concentrations. In certain such embodiments, a first temperature is selected (*T1*), three solutions of the set of at least three solutions are heated and/or cooled to a temperature substantially similar to the selected first temperature, and a first set of at least three signals is generated using the first test analyte sensing element, each signal indicative of the concentration of analyte in a different one of the three solutions at the first temperature. Measurements are then made at a second temperature. Thus, in certain embodiments, a second temperature is selected (*T2*), three solutions of the set of at least three solutions (each of the three may be the same or different than a solution chosen for the first temperature) are heated and/or cooled to a temperature substantially similar to the selected second temperature, and a second set of at least three signals is generated using the first test analyte sensing element, each signal indicative of the concentration of analyte in a different one of the three solutions at the second temperature. Of course, more than three solutions may be used in either of these steps. And more than two temperatures may also be employed. Generally, the more solutions of differing concentration and the greater number of different temperatures that are employed, the greater the accuracy of the resulting calibration data.

Once the solutions having known analyte concentrations have been measured, and the first and second sets of at least three signals have been generated, in some embodiments, the sets of signals are used to determine (usually approximately) the relationship between one or more of the Michaelis-Menten parameters and temperature. For example, in some embodiments, the temperature calibration method may further include computing values of each of a first, second, and third Michaelis-Menten parameter at the first temperature (*a_{T1}, b_{T1},* and *c_{T1}*) by an algorithm comprising fitting a modified Michaelis-Menten equation to a first fit dataset comprising the first set of at least three signals. In certain such embodiments, the temperature calibration method may further include computing values of each of a first, second, and third Michaelis-Menten parameter at the second temperature (*a_{T2}, b_{T2},* and *c_{T2}*) by an algorithm comprising fitting a modified Michaelis-Menten equation to a second fit dataset comprising the second set of at least three signals. Thus, in methods such as these, each of the three Michaelis-Menten parameters has been determined at least two temperatures, providing data which may be used to create a model of the temperature dependence of each of the three Michaelis-Menten parameters.

To model the temperature dependence of the Michaelis-Menten parameters, in some embodiments, the temperature calibration method may further include selecting an equation relating the first Michaelis-Menten parameter (*a_{T}*) to temperature, the equation depending on a first set of temperature calibration parameters; and setting a value for each calibration parameter of the first set of calibration parameters based on the value of the first Michaelis-Menten parameter at the first temperature (*a_{T1}*) and the value of the first Michaelis-Menten parameter at the second temperature (*a_{T2}*)*.* In some embodiments, similar steps are performed with respect to the second and third Michaelis-Menten parameters (*b_{T}* and *c_{T}*). Thus, for example, the temperature calibration method may further include selecting an equation relating the second Michaelis-Menten parameter (*b_{T}*) to temperature, the equation depending on a second set of temperature calibration parameters; and setting a value for each calibration parameter of the second set of calibration parameters based on the value of the second Michaelis-Menten parameter at the first temperature (*b_{T1}*) and the value of the second Michaelis-Menten parameter at the second temperature (*b_{T2}*). Similarly, in some embodiments, the temperature calibration method may further include selecting an equation relating the third Michaelis-Menten parameter (*c_{T}*) to temperature, the equation depending on a third set of temperature calibration parameters; and setting a value for each calibration parameter of the third set of calibration parameters based on the value of the third Michaelis-Menten parameter at the first temperature (*c_{T1}*) and the value of the third Michaelis-Menten parameter at the second temperature (*c_{T2}*)*.*

Furthermore, in some embodiments, equations linear in temperature may be selected to relate the first, second, and third Michaelis-Menten parameters to temperature. For instance, in some embodiments, the first, second, and third Michaelis-Menten parameters may be written as $\begin{matrix}{a}_{T} = {a}_{37} ∗ {τ}_{{a}_{T}} \left(T\right) , \\ {b}_{T} = {b}_{37} ∗ {τ}_{{b}_{T}} \left(T\right) , and \\ {c}_{T} = {c}_{37} ∗ {τ}_{{c}_{T}} \left(T\right)\end{matrix}$ where *τ_{a_{T}}*(*T*), *τ_{b_{T}}*(*T*), and *τ_{c_{T}}*(*T*) are "temperature correction factors" which approximately account for the temperature dependence of *a_{T}, b_{T},* and *c_{T}.* When the relationship between Michaelis-Menten parameter and temperature is written as such, each Michaelis-Menten parameter *a_{T}, b_{T},* and *c_{T},* is determined by multiplying the *37 °C* Michaelis-Menten parameter *a₃₇, b₃₇,* and *c₃₇,* by its corresponding "temperature correction factor," *τ_{a_{T}}*(*T*), *τ_{b_{T}}*(*T*), or *τ_{c_{T}}*(*T*), respectively. The *37 °C* Michaelis-Menten parameters may be determined by fitting a modified Michaelis-Menten equation to a set of signals indicative of the analyte concentration of a plurality of solutions of differing analyte concentrations held at a temperature of *37 °C,* as described above with respect to, for example, *T1* and *T2.* Alternatively, the parameters *a₃₇, b₃₇,* and *c₃₇* may be supplied by a factory calibration as described in provisional U.S. Patent Publication No. 2010/0312483. As yet another alternative, *a₃₇, b₃₇,* and *c₃₇* may be determined via a one-point in vivo calibration as also disclosed in the same application.

To determine the "temperature correction factors," *τ_{a_{T}}*(*T*), *τ_{b_{T}}*(*T*), and *τ_{c_{T}}*(*T*), some embodiment methods may employ a linear approximation. For instance, the temperature correction factors may be written as $\begin{matrix}{τ}_{{a}_{T}} \left(T\right) = {m}_{{a}_{T}} ∗ T + {β}_{{a}_{T}} , \\ {τ}_{{b}_{T}} \left(T\right) = {m}_{{b}_{T}} ∗ T + {β}_{{b}_{T}} , and \\ {τ}_{{c}_{T}} \left(T\right) = {m}_{{c}_{T}} ∗ T + {β}_{{c}_{T}} ,\end{matrix}$ where the slopes, *m_{a_{T}}*, *m_{b_{T}}*, *m_{c_{T}}*, and intercepts, *β_{a_{T}}*, *β_{b_{T}}*, *β_{c_{T}}*, are collectively referred to as "temperature calibration coefficients" ("TempCos").

In some embodiments, a temperature calibration method used to determine values of these TempCos may require that values of the Michaelis-Menten parameters be determined at a second temperature (*T2*), different than *37 °C.* Values of the parameters at the second temperature (*a_{T2}*, *b_{T2}*, and *c_{T2}*) may be determined by fitting a modified Michaelis-Menten equation to a set of signals indicative of the analyte concentration of a plurality of solutions of differing analyte concentrations held at the second temperature, as described above with respect to, for example, *T1* and *T2.* Once this is done, the temperature calibration coefficients *mₐ* and *bₐ* may be determined by normalizing to *a₃₇* both *a_{T2}.* and *a₃₇,* yielding *a_{T2}*/ *a₃₇* and *1*, and fitting a line to the normalized values versus the two temperatures, *T2* and *37 °C.* The fit may be determined using linear least squares or any other method of fitting a line to a set of points. The temperature calibration coefficient *m_{a_{T}}* is set equal to the slope of the resulting line and the temperature calibration coefficient *β_{a_{T}}* is set equal to the intercept. The other temperature calibration coefficients, *m_{b_{T}}* and *β_{b_{T}}*, may be determined similarly from values of *b_{T2}* and *b₃₇,* and *m_{c_{T}}* and *β_{c_{T}}* may be determined from values of *c_{T2}* and *c₃₇.* Once the calibration is complete, a temperature corrected estimated glucose concentration ([*Glu*]) may be computed from a fluorescent signal (*Gᵢ*) measured at temperature (*T*), by using the TempCos (*m_{a_{T}}*, *β_{a_{T}}*, *m_{b_{T}}*, *β_{b_{T}}*, *m_{c_{T}}*, and *β_{c_{T}}*), the *37 °C* Michaelis-Menten parameters *37 °C* (*a₃₇, b₃₇,* and *c₃₇*), and the temperature (*T*) in Equations 2 and 3 to compute *a_{T}, b_{T},* and *c_{T},* and then plugging *a_{T}, b_{T}, c_{T}* and the measured fluorescent signal (*Gᵢ*) into Equation 1.

Thus, in some embodiments the first, second, and third sets of temperature calibration parameters may include a slope and an intercept relating temperature to the value of either the first, second, or third Michaelis-Menten parameter. However, equations of other forms may be selected to relate the first, second, or third Michaelis-Menten equation to temperature. In some embodiments, a quadratic or higher-order polynomial in temperature may be suitable and/or desirable.

When measurement devices are mass produced, it may not be feasible or practical to individually calibrate each measurement device-i.e. use each individual measurement device to generate individual calibration data. It may be more cost effective to select one or more test devices from a batch of mass produced devices, generate calibration data using the one or more test devices, and provide that calibration data to each individual devices produced in the batch. In some embodiments, variability between measurement devices from the same production batch may be, to a large extent, attributable to a particular part of the measurement device. In particular, variability between devices may be attributable to the part of the measurement device which generates a signal indicative of analyte concentration-e.g. the analyte sensing element- and/or the part of the measurement device that generates a signal indicative of temperature-e.g. the temperature sensing element. In these circumstances, as well as others, it may be advantageous to use a calibration method employing multiple test measurement devices, and/or multiple test sensing elements, because calibration over multiple test devices and/or sensing elements may yield more accurate calibration data than calibration methods which only utilize a single test device and/or sensing element. Accordingly, in some embodiments, the calibration method may further include selecting a second test analyte sensing element; generating a third set of at least three signals using the second test analyte sensing element, each signal indicative of the concentration of analyte in a different solution of known analyte concentration at the first temperature (*T1*); and generating a fourth set of at least three signals using the second test analyte sensing element, each signal indicative of the concentration of analyte in a different solution of known analyte concentration at the second temperature (*T2*). Obviously, calibration methods may similarly employ more than two test devices, or more particularly, for instance, more than two test analyte sensing elements.

In a manner similar to methods utilizing a single test analyte sensing element, after the solutions having known analyte concentrations have been measured and the first, second, third, and fourth sets of at least three signals have been generated, in some embodiments, the sets of signals are used to determine (usually approximately) the relationship between one or more of the Michaelis-Menten parameters and temperature. For example, in some embodiments, the temperature calibration method may further include (1) computing values of each of a first, second, and third Michaelis-Menten parameter at the first temperature (*a_{T1}, b_{T1},* and *c_{T1}*) by an algorithm comprising fitting a modified Michaelis-Menten equation to a first fit dataset comprising both the first set of at least three signals (which was generated with the first test analyte sensing element at *T1*) and the third set of at least three signals (which was generated with the second test analyte sensing element at *T1*); and (2) computing values of each of a first, second, and third Michaelis-Menten parameter at the second temperature (*a_{T2}, b_{T2},* and *c_{T2}*) by an algorithm comprising fitting a modified Michaelis-Menten equation to a second fit dataset comprising both the second set of at least three signals (which was generated with the first test analyte sensing element at *T2*) and fourth set of at least three signals (which was generated with the second test analyte sensing element at *T2*). Essentially, in these types of methods, the signals generated with the second test analyte sensing element are used in a combined fit with the signals generated with the first test analyte sensing element, which results in values for each of the first, second, and third Michaelis-Menten parameters which take both test analyte sensing elements into account. Alternatively, in some embodiments, a temperature calibration method may take both test analyte sensing elements into account by fitting the signals generated from each test analyte sensing element separately, and then averaging the results to obtain better estimates of the Michaelis-Menten parameters. Thus, in some embodiments, the step of computing values of each of a first, second, and third Michaelis-Menten parameter at the first temperature (*a_{T1}, b_{T1},* and *c_{T1}*) by an algorithm may further include fitting a modified Michaelis-Menten equation to a third fit dataset comprising the third set of at least three signals, and averaging the results of fitting the third fit dataset with the results of fitting the first fit dataset. In addition, the step of computing values of each of a first, second, and third Michaelis-Menten parameter at the second temperature (*a_{T2}, b_{T2},* and *c_{T2}*) by an algorithm may further include fitting a modified Michaelis-Menten equation to a fourth fit dataset comprising the fourth set of at least three signals, and averaging the results of fitting the fourth fit dataset with the results of fitting the second fit dataset.

Other methods for estimating analyte concentration which incorporate temperature correction features and temperature calibration steps are also disclosed herein. In some embodiments, these methods are similar to those already described above and incorporate similar features, however, additional features may also be disclosed and, in some embodiments, the disclosed methods may be more general and described in more general terms. Since there are many ways to feasibly implement the discoveries disclosed herein for use in estimating analyte concentration, the following additional methods are described in order to illustrate the breadth of implementations that are possible.

In some embodiments, for instance, a method of estimating an analyte concentration from a signal indicative of the analyte concentration may include transforming the signal using an equation of the form of a modified Michaelis-Menten equation wherein the values of one or more Michaelis-Menten parameters have been adjusted for temperature.

In some embodiments, for instance, a method of estimating an analyte concentration may include generating a signal indicative of the analyte concentration and generating a signal indicative of a temperature, and transforming the signal indicative of the analyte concentration utilizing an equation of the form of a modified Michaelis-Menten equation wherein at least one of the Michaelis-Menten parameters has been substituted with a calibration equation functionally depending on a set of one or more temperature calibration parameters and the signal indicative of temperature. One could refer to such an equation as a "substituted" modified Michaelis-Menten equation since the Michaelis-Menten parameters have been explicitly substituted with equations depending on one or more other variables-temperature and the temperature calibration parameters. However, although such a "substituted" equation exhibits a more complicated analytic form, it nevertheless will still express the basic functional relationships of the modified Michaelis-Menten equation.

In some embodiments, the step of transforming the signal indicative of analyte concentration may utilize a "substituted" modified Michaelis-Menten equation in which each of the first, second, and third Michaelis-Menten parameters have been substituted with first, second, and third calibration equations (respectively), each of the equations depending on sets of first, second, and third temperature calibration parameters (respectively), and each also depending on the signal indicative of temperature. In certain embodiments, at least one of the first, second, and third calibration equations is a polynomial in the signal indicative of temperature. In certain such embodiments, each of the first, second, and third calibration equations is a polynomial in the signal indicative of temperature. In certain embodiments, at least one of the first, second, and third calibration equations is a linear equation in the signal indicative of temperature. In certain such embodiments, each of the first, second, and third calibration equations is a linear equation in the signal indicative of temperature. Thus, for example, if each Michaelis-Menten parameter of Equation 1 above is assumed to exhibit a linear relationship with temperature, then the "substituted" modified Michaelis-Menten equation might appear as $\left[Glu\right] = \frac{\left({χ}_{{c}_{T} ,1}⋅T+{χ}_{{c}_{T} ,0}\right) ∗ ⌊ {G}_{i} - \left({χ}_{{a}_{T} ,1}⋅T+{χ}_{{a}_{T} ,0}\right) ⌋}{\left({χ}_{{a}_{T} ,1}⋅T+{χ}_{{a}_{T} ,0}\right) + \left({χ}_{{b}_{T} ,1}⋅T+{χ}_{{b}_{T} ,0}\right) - {G}_{i}}$ and, similarly, if each Michaelis-Menten parameter is assumed to exhibit a quadratic relationship with temperature then the "substituted" modified Michaelis-Menten equation might appear as $\left[Glu\right] = \frac{\left({χ}_{{c}_{T} ,2}⋅{T}^{2}+{χ}_{{c}_{T} ,1}⋅T+{χ}_{{c}_{T} ,0}\right) ∗ ⌊ {G}_{i} - \left({χ}_{{a}_{T} ,2}⋅{T}^{2}+{χ}_{{a}_{T} ,1}⋅T+{χ}_{{a}_{T} ,0}\right) ⌋}{\left({χ}_{{a}_{T} ,2}⋅{T}^{2}+{χ}_{{a}_{T} ,1}⋅T+{χ}_{{a}_{T} ,0}\right) + \left({χ}_{{b}_{T} ,2}⋅{T}^{2}+{χ}_{{b}_{T} ,1}⋅T+{χ}_{{b}_{T} ,0}\right) - {G}_{i}}$ where:
[*Glu*] is the estimated glucose concentration,
*χ*_{*a_{T}*,2}, *χ*_{*a_{T}*,1}, and *χ*_{*a_{T}*,0} are polynomial coefficients parameterizing *a_{T}*'s dependence on the temperature *T,*
*χ*_{*b_{T}*,2}, *χ*_{*b_{T}*,1}, and *χ*_{*b_{T}*,0} are polynomial coefficients parameterizing *b_{T}*'s dependence on the temperature *T,*
*χ*_{*c_{T}*,2}, *χ*_{*c_{T}*,1}, and *χ*_{*c_{T}*,0} are polynomial coefficients parameterizing *c_{T}*'s dependence on the temperature *T*, and
*Gᵢ* is the fluorescent signal (*i* = 1,2), either referenced or unreferenced, where *G*₁ is the fluorescence emission at 550 nm or 583 nm when the fluorophore is excited at 470 nm (which is the absorption maximum of the fluorophore's base-form), and *G*₂ is the fluorescence emission at 550 nm or 583 nm when the fluorophore is excited at 420 nm or 430 nm (which is the absorption maximum of the fluorophore's acid-form). Note, however, that other combinations of excitation and emission wavelengths are also feasible for use in Equations 4 and 5.

As stated above, although, the "substituted" equations (Equations 4 and 5) exhibit a more complicated analytic form, they nevertheless still exhibit the basic functional relationships of the modified Michaelis-Menten equation (Equation 1). In other embodiments, the calibration equations substituted into the modified Michaelis-Menten equation may have a functional form other than a polynomial in temperature.

Thus, as described above, the calibration equations substituted into the modified Michaelis-Menten equation for the Michaelis-Menten parameters may take a variety of functional forms and each may have varying numbers of temperature calibration parameters. Obviously, more complicated equations may have a greater numbers of temperature calibration parameters. In any event, depending on the embodiment, various temperature calibration methods may be used to determine the values of the first, second, and third sets of the one or more temperature calibration parameters. In certain such embodiments, each set of temperature calibration parameters may be determined by fitting the "substituted" modified Michaelis-Menten equation to a plurality of signals, the plurality of signals indicative of analyte concentration in a plurality of solutions at a plurality of temperatures. Once values of the various temperature calibration parameters are determined, temperature corrected estimates of analyte concentrations may be generated from signals indicative of analyte concentration and temperature.

### Example 1

This example concerns the temperature calibration of an equilibrium fluorescence glucose sensor, referred to herein as a GluCath sensor, employing an HPTS-Cys-MA dye operably coupled to a 3,3'-oBBV quencher. The dye and quencher are immobilized within a hydrogel disposed along the distal region of an optical fiber, while the proximal end of the optical fiber is coupled to a light source. The temperature dependence of this sensor's fluorescence response to glucose was assumed to be described by the modified Michaelis-Menten equation of Equation 1 as described above. The Michaelis-Menten parameters were assumed to bear a linear relationship to temperature as set forth in Equations 2 and 3 above. Using this model of the glucose sensor's temperature dependence, the TempCos (*m_{a_{T}}*, *β_{a_{T}}*, *m_{b_{T}}*, *β_{b_{T}}*, *m_{c_{T}}*, and *β_{c_{T}}*) were determined by the methodology described above in reference to Equations 2 and 3. Specifically, the effect of temperature on the fluorescent signal was determined experimentally by measuring the signal at four temperatures (*15 °C, 25 °C, 37 °C,* and *45 °C*) and four glucose concentrations *(50 mg*/*dL, 100 mg*/*dL, 200 mg*/*dL,* and *400 mg*/*dL*). At each temperature and glucose level the stable signal, *G*₂, was recorded.

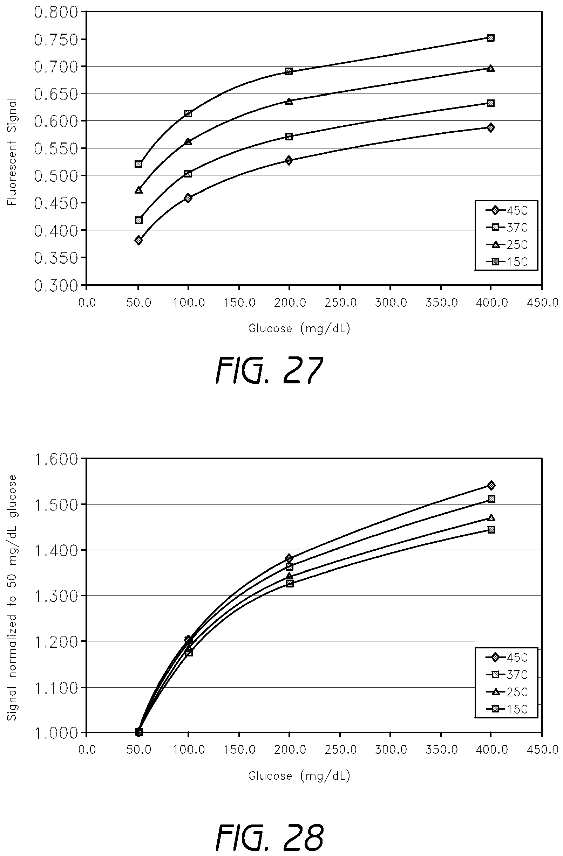

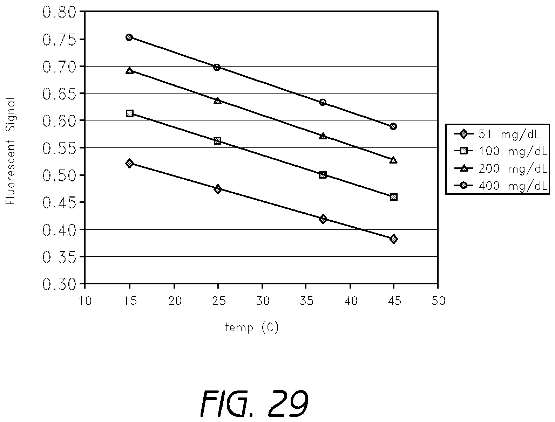

These data are displayed in **FIGS. 27-29****.** **FIG. 27** displays four plots of fluorescent signal versus glucose concentration-one plot for each of these four temperatures. **FIG. 28** displays the same data with each constant temperature plot normalized to the value of its fluorescent signal at *50 mg*/*dL.* **FIG. 29** displays essentially the same raw data as **FIG. 27****,** but instead displays four plots of fluorescent signal versus temperature-one plot for each of the four glucose concentrations. Apparent from **FIG. 29****,** is that the fluorescent signal's temperature dependence-at constant glucose concentration-is approximately linear.

Using the data plotted in **FIG. 27****,** values of the Michaelis-Menten parameters at *15 °C, a₁₅, b₁₅,* and *c₁₅,* were determined by fitting (using linear least squares) the modified Michaelis-Menten equation of Equation 1 to the fluorescence data generated at *15 °C.* Similarly, values of *a₂₅*, *b₂₅*, and c₂₅ were determined by fitting Equation 1 to the fluorescence data generated at *25 °C*; values of *a₃₇, b₃₇,* and c₃₇ were determined by fitting Equation 1 to the fluorescence data generated at *37 °C*; and finally, values of *a₄₅*, *b₄₅*, and c₄₅ were determined by fitting Equation 1 to the fluorescence data generated at *45 °C.*

The process was repeated over four additional glucose sensors of the same design as the first to improve the accuracy of the calibration. Thus, fluorescent signals were generated with each of the four additional glucose sensors, at each of the same four temperatures (*15 °C, 25 °C, 37 °C,* and *45 °C*), and at each of the same four glucose concentrations *(50 mg*/*dL, 100 mg*/*dL, 200 mg*/*dL,* and *400 mg*/*dL).* This data corresponding to each of the four sensors was fit with Equation 1 (as was done with the initial sensor) in order to generate values of *a₁₅, a₂₅, a₃₇, a₄₅*, *b₁₅, b₂₅, b₃₇, b₄₅, c₁₅, c₂₅, c₃₇,* and *c₄₅* for each additional glucose sensor. The data was averaged over all five sensors for each of these quantities to generate *a̅*₁₅, *a̅*₂₅, *a̅*₃₇, *a̅*₄₅, *b̅*₁₅, *b̅*₂₅, *b̅*₃₇, *b̅*₄₅, *c̅*₁₅, *c̅*₂₅, *c̅*₃₇, and *c̅*₄₅.

Determination of the temperature calibration parameters (TempCos) corresponding to Equations 2 and 3 was done using these averaged values. Thus, the temperature calibration parameters corresponding to the "a" Michaelis-Menten parameter, i.e. *m_{a_{T}}* and *β_{a_{T}}*, were determined by normalizing each of the "*a*" parameters to *a̅*₃₇ and fitting a line (again using linear least squares) to a plot of these values, i.e. *a̅*₁₅/*a̅*₃₇, *a̅*₂₅/*a̅*₃₇, 1, *a̅*₄₅/*a̅*₃₇ versus temperature, the slope and intercept being *m_{a_{T}}* and *β_{a_{T}}*, respectively. The same was done with the temperature calibration parameters *m_{b_{T}}* and *β_{b_{T}}*, corresponding to the "b" Michaelis-Menten parameter, and *m_{c_{T}}* and *β_{c_{T}}*, corresponding to the "c" Michaelis-Menten parameter. The resulting values for these TempCos are summarized in the Table 1, below.

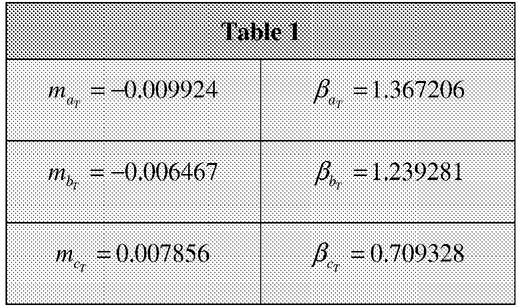

An equation for computing a temperature corrected glucose concentration from a fluorescent signal and these temperature calibration parameters may be derived by substituting Equation 2 into Equation 1 which yields $\left[Glu\right] = \frac{{c}_{37} ∗ {τ}_{c} \left(T∗\left[{G}_{i}-{a}_{37}*{τ}_{a}\left(T\right)\right]\right)}{{c}_{37} ∗ {τ}_{a} \left(T\right) + {b}_{37} * {τ}_{b} \left(T\right) - {G}_{i}}$ and then, using Equation 3, further substituting for *τₐ*(*T*), *τ_{b}*(*T*), and *τ_{c}*(*T*) in Equation (6), which yields $\left[Glu\right] = \frac{{c}_{37} ∗ \left({m}_{c}∗T+{b}_{c}\right) ∗ \left[{G}_{i}-{a}_{37}∗\left({m}_{a}∗T+{b}_{a}\right)\right]}{{a}_{37} ∗ \left({m}_{a}∗T+{b}_{a}\right) + {b}_{37} ∗ \left({m}_{b}∗T+{b}_{b}\right) - {G}_{i}}$

### Example 2

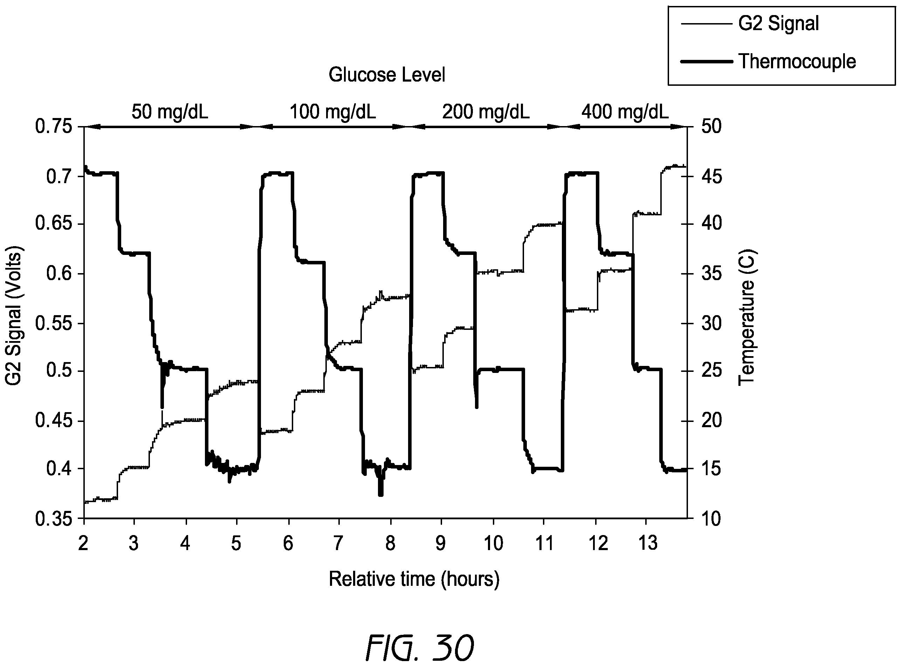

This example also concerns the temperature calibration of an equilibrium fluorescence glucose GluCath sensor. Again, the GluCath sensor employs an HPTS-Cys-MA dye operably coupled to a 3,3'-oBBV quencher, with the dye and quencher immobilized within a hydrogel disposed along the distal region of an optical fiber, while the proximal end of the optical fiber is coupled to a light source. The temperature dependence of this sensor's fluorescence response to glucose was assumed to be described by the modified Michaelis-Menten equation of Equation 1 as described above. The Michaelis-Menten parameters were assumed to bear a linear relationship to temperature as set forth in Equations 2 and 3 above. Using this model of the glucose sensor's temperature dependence, the TempCos, *m_{a_{T}}*, *β_{a_{T}}*, *m_{b_{T}}*, *β_{b_{T}}*, *m_{c_{T}},* and *β_{c_{T}}*, were determined by the methodology described above in reference to Equations 2 and 3. Specifically, the effect of temperature on the fluorescent signal was determined experimentally by measuring the signal at four temperatures (*15 °C, 25 °C, 37 °C,* and *45 °C*) and four glucose concentrations *(50 mg*/*dL, 100 mg*/*dL, 200 mg*/*dL,* and *400 mg*/*dL).* At each temperature and glucose level the stable signal, *G*₂, was recorded. The raw data from this experiment is plotted in **FIG. 30****.**

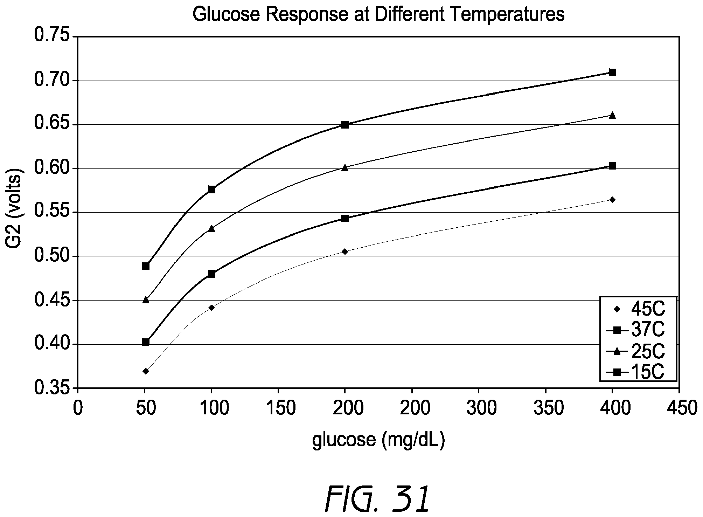

**FIG. 31** plots glucose response-*G*₂ versus glucose concentration-for each of the four temperatures. This figure illustrates that the fluorescent signal *G*₂ is inversely related to temperature and also that there are large differences in the fluorescent signal *G*₂ at all glucose levels over the range of temperatures likely to be encountered in the intensive care unit-i.e. *15 °C* to *45 °C.* At each fixed temperature, the modified Michaelis-Menten equation (equation 1) was fit to the fluorescent signal *G*₂ versus glucose concentration to determine best-fit values of the Michaelis-Menten parameters *a_{T}, b_{T},* and *c_{T}* at each temperature. The four best-fit modified Michaelis-Menten equations are overlaid on the data in **FIG. 31****.** The quality of the fits is evident from the figure.

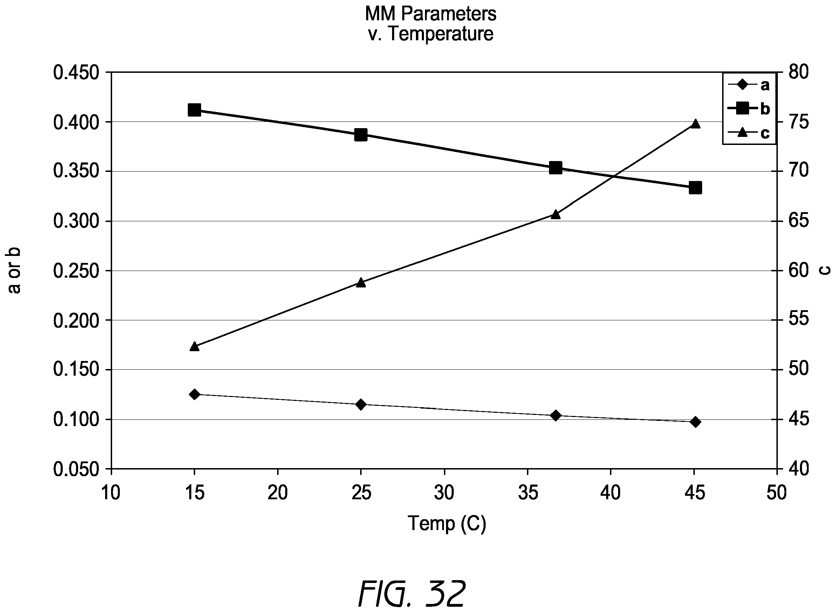

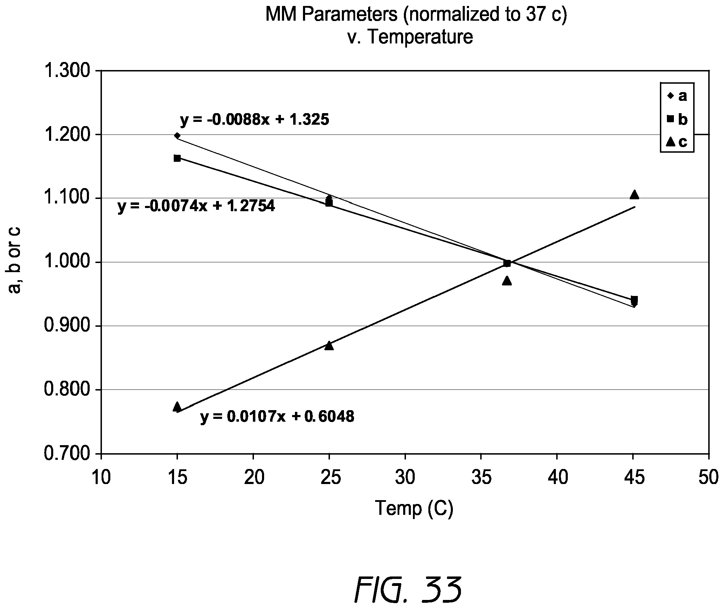

**FIG. 32** plots each of *a_{T}, b_{T},* and *c_{T}* versus temperature, the values of the Michaelis-Menten parameters corresponding to the best-fits displayed in **FIG. 31****.** The plots of each of *a_{T}, b_{T},* and *c_{T}* versus temperature illustrate that, in some embodiments, best-fit values of the Michaelis-Menten parameters change approximately linearly with temperature. The best-fit values of the Michaelis-Menten parameters at each temperature were normalized to their values at *37 °C* and the normalized values were fit using linear regression to compute slopes and intercepts as shown in **FIG. 33****.**

The slopes and intercepts displayed in **FIG. 33** correspond to the "temperature calibration coefficients" *m_{a_{T}}*, *m_{b_{T}}*, *m_{c_{T}}, β_{a_{T}}*, *β_{b_{T}}*, and *β_{c_{T}}*, i.e. "TempCos," as described above. The particular values computed from the data in this example are listed in Table 2, below.

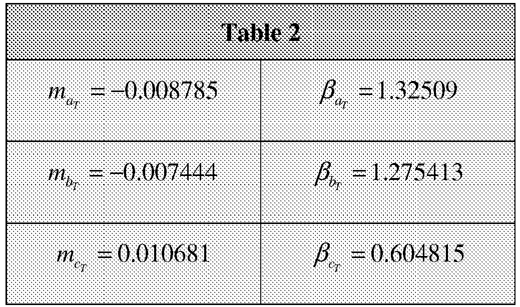

The TempCos can then be used to predict the temperature dependent Michaelis-Menten parameters *a_{T}, b_{T},* and *c_{T}* by multiplying the *37 °C* Michaelis-Menten parameters *a₃₇, b₃₇,* and *c₃₇,* by a temperature correction factor, *τ_{a_{T}}*(*T*), *τ_{b_{T}}*(*T*), or *τ_{c_{T}}*(*T*), respectively, as indicated by Equation 3. Again, the *37 °C* Michaelis-Menten parameters *a₃₇, b₃₇,* and *c₃₇* may be measured at calibration, determined by a factory calibration, or potentially supplied by any other appropriate method.

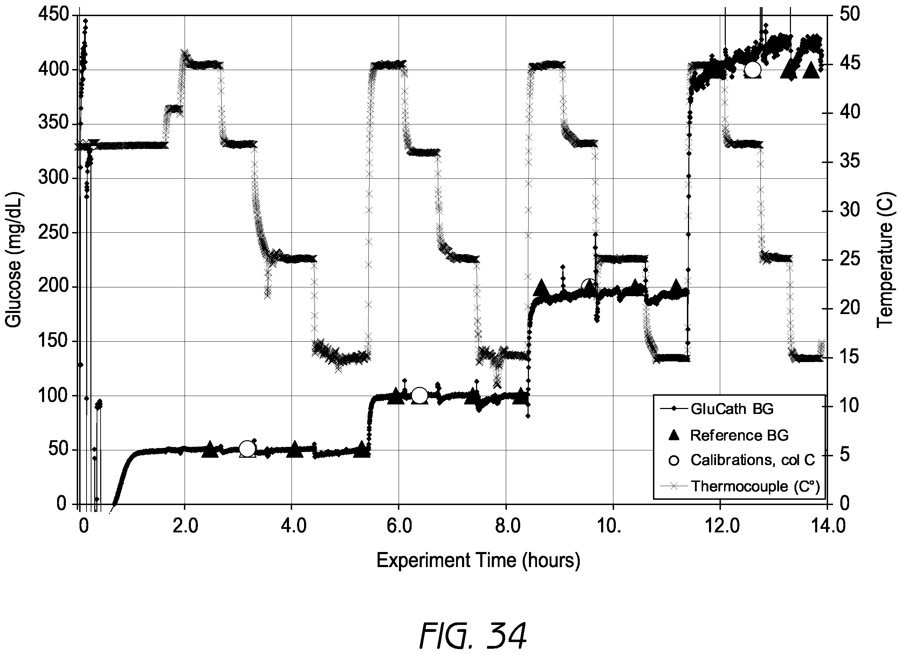

In order to illustrate the accuracy of employing the above-described temperature correction methodology, these TempCos were used to perform temperature correction on an independent data set, as illustrated in **FIG. 34****.** In the independent data set, the *G*₂ fluorescent signal versus plasma glucose concentration was measured at four reference plasma glucose levels *(50 mg*/*dL, 100 mg*/*dL, 200 mg*/*dL,* and *400 mg*/*dL)* and at four temperatures (*15 °C, 25 °C, 37 °C,* and *45 °C*). The reference measurements of plasma glucose level are indicated as single points (small and medium sized circles) in **FIG. 34****.** Temperature measurements as determined by thermocouple are also displayed.

The other curve displayed in **FIG. 34** is the temperature corrected plasma glucose concentration. To compute this curve, the four reference plasma glucose levels at *37 °C* (indicated as Calibrations in **FIG. 34**) were fit to a modified Michaelis-Menten equation (Equation 1, as previously described) to generate values of the *37 °C* Michaelis-Menten parameters (*a₃₇, b₃₇,* and *c₃₇*). The temperature corrected plasma glucose concentration curve in **FIG. 34** was then computed at each temperature read by the thermocouple by inputting into Equations 2 and 3 these *37 °C* Michaelis-Menten parameters (*a₃₇, b₃₇,* and *c₃₇*), the TempCos (*m_{a_{T}}*, *m_{b_{T}}*, *m_{c_{T}}*, *β_{a_{T}}*, *β_{b_{T}}*, and *β_{c_{T}}*, as determined in the table above), and the corresponding temperature read by the thermocouple. The temperature corrected plasma glucose concentration curve (indicated as GluCath BG) matches closely the reference plasma glucose levels in **FIG. 34****.** In fact, the mean absolute relative deviation ("MARD") between the computed temperature corrected plasma glucose concentrations and the reference plasma glucose levels was only 2.46% (excluding the *37 °C* reference values used for calibration). The MARD for the same data set without temperature correction was 170%.

### Methods of Measuring pH

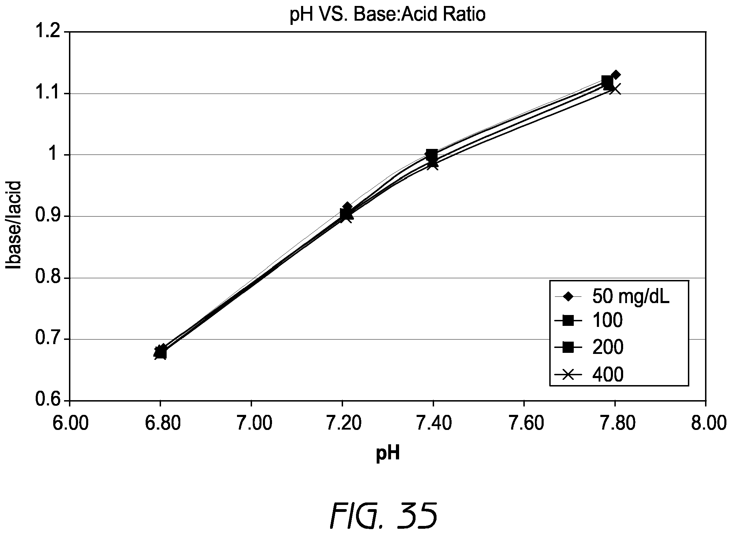

If a solution's measured analyte concentration is to be corrected for pH effects, the pH of the solution must be measured or estimated in some manner. In some embodiments a separate pH sensor may be used to measure pH. In other embodiments, the same indicator system which is used to generate a signal indicative of analyte concentration may be used to measure pH. For instance, the ratio of two green signals generated by the indicator system may be used to compute pH through the following relationship: $pH = {m}_{pH} ∗ \frac{{G}_{1}}{{G}_{2}} + {β}_{pH}$ where *m_{pH}* is the slope and *β_{pH}* is the intercept of *pH* versus *G*₁/*G*₂. The ratio, *G*₁/*G*₂, is calculated from *G*₁ which is the fluorescence emission at 550 nm or 583 nm when the fluorophore is excited at 470 nm, and *G*₂ which is the fluorescence emission at 550 nm or 583 nm when the fluorophore is excited at 420 nm or 430 nm. The approximate linearity of the relationship between *G*₁/*G*₂ and *pH* is illustrated in **FIG.** 35 over a range of glucose concentrations *(50 mg*/*dL, 100 mg*/*dL, 200 mg*/*dL,* and *400 mg*/*dL*) and pH levels (6.8, 7.2, 7.4, and 7.8), although some greater deviation from linearity occurs between pH 7.4 and pH 7.8. Note, that *G*₁/*G*₂ is represented in **FIG.** 35 as *Ibase*/*Iacid* since, as indicated above, 470 nm is the absorption maximum of the fluorophore's base-form, and 430 nm is the absorption maximum of the fluorophore's acid-form. Also, note that the values of *G*₁/*G*₂ plotted in **FIG.** 35 have been normalized to the *100 mg*/*dL* , pH 7.4 value of *G*₁/*G*₂. Thus, Equation 8 may be used to predict pH level from the *G*₁/*G*₂ ratio once the constants *m_{pH}* and *β_{pH}* have been determined. In some embodiments, each analyte measurement device may be individually calibrated to determine the constants *m_{pH}* and *β_{pH}* appropriate for that individual device. In other embodiments, an entire batch of measurement devices may be calibrated by selecting several devices from the batch, determining values of *m_{pH}* and *β_{pH}* for each selected device, and averaging the values of *m_{pH}* and *β_{pH}* obtained for each selected devices to produce averaged values of *m_{pH}* and *β_{pH}* valid for the entire batch of measurement devices for use with Equation 8. In still other embodiments, averaged values of *m_{pH}* and *β_{pH}* may be determined as just described, but a one-point calibration is performed to individually calibrate each sensor in the batch while taking advantage of the averaged values of *m_{pH}* and *β_{pH}* obtained for the entire batch. For instance, in some embodiments, the one point calibration performed on each individual measuring device may involve using the individual device to measure *G*₁ and *G*₂ for a standard solution having a glucose concentration of *100 mg*/*dL* at *pH 7.4.* These values may then be used in Equation 9 $pH = {m}_{pH} ∗ \frac{\frac{{G}_{1}}{{G}_{2}}}{\frac{{G}_{1,7.4}}{{G}_{2,7.4}}} + {β}_{pH}$ where:
*G*₁ is the fluorescent emission, either referenced or unreferenced, at 550 nm or 583 nm when the fluorophore is excited at 470 nm, which is the absorption maximum of the fluorophore's base-form (although other combinations of excitation and emission wavelengths are also feasible for use as the numerator of the *G*₁/*G*₂ ratio in Equation 9),
*G*₂ is the fluorescence emission, either referenced or unreferenced, at 550 nm or 583 nm when the fluorophore is excited at 430nm, which is the absorption maximum of the fluorophore's acid-form (although other combinations of excitation and emission wavelengths are also feasible for use as the denominator of the *G*₁/*G*₂ ratio in Equation 9),
*G*_{1,7.4} = *G*₁ signal at *pH 7.4* and *100 mg*/*dL* glucose concentration,
*G*_{2,7.4} = *G*₂ signal at *pH 7.4* and *100 mg*/*dL* glucose concentration,
*m_{pH}* = pH slope, and
*β_{pH}* = pH intercept.

Thus, once universal values of *m_{pH}* and *β_{pH}* are determined for the batch of measurement devices , and *G*_{1,7.4} and *G*_{2,7.4} are determined via one-point calibration for the individual measurement device, a measured ratio *G*₁/*G*₂ may be used in Equation 9 to compute the pH level of the solution of analyte. It was also discovered that the determination of pH from *G*₁/*G*₂ is effected by the temperature of the solution - see for instance, Example 3, below. Moreover, for purposes of estimating pH from the measured ratio *G*₁/*G*₂ , the temperature dependence may be taken into account by allowing *m_{pH}* and *β_{pH}* to vary with temperature. In particular, the temperature dependence of *m_{pH}* and *β_{pH}* may be modeled using Equations 10 and 11: ${m}_{pH} = \frac{h}{T} + i$ ${β}_{pH} = j ∗ \sqrt{T} + k$ where *h, i, j,* and *k* are empirically determined constants - see, for instance, Example 3, below. Substituting Equations 10 and 11 into Equation 9 gives an expression for computing pH from the ratio *G*₁/*G*₂ which accurately, albeit approximately, takes temperature into account. $pH = \left(\frac{h}{T}+i\right) ∗ \frac{\left(\frac{{G}_{1}}{{G}_{2}}\right)}{\left(\frac{{G}_{1,7.4}}{{G}_{2,7.4}}\right)} + j ∗ \sqrt{T} + k$

### Example 3

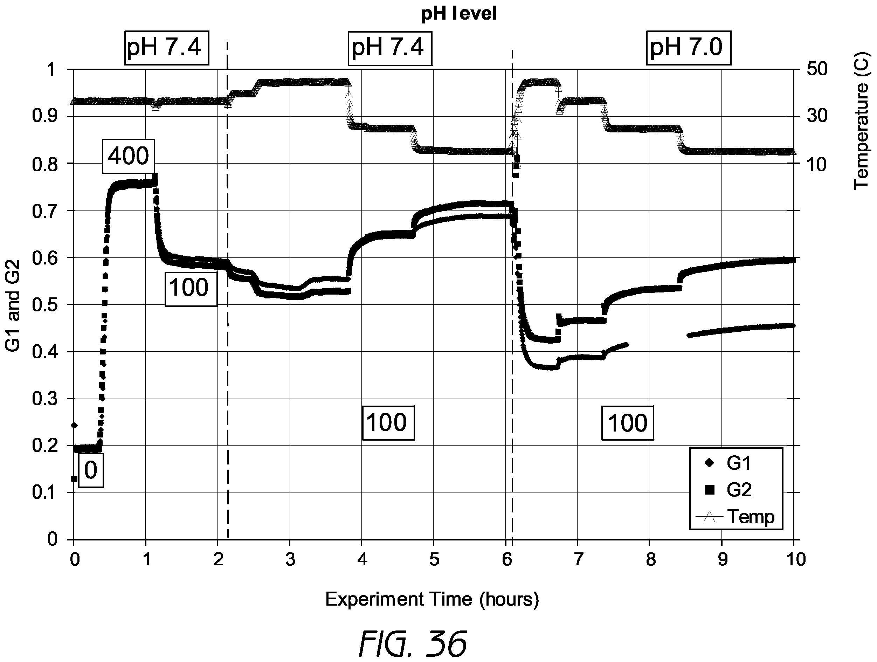

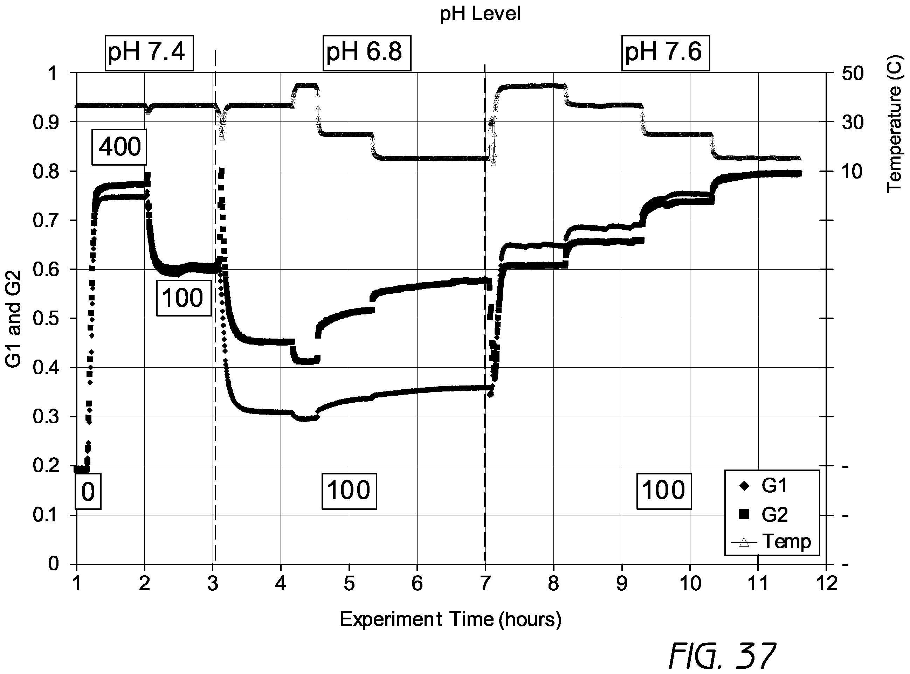

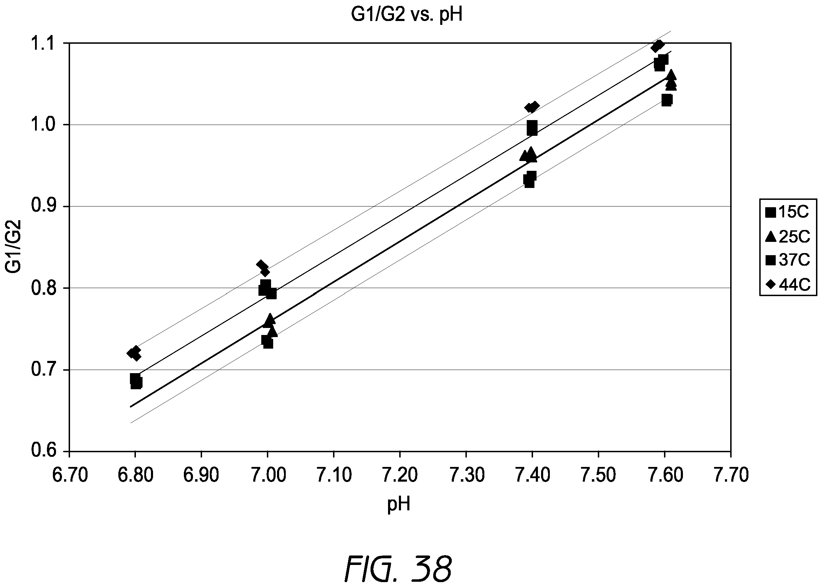

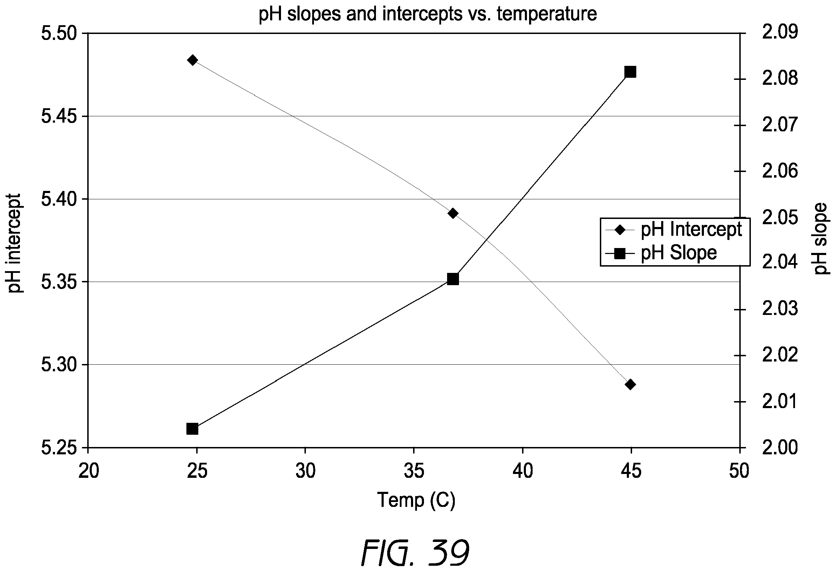

In order to accurately determine pH from the measured *G*₁/*G*₂ ratio using Equation 12, so as to take temperature into account, testing was conducted to empirically determine the constants, *h, i, j,* and *k.* In the experiment displayed in **FIGS.** 36 and 37, *G*₁ and *G*₂ were measured with a glucose sensor at four temperatures (*15 °C, 25 °C, 37 °C,* and *45 °C*) and four pH levels (6.8, 7.0, 7.4, 7.6), all at a glucose concentration of *100 mg*/*dL.* Note that **FIGS.** 36 and 37 demonstrate, among other things, that the *G*₁ and *G*₂ fluorescent signals are effected by temperature change, even when the pH level is held constant. **FIG.** 38 shows the ratio of the measured values of *G*₁ and *G*₂ from **FIGS.** 36 and 37 plotted versus pH at each of the four temperatures. A line was fit (using linear regression) to each series of *G*₁/*G*₂ values corresponding to the same temperature. **FIG.** 38 illustrates that the intercepts of these lines, and to a lesser extent the slopes of these lines, vary with temperature. To model this temperature dependence, the associated slopes and intercepts are plotted as functions of temperature in **FIG.** 39. Equation 10 was used to model the temperature dependence of the slope *m_{pH}*, and based on the data displayed in **FIG.** 39 best fit values of the constants *h,* and *i* were determined. Similarly, Equation 11 was used to model the temperature dependence of the intercept *β_{pH}*, and based on the data displayed in **FIG.** 39 best fit values of the constants *j*, and *k* were determined. The best fit values of *h, i, j,* and *k* to be used in Equation 12 are listed in Table 3.

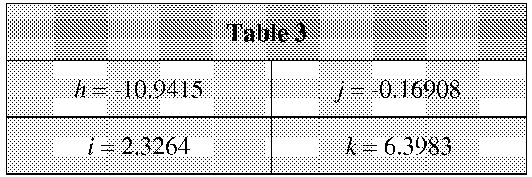

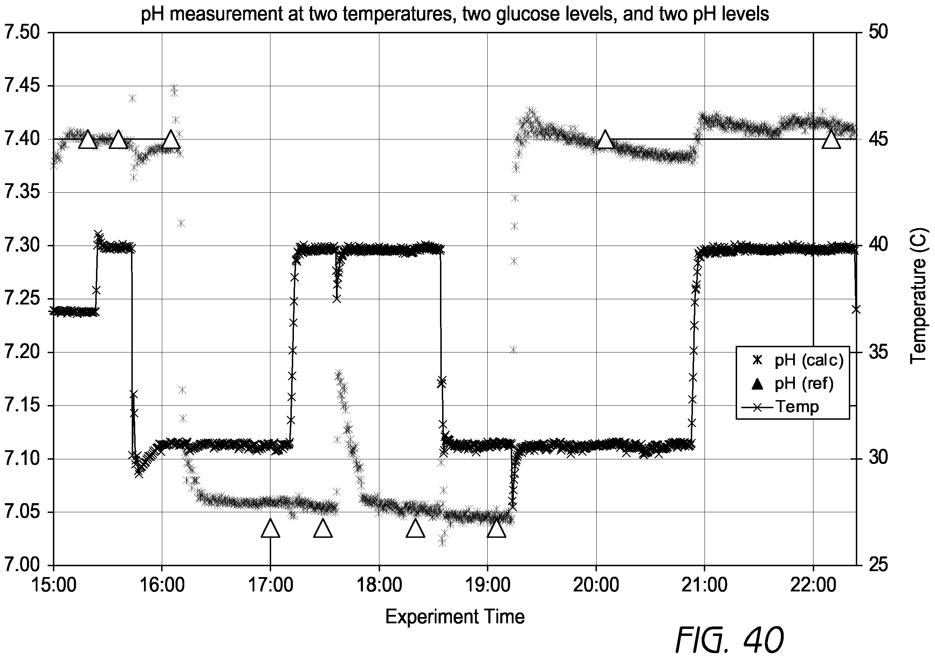

An independent data set was generated to test the accuracy of using Equation 12 to compute pH level from measured *G*₁/*G*₂ ratio, using these values of *h, i, j,* and *k*. **FIG.** 40 displays both measured reference pH values (small and medium sized dots) and values of pH computed from *G*₁/*G*₂ with Equation 12 (sequence of 'x' marks)-at two temperatures (*30 °C,* and *40 °C*) and two glucose levels (*50 mg*/*dL* and *100 mg*/*dL*). Note that the medium sized dot (corresponding to a glucose concentration of *100 mg*/*dL* and *pH 7.4*) served as the reference point for the one-point calibration as described above. The results are displayed in the following table. As indicated in Table 4, the average pH offset for this data set was +0.008.

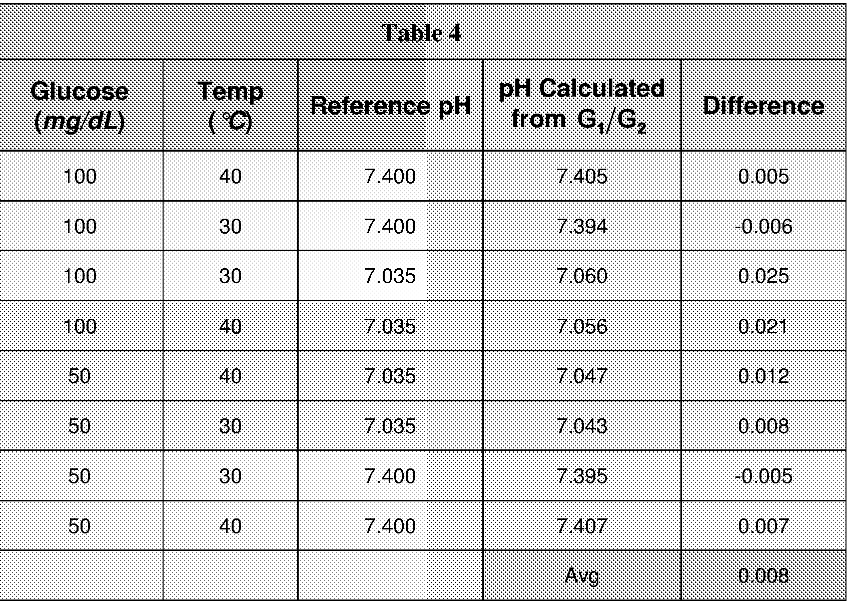

### Methods of Estimating Analyte Concentration Incorporating pH Correction

Some embodiments of the measurement devices disclosed herein generate a signal indicative of analyte concentration which exhibits a pH dependence. For example, if two solutions of precisely the same analyte concentration are measured at two different pH levels with the same measurement device, in some embodiments, the measurement device may generate differing signals indicative of the two analyte concentrations. Thus, the accuracy of determining a solution's true analyte concentration based on such as signal may be improved by taking the pH of the solution into account.

It has been discovered that for some embodiments of the measurement devices disclosed herein, and in particular, for glucose measurement devices employing a quencher binding moiety operably coupled to a fluorophore, the pH dependence of the fluorescent signal approximately follows a modified version of the classic Michaelis-Menten equation from enzyme kinetics: $\left[Glu\right] = \frac{{c}_{pH} ∗ ⌊ {G}_{i} - {a}_{pH} ⌋}{{a}_{pH} + {b}_{pH} - {G}_{i}}$ where:
*[Glu]* is the estimated glucose concentration,
*a_{pH}* is the first Michaelis-Menten parameter "*a*", at a particular pH,
*b_{pH}* is the second Michaelis-Menten parameter "*b*", at the same particular pH,
*c_{pH}* is the third Michaelis-Menten parameter "*c*", at the same particular pH, and
*Gᵢ* is the fluorescent signal (*i* = 1,2), either referenced or unreferenced, where *G*₁ is the fluorescence emission at 550 nm or 583 nm when the fluorophore is excited at 470 nm (which is the absorption maximum of the fluorophore's base-form), and *G*₂ is the fluorescence emission at 550 nm or 583 nm when the fluorophore is excited at 430 nm (which is the absorption maximum of the fluorophore's acid-form). Note, however, that other combinations of excitation and emission wavelengths are also feasible for use in Equation 13. In the Examples below, *G*₂ has been used, unless indicated otherwise.

As with temperature dependence, the fact that pH dependence may be described by a modified Michaelis-Menten equation is an interesting and surprising result. Various embodiments of the measurement devices disclosed herein employ a quencher-fluorophore indicator system which measures analyte concentration through the establishment of an equilibrium between the analyte of interest, the binding moiety (e.g. quencher), and the fluorophore. In such a system, analyte concentration is not measured by enzymatic consumption or conversion of the analyte. In contrast, the classic Michaelis-Menten equation specifically describes enzyme kinetics, a non-equilibrium phenomena involving the consumption/conversion of the enzyme's substrate by the enzyme. Therefore, it is not to be expected that an equation closely related to the classic Michaelis-Menten equation would effectively describe the pH dependence of these types of quencher-fluorophore-based measurement devices and analyte sensing elements (or other measurement devices and analyte sensing elements functioning through analogous equilibrium mechanisms). In any event, knowledge that these devices (and similar devices) exhibit a pH dependence which follows a modified Michaelis-Menten equation allows the use of pH correction methods and algorithms to improve the accuracy of analyte concentration measurements. Such methods and algorithms are disclosed herein, along with measurement devices which implement such methods and algorithms.

Accordingly, some embodiment methods of estimating an analyte concentration include generating a signal indicative of analyte concentration and a signal indicative of pH. In some embodiments, the signal indicative of the analyte concentration and the signal indicative of the pH are both generated from a set of at least two signals each of which is indicative of both the pH and the analyte concentration. For instance, these could be the *G*₁ and *G*₂ fluorescent signals described above, both of which are indicative of both analyte concentration and pH. In some embodiments, the *G*₂ fluorescent signal itself may be treated as the signal indicative of analyte concentration, while *G*₁ and *G*₂ are used as signals indicative of pH, for example, in the pH determination algorithm described above. Since, in some embodiments, the signal indicative of analyte concentration exhibits a pH dependence, in some embodiments, the signal indicative of pH may be used to adjust the signal indicative of analyte concentration to correct for pH dependence. Thus, in certain such embodiments, the methods further include transforming the signal indicative of the analyte concentration utilizing an equation of the form of a modified Michaelis-Menten equation, such as Equation 1 above, depending on Michaelis-Menten parameters, such as the parameters "*a*", "*b*", and "*c*", as described above as first, second, and third Michaelis-Menten parameters with reference to Equation 13.

Of course, it is to be understood that when a signal is described herein as being indicative of one physical quantity, such description is not meant to necessarily preclude that signal from also being indicative of another physical quantity. For instance, a computed analyte concentration that may be improved through pH correction, was likely computed from a signal indicative of analyte concentration which contained some pH dependency. Therefore, to a certain extent, such a signal indicative of analyte concentration may also be considered a signal indicative of pH, as will be readily appreciated by one of skill in the art.

The pH dependence of Equation 13 is exhibited through the Michaelis-Menten parameters *a_{pH}, b_{pH},* and *c_{pH},* as indicated by the subscript "*pH*" labeling these parameters. In some embodiments, the pH dependence may need to be determined through a pH calibration. Thus, in certain embodiment methods, the values of one or more of the Michaelis-Menten parameters may be set based on data which includes pH calibration data and the signal indicative of a pH.

For example, in some embodiment methods, the pH calibration data may be generated by a pH calibration method. The pH calibration method may include selecting a first test analyte sensing element, and creating and/or providing a set of at least three solutions of differing known analyte concentrations. In certain such embodiments, a first pH is selected (*pH1*), three solutions of the set of at least three solutions are adjusted to a pH substantially similar to the selected first pH, and a first set of at least three signals is generated using the first test analyte sensing element, each signal indicative of the concentration of analyte in a different one of the three solutions at the first pH. Measurements are then made at a second pH. Thus, in certain embodiments, a second pH is selected (*pH2*), three solutions of the set of at least three solutions (each of the three may be the same or different than a solution chosen for the first pH) are adjusted to a pH substantially similar to the selected second pH, and a second set of at least three signals is generated using the first test analyte sensing element, each signal indicative of the concentration of analyte in a different one of the three solutions at the second pH. Of course, more than three solutions may be used in either of these steps. And more than two pHs may also be employed. Generally, the more solutions of differing concentration and the greater number of different pHs that are employed, the greater the accuracy of the resulting calibration data.

Once the solutions having known analyte concentrations have been measured, and the first and second sets of at least three signals have been generated, in some embodiments, the sets of signals are used to determine (usually approximately) the relationship between one or more of the Michaelis-Menten parameters and pH. For example, in some embodiments, the pH calibration method may further include computing values of each of a first, second, and third Michaelis-Menten parameter at the first pH (*a_{pH1}*, *b_{pH1}*, and *c_{pH1}*) by an algorithm comprising fitting a modified Michaelis-Menten equation to a first fit dataset comprising the first set of at least three signals. In certain such embodiments, the pH calibration method may further include computing values of each of a first, second, and third Michaelis-Menten parameter at the second pH (*a_{pH2}*, *b_{pH2}*, and *c_{pH2}*) by an algorithm comprising fitting a modified Michaelis-Menten equation to a second fit dataset comprising the second set of at least three signals. Thus, in methods such as these, each of the three Michaelis-Menten parameters has been determined at least two pHs, providing data which may be used to create a model of the pH dependence of each of the three Michaelis-Menten parameters.

To model the pH dependence of the Michaelis-Menten parameters, in some embodiments, the pH calibration method may further include selecting an equation relating the first Michaelis-Menten parameter (*a_{pH}*) to pH, the equation depending on a first set of pH calibration parameters; and setting a value for each calibration parameter of the first set of calibration parameters based on the value of the first Michaelis-Menten parameter at the first pH (*a_{pH1}*) and the value of the first Michaelis-Menten parameter at the second pH (*a_{pH2}*). In some embodiments, similar steps are performed with respect to the second and third Michaelis-Menten parameters (*b_{pH}* and *c_{PH}*). Thus, for example, the pH calibration method may further include selecting an equation relating the second Michaelis-Menten parameter (*b_{pH}*) to pH, the equation depending on a second set of pH calibration parameters; and setting a value for each calibration parameter of the second set of calibration parameters based on the value of the second Michaelis-Menten parameter at the first pH (*b_{pH1}*) and the value of the second Michaelis-Menten parameter at the second pH (*b_{pH2}*)*.* Similarly, in some embodiments, the pH calibration method may further include selecting an equation relating the third Michaelis-Menten parameter (*c_{pH}*) to pH, the equation depending on a third set of pH calibration parameters; and setting a value for each calibration parameter of the third set of calibration parameters based on the value of the third Michaelis-Menten parameter at the first pH (*c_{pH1}*) and the value of the third Michaelis-Menten parameter at the second pH (*c_{pH2}*).

Furthermore, in some embodiments, equations linear in pH may be selected to relate the first and second Michaelis-Menten parameters to pH, while a more complicated equation may be selected to relate the third Michaelis-Menten parameter to pH. For instance, in some embodiments, the first, second, and third Michaelis-Menten parameters may be written as $\begin{matrix}{a}_{pH} = {a}_{7.4} ∗ {ρ}_{{a}_{pH}} \left(pH\right) , \\ {b}_{pH} = {a}_{7.4} ∗ {ρ}_{{b}_{pH}} \left(pH\right) , and \\ {c}_{pH} = {c}_{7.4} ∗ {ρ}_{{c}_{pH}} \left(pH\right)\end{matrix}$ where *ρ_{a_{pH}}*(*pH*), *ρ_{b_{pH}}*(*pH*), and *ρ_{c_{pH}}*(*pH*) are "pH correction factors" which approximately account for the pH dependence of *a_{pH}, b_{pH},* and *c_{pH}.* When the relationship between Michaelis-Menten parameter and pH is written as such, each Michaelis-Menten parameter *a_{pH}, b_{pH},* and *c_{pH},* is determined by multiplying the *pH 7.4* Michaelis-Menten parameter *a_{7.4}, b_{7.4},* and *c_{7.4},* by its corresponding "pH correction factor," *ρ_{a_{pH}}*(*pH*), *ρ_{b_{pH}}*(*pH*), or *ρ_{c_{pH}}*(*pH*), respectively. The *pH 7.4* Michaelis-Menten parameters may be determined by fitting a modified Michaelis-Menten equation to a set of signals indicative of the analyte concentration of a plurality of solutions of differing analyte concentrations held at *pH 7.4,* as described above with respect to, for example, *pH1* and *pH2.* Alternatively, the parameters *a_{7.4}, b_{7.4},* and *c_{7.4}* may be supplied by a factory calibration as described in U.S. Patent Publication No. 2010/0312483. As yet another alternative, *a_{7.4}, b_{7.4},* and *c_{7.4}* may be determine via a one-point in vivo calibration as also disclosed in the same application.

To determine the "pH correction factors," *ρ_{a_{pH}}*(*pH*), *p_{b_{pH}}*(*pH*), *ρ_{c_{pH}}*(*pH*), some embodiment methods may select a first equation linear in pH to relate the first Michaelis-Menten parameter to pH, and select a second equation linear in pH to relate the second Michaelis-Menten parameter to pH. In certain such embodiment methods, an equation is selected to relate the third Michaelis-Menten parameter to pH which comprises a fraction wherein the numerator is equal to an exponential function of an equation linear in the inverse of pH, and the denominator is equal to an exponential function of the same linear function in the inverse of pH evaluated at pH 7.4. If such equations in pH are selected, then the pH correction factors may be written as $\begin{matrix}{ρ}_{{a}_{pH}} \left(pH\right) = {m}_{{a}_{pH}} ∗ pH + {β}_{a} {}_{{}_{pH}} , \\ {ρ}_{{b}_{pH}} \left(pH\right) = {m}_{{b}_{pH}} ∗ pH + {β}_{{b}_{pH}} , and \\ {ρ}_{{c}_{pH}} \left(pH\right) = \frac{{e}^{\left(\frac{{m}_{{c}_{pH}}}{pH}+{β}_{{c}_{pH}}\right)}}{{e}^{\left(\frac{{m}_{{c}_{pH}}}{7.4}+{β}_{{c}_{pH}}\right)}} .\end{matrix}$ where the slopes, *m_{a_{pH}}*, *m_{b_{pH}}*, *m_{c_{pH}}*, and intercepts, *β_{a_{pH}}*, *β_{b_{pH}}*, *β_{c_{pH}}*, are collectively referred to as pH calibration coefficients ("pHCos"). However, analytic functional forms other than linear equations may be chosen to relate the pH correction factors and/or Michaelis-Menten parameters to pH (or to inverses of pH as indicated by Equation 15's expression for *ρ_{c_{pH}}*(*pH*)). For instance, in some embodiments, quadratic or higher-order polynomials in pH may be appropriate and/or desirable.

In various embodiments, a pH calibration method used to determine values of these pHCos may require that values of the Michaelis-Menten parameters be determined at a second pH (*pH2*), different than *pH 7.4.* Values of the parameters at the second pH (*a_{pH2}*, *b_{pH2}*, and *c_{pH2}*) may be determined by fitting a modified Michaelis-Menten equation to a set of signals indicative of the analyte concentration of a plurality of solutions of differing analyte concentrations held at the second pH, as described above with respect to, for example, *pH1* and *pH2.* Once this is done, the pH calibration coefficients *m_{a_{pH}}* and *β_{a_{pH}}* may be determined by normalizing to *a_{7.4}* both *a_{pH2}* and *a_{7.4},* yielding *a_{pH2}*/*a_{7.4}* and *1,* and fitting a line to the normalized values versus the two pHs, *pH2* and *pH 7.4.* The fit may be determined using linear least squares or any other method of fitting a line to a set of points. The pH calibration coefficient, *m_{a_{pH}}*, is set equal to the slope of the resulting line and the pH calibration coefficient, *β_{a_{pH}}*, is set equal to the intercept. The pH calibration coefficients *m_{b_{pH}}* and *β_{b_{pH}}* may be determined the same way from values of *b_{pH2}* and *b_{7.4}.* Finally, in a manner analogous to the determination of *m_{a_{pH}}*, *β_{a_{pH}}*, *m_{b_{pH}}*, and *β_{b_{pH}}*, the pH calibration coefficients *m_{c_{pH}}* and *β_{c_{pH}}* may be determined from values of *c_{pH2}* and *c_{7.4},* however an additional step of linearizing Equation 15's expression for *p_{c_{pH}}*(*pH*) must first be performed. Once the calibration is complete, a pH corrected estimated glucose concentration ([*Glu*]) may be computed from a fluorescent signal (*Gᵢ*) measured at a particular pH, by using the pHCos (*m_{a_{pH}}*, *β_{a_{pH}}*, *m_{b_{pH}}*, *β_{b_{pH}}*, *m_{c_{pH}}*, and *β_{c_{pH}}*), the *pH 7.4* Michaelis-Menten parameters (*a_{7.4}, b_{7.4},* and *c*_{*7*.*4*}), and the measured pH (*pH*) in Equations 14 and 15 to compute *a_{pH}, b_{pH},* and *c_{pH},* and then plugging *a_{pH}, b_{pH}, c_{pH}* and the measured fluorescent signal (*Gᵢ*) into Equation 13.

Thus, in some embodiments, the first set of pH calibration parameters comprises the slope and intercept of a first equation linear in pH, and in some embodiments, the second set of pH calibration parameters comprises the slope and intercept of a second equation linear in pH. In certain such embodiments, the third set of pH calibration parameters may comprise the slope and intercept of an equation linear in the inverse of pH which is related to the third Michaelis-Menten parameter through an exponential function divided by a constant-wherein the constant is equal to the result of evaluating the exponential function of the same equation linear in the inverse of pH evaluated at a fixed pH level. However, the pH calibration parameters (pHCos) may comprise constants associated with analytic functional forms other than linear equations which may be suitable and/or desirable. For instance, in some embodiments, the pHCos may include the coefficients of quadratic or higher-order polynomials in pH.

When measurement devices are mass produced, it may not be feasible or practical to individually calibrate each measurement device-i.e. use each individual measurement device to generate individual calibration data. It may be more cost effective to select one or more test devices from a batch of mass produced devices, generate calibration data using the one or more test devices, and provide that calibration data to each individual devices produced in the batch. In some embodiments, variability between measurement devices from the same production batch may be, to a large extent, attributable to a particular part of the measurement device. In particular, variability between devices may be attributable to the part of the measurement device which generates a signal indicative of analyte concentration-e.g. the analyte sensing element- and/or the part of the measurement device that generates a signal indicative of pH-e.g. the pH sensing element. In these circumstances, as well as others, it may be advantageous to use a calibration method employing multiple test measurement devices, and/or multiple test sensing elements, because calibration over multiple test devices and/or sensing elements may yield more accurate calibration data than calibration methods which only utilize a single test device and/or sensing element. Accordingly, in some embodiments, the calibration method may further include selecting a second test analyte sensing element; generating a third set of at least three signals using the second test analyte sensing element, each signal indicative of the concentration of analyte in a different solution of known analyte concentration at the first pH (*pH1*); and generating a fourth set of at least three signals using the second test analyte sensing element, each signal indicative of the concentration of analyte in a different solution of known analyte concentration at the second pH *(pH2).* Obviously, calibration methods may similarly employ more than two test devices, or more particularly, for instance, more than two test analyte sensing elements.

In a manner similar to methods utilizing a single test analyte sensing element, after the solutions having known analyte concentrations have been measured and the first, second, third, and fourth sets of at least three signals have been generated, in some embodiments, the sets of signals are used to determine (usually approximately) the relationship between one or more of the Michaelis-Menten parameters and pH. For example, in some embodiments, the pH calibration method may further include (1) computing values of each of a first, second, and third Michaelis-Menten parameter at the first pH (*a_{pH1}, b_{pH1},* and *c_{pH1}*) by an algorithm comprising fitting a modified Michaelis-Menten equation to a first fit dataset comprising both the first set of at least three signals (which was generated with the first test analyte sensing element at *pH1*) and the third set of at least three signals (which was generated with the second test analyte sensing element at *pH1*); and (2) computing values of each of a first, second, and third Michaelis-Menten parameter at the second pH (*a_{pH2}*, *b_{pH2}*, and *c_{pH2}*) by an algorithm comprising fitting a modified Michaelis-Menten equation to a second fit dataset comprising both the second set of at least three signals (which was generated with the first test analyte sensing element at *pH2*) and fourth set of at least three signals (which was generated with the second test analyte sensing element at *pH2*). Essentially, in these types of methods, the signals generated with the second test analyte sensing element are used in a combined fit with the signals generated with the first test analyte sensing element, which results in values for each of the first, second, and third Michaelis-Menten parameters which take both test analyte sensing elements into account. Alternatively, in some embodiments, a pH calibration method may take both test analyte sensing elements into account by fitting the signals generated from each test analyte sensing element separately, and then averaging the results to obtain better estimates of the Michaelis-Menten parameters. Thus, in some embodiments, the step of computing values of each of a first, second, and third Michaelis-Menten parameter at the first pH (*a_{pH1}*, *b*_{*pH1*,} and *c_{pH1}*) by an algorithm may further include fitting a modified Michaelis-Menten equation to a third fit dataset comprising the third set of at least three signals, and averaging the results of fitting the third fit dataset with the results of fitting the first fit dataset. In addition, the step of computing values of each of a first, second, and third Michaelis-Menten parameter at the second pH (*a_{pH2}*, *b_{pH2},* and *c_{pH2}*) by an algorithm may further include fitting a modified Michaelis-Menten equation to a fourth fit dataset comprising the fourth set of at least three signals, and averaging the results of fitting the fourth fit dataset with the results of fitting the second fit dataset.

Other methods for estimating analyte concentration which incorporate pH correction features and pH calibration steps are also disclosed herein. In some embodiments, these methods are similar to those already described above and incorporate similar features, however, additional features may also be disclosed and, in some embodiments, the disclosed methods may be more general and described in more general terms. Since there are many ways to feasibly implement the discoveries disclosed herein for use in estimating analyte concentration, the following additional methods are described in order to illustrate the breadth of implementations that are possible.

In some embodiments, for instance, a method of estimating an analyte concentration from a signal indicative of the analyte concentration may include transforming the signal using an equation of the form of a modified Michaelis-Menten equation wherein the values of one or more Michaelis-Menten parameters have been adjusted for pH.

In some embodiments, for instance, a method of estimating an analyte concentration may include generating a signal indicative of the analyte concentration and generating a signal indicative of a pH, and transforming the signal indicative of the analyte concentration utilizing an equation of the form of a modified Michaelis-Menten equation wherein at least one of the Michaelis-Menten parameters has been substituted with a calibration equation functionally depending on a set of one or more pH calibration parameters and the signal indicative of pH. One could refer to such an equation as a "substituted" modified Michaelis-Menten equation since the Michaelis-Menten parameters have been explicitly substituted with equations depending on one or more other variables-pH and the pH calibration parameters. However, although such a "substituted" equation exhibits a more complicated analytic form, it nevertheless will still express the basic functional relationships of the modified Michaelis-Menten equation.

In some embodiments, the step of transforming the signal indicative of analyte concentration may utilize a "substituted" modified Michaelis-Menten equation in which each of the first, second, and third Michaelis-Menten parameters have been substituted with first, second, and third calibration equations (respectively), each of the equations depending on sets of first, second, and third pH calibration parameters (respectively), and each also depending on the signal indicative of pH. In certain embodiments, at least one of the first, second, and third calibration equations is a polynomial in the signal indicative of pH. In certain such embodiments, each of the first, second, and third calibration equations is a polynomial in the signal indicative of pH. In certain embodiments, at least one of the first, second, and third calibration equations is a linear equation in the signal indicative of pH. In certain such embodiments, the first and second calibration equations are a linear equations in the signal indicative of pH, and the third calibration equation comprises a fraction wherein the numerator is equal to an exponential function of an equation linear in the inverse of the signal indicative of pH, and the denominator is equal to an exponential function of the same linear function in the inverse of the signal indicative of pH evaluated at fixed pH.

Thus, for example, if each Michaelis-Menten parameter of Equation 13 above is assumed to exhibit a linear relationship with pH, then the "substituted" modified Michaelis-Menten equation might appear as $\left[Glu\right] = \frac{\left({χ}_{{c}_{pH} ,1}⋅pH+{χ}_{{c}_{pH} ,0}\right) ∗ \left[{G}_{i}-\left({χ}_{{a}_{pH} ,1}⋅pH+{χ}_{{a}_{pH} ,0}\right)\right]}{\left({χ}_{{a}_{pH} ,1}⋅pH+{χ}_{{a}_{pH} ,0}\right) + \left({χ}_{{b}_{pH} ,1}⋅pH+{χ}_{{b}_{pH} ,0}\right) - {G}_{i}}$ and, similarly, if each Michaelis-Menten parameter is assumed to exhibit a quadratic relationship with pH then the "substituted" modified Michaelis-Menten equation might appear as $\left[Glu\right] = \frac{\left({χ}_{{c}_{pH} ,2}⋅{pH}^{2}+{χ}_{{c}_{pH} ,1}⋅pH+{χ}_{{c}_{pH} ,0}\right) ∗ ⌊ {G}_{i} - \left({χ}_{{a}_{pH} ,2}⋅{pH}^{2}+{χ}_{{a}_{pH} ,1}⋅pH+{χ}_{{a}_{pH} ,0}\right) ⌋}{\left({χ}_{{a}_{pH} ,2}⋅{pH}^{2}+{χ}_{{a}_{pH} ,1}⋅pH+{χ}_{{a}_{pH} ,0}\right) + \left({χ}_{{b}_{pH} ,2}⋅{pH}^{2}+{χ}_{{b}_{pH} ,1}⋅pH+{χ}_{{b}_{pH} ,0}\right) - {G}_{i}}$ where:
[*Glu*] is the estimated glucose concentration,
*χ*_{*a_{pH}*,2}, *χ*_{*a_{pH}*,1}, and *χ*_{*a_{pH}*,0} are polynomial coefficients parameterizing *a_{pH}*'s dependence on the pH level,
*χ*_{*b_{pH}*,2}, *χ*_{*b_{pH}*,1}, and *χ*_{*b_{pH}*,0} are polynomial coefficients parameterizing *b_{pH}*'s dependence on the pH level,
*χ*_{*c_{pH}*,2}, *χ*_{*c_{pH}*,1}, and *χ*_{*c_{pH}*,0} are polynomial coefficients parameterizing *c_{pH}*'s dependence on the pH level, and
*Gᵢ* is the fluorescent signal (*i* = 1,2), either referenced or unreferenced, where *G*₁ is the fluorescence emission at 550 nm or 583 nm when the fluorophore is excited at 470 nm (which is the absorption maximum of the fluorophore's base-form), and *G*₂ is the fluorescence emission at 550 nm or 583 nm when the fluorophore is excited at 430 nm (which is the absorption maximum of the fluorophore's acid-form). Note, however, that other combinations of excitation and emission wavelengths are also feasible for use in Equations 16 and 17.

As stated above, although, the "substituted" equations (Equations 16 and 17) exhibit a more complicated analytic form, they nevertheless still exhibit the basic functional relationships of the modified Michaelis-Menten equation (Equation 13). In other embodiments, the calibration equations substituted into the modified Michaelis-Menten equation may have a functional form other than a polynomial in pH.

Thus, as described above, the calibration equations substituted into the modified Michaelis-Menten equation for the Michaelis-Menten parameters may take a variety of functional forms and each may have varying numbers of pH calibration parameters. Obviously, more complicated equations may have a greater numbers of pH calibration parameters. In any event, depending on the embodiment, various pH calibration methods may be used to determine the values of the first, second, and third sets of the one or more pH calibration parameters. In certain such embodiments, each set of pH calibration parameters may be determined by fitting the "substituted" modified Michaelis-Menten equation to a plurality of signals, the plurality of signals indicative of analyte concentration in a plurality of solutions at a plurality of pHs. Once values of the various pH calibration parameters are determined, pH corrected estimates of analyte concentrations may be generated from signals indicative of analyte concentration and pH.

### Temperature and pH Correction

Furthermore, in certain embodiments, the analyte sensors further include a receiving and processing unit configured to transform the signal indicative of the analyte concentration based, in part, on a signal indicative of temperature or pH generated by the temperature or pH sensing element. In certain embodiments, the receiving and processing unit is configured to transform the signal indicative of the analyte concentration utilizing an equation of the form of a modified Michaelis-Menten equation depending on Michaelis-Menten parameters. In some embodiments, the values of the Michaelis-Menten parameters are set based on data comprising temperature calibration data and the signal indicative of the temperature, as well as pH calibration data and signal indicative of the pH. Such calibration and correction methods are described in greater detail in co-pending U.S. Application No. 13/046,571.

Some embodiments of the analyte sensors disclosed herein generate a signal indicative of analyte concentration which exhibits a temperature dependence. For example, if two solutions of precisely the same analyte concentration are measured at two different temperatures with the same analyte sensor, in some embodiments, the analyte sensor may generate differing signals indicative of the two analyte concentrations. Thus, the accuracy of determining a solution's true analyte concentration based on such as signal may be improved by taking the temperature of the solution into account.

It has been discovered that for some embodiments of the analyte sensors disclosed herein, and in particular, for glucose sensors employing a quencher binding moiety operably coupled to a fluorophore, the temperature dependence of the fluorescent signal approximately follows a modified version of the classic Michaelis-Menten equation from enzyme kinetics: $\left[Glu\right] = \frac{{c}_{T} ∗ ⌊ F - {a}_{T} ⌋}{{a}_{T} + {b}_{T} - F}$ where
*[Glu]* is the estimated glucose concentration
*F* is the fluorescent signal,
*a_{T}* is the Michaelis-Menten parameter "*a*", at a temperature T,
*b_{T}* is the Michaelis-Menten parameter "*b*", at the same temperature T, and *c_{T}* is the Michaelis-Menten parameter "*c*", at the same temperature T.

In itself, this is an interesting and surprising result. Various embodiments of the analyte sensors disclosed herein employ a quencher-fluorophore indicator system which measures analyte concentration through the establishment of an equilibrium between the analyte of interest, the binding moiety (e.g. quencher), and the fluorophore. In such a system, analyte concentration is not measured by enzymatic consumption or conversion of the analyte. In contrast, the classic Michaelis-Menten equation specifically describes enzyme kinetics, a non-equilibrium phenomena involving the consumption/conversion of the enzyme's substrate by the enzyme. Therefore, it is not to be expected that an equation closely related to the classic Michaelis-Menten equation would effectively describe the temperature dependence of these types of quencher-fluorophore based sensors (or other sensors functioning through analogous equilibrium mechanisms). In any event, knowledge that these sensors (and similar sensors) exhibit a temperature dependence which follows a modified Michaelis-Menten equation allows the use of temperature correction methods and algorithms to improve the accuracy of analyte concentration measurements. Such methods and algorithms are disclosed herein, along with devices which implement such methods and algorithms.

Accordingly, some embodiment methods of estimating an analyte concentration include generating a signal indicative of analyte concentration and a signal indicative of temperature using an analyte sensor. Since, in some embodiments, the signal indicative of analyte concentration exhibits the temperature dependence just described, in some embodiments, the signal indicative of temperature may be used to adjust the signal indicative of analyte concentration to correct for temperature dependence. Thus, in certain such embodiments, the methods further include transforming the signal indicative of the analyte concentration utilizing an equation of the form of a modified Michaelis-Menten equation, depending on Michaelis-Menten parameters, such as the parameters "*a*", "*b*", and "*c*", as described above.

The temperature dependence is exhibited through the Michaelis-Menten parameters *a_{T}, b_{T},* and *c_{T},* as indicated by the subscript "*T*" labeling these parameters. In some embodiments, the temperature dependence may need to be determined through a temperature calibration. Thus, in certain embodiment methods, the values of one or more of the Michaelis-Menten parameters may be set based on data which includes temperature calibration data and the signal indicative of a temperature.

For example, in some embodiment methods, the temperature calibration data may be generated by a temperature calibration method. The temperature calibration method may include selecting a first analyte test sensor, and creating and/or providing a set of at least three solutions of differing known analyte concentrations. In certain such embodiments, a first temperature is selected (*T1*), three solutions of the set of at least three solutions are heated and/or cooled to a temperature substantially similar to the selected first temperature, and a first set of at least three signals is generated using the first analyte test sensor, each signal indicative of the concentration of analyte in a different one of the three solutions at the first temperature. Measurements are then made at a second temperature. Thus, in certain embodiments, a second temperature is selected (*T2*), three solutions of the set of at least three solutions (each of the three may be the same or different than a solution chosen for the first temperature) are heated and/or cooled to a temperature substantially similar to the selected second temperature, and a second set of at least three signals is generated using the first analyte test sensor, each signal indicative of the concentration of analyte in a different one of the three solutions at the second temperature. Of course, more than three solutions may be used in either of these steps. And more than two temperatures may also be employed. Generally, the more solutions of differing concentration and the greater number of different temperatures that are employed, the greater the accuracy of the resulting calibration data.

Once the solutions having known analyte concentrations have been measured, and the first and second sets of at least three signals have been generated, in some embodiments, the sets of signals are used to determine (usually approximately) the relationship between one or more of the Michaelis-Menten parameters and temperature.

In some embodiments the first, second, and third sets of temperature calibration parameters may include a slope and an intercept relating temperature to the value of either the first, second, or third Michaelis-Menten parameter. However, equations of other forms may be selected to relate the first, second, or third Michaelis-Menten equation to temperature. In some embodiments, a quadratic or higher-order polynomial in temperature may be suitable and/or desirable.

Other methods for estimating analyte concentration which incorporate temperature correction features and temperature calibration steps are also disclosed herein. In some embodiments, these methods are similar to those already described above and incorporate similar features, however, additional features may also be disclosed and, in some embodiments, the disclosed methods may be more general and described in more general terms. Since there are many ways to feasibly implement the discoveries disclosed herein for use in estimating analyte concentration, the following additional methods are described in order to illustrate the breadth of implementations that are possible.

In some embodiments, for instance, a method of estimating an analyte concentration from a signal indicative of the analyte concentration may include transforming the signal using an equation of the form of a modified Michaelis-Menten equation wherein the values of one or more Michaelis-Menten parameters have been adjusted for temperature.

In some embodiments, for instance, a method of estimating an analyte concentration may include generating a signal indicative of the analyte concentration and generating a signal indicative of a temperature using an analyte sensor, and transforming the signal indicative of the analyte concentration utilizing an equation of the form of a modified Michaelis-Menten equation wherein at least one of the Michaelis-Menten parameters has been substituted with a calibration equation functionally depending on a set of one or more temperature calibration parameters and the signal indicative of temperature. One could refer to such an equation as a "substituted" modified Michaelis-Menten equation since the Michaelis-Menten parameters have been explicitly substituted with equations depending on one or more other variables-temperature and the temperature calibration parameters. However, although such a "substituted" equation exhibits a more complicated analytic form, it nevertheless will still express the basic functional relationships of the modified Michaelis-Menten equation.

### Calibration Process

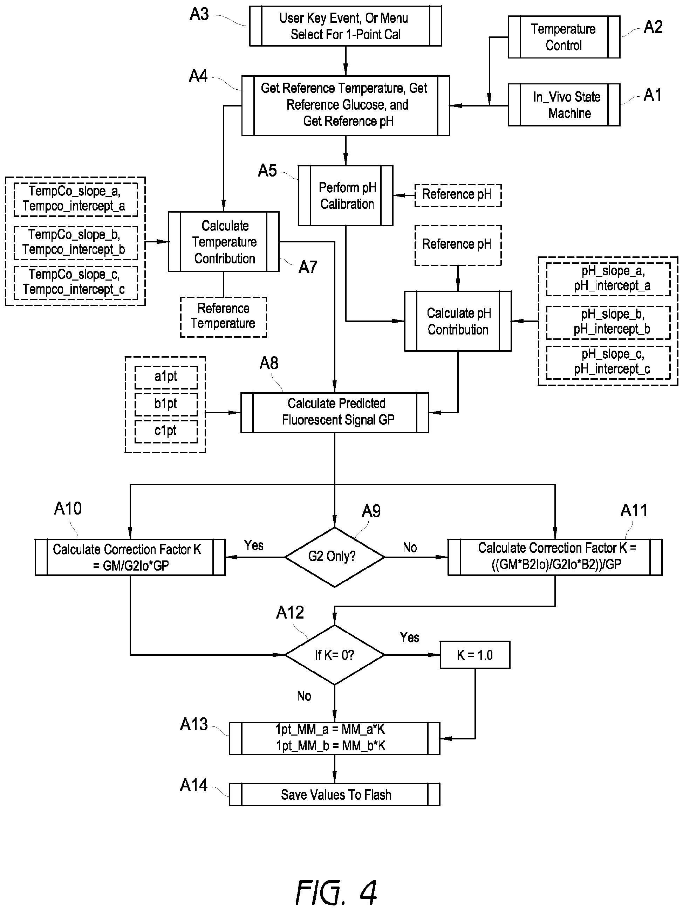

Various processes, algorithms, and steps can be used with the systems and methods disclosed herein. For example, **FIG. 4** is a flow chart showing a one-point calibration routine in accordance with an embodiment of the invention. In this embodiment, the analyte sensor is calibrated after it is placed *in vivo* in the subject as shown in **A1,** where the temperature is being controlled by a temperature control **A2.** The one-point calibration routine can also be conducted in an *in vitro* setting. In state **A3,** the user or an external signal selects the sensor and monitor combination, or the system, to initial a one-point calibration. In state **A4,** the system then acquires and/or receives reference signals for several parameters, including temperature, analyte concentration, and pH. The reference temperature can be measured by the reference thermocouple of the sensor, or can be a temperature reference signal generated by a separate signal generator. Similarly the analyte concentration reference signal can be generated by a reference analyte sensor that is separate from or combined with the main sensor assembly. The reference analyte concentration signal can also be generated by a separate signal generator, or it can be generated by measuring the portion of the excitation light source that is reflected against a mirror or an opaque surface. The reference pH signal can be measured by a pH sensor within the sensor assembly or separate, or it can be calculated based on the comparison of the two or more chemical indicator system emission intensity or profile. In another embodiment the reference signals are simply measured by a separate machine, such as a lab analyzer or other monitoring device that measures real time patient conditions.

In state **A5,** the system performs a pH calibration by acquiring the reference pH signal. Then in state **A6,** the system portion such as a pH contribution calculation module calculates the pH contribution calculated, for example, using the equations and algorithms disclosed herein. The calculation involves receiving the reference pH signal and calculating one or more slope and intercepts according to the calculations disclosed above. Concurrently, before, or after the state of **A5** and **A6,** the system portion such as a temperature calculation module calculates the temperature contribution in state **A7** using the equations and algorithms disclosed herein. The calculation involves receiving the reference temperature signal and calculating one or more slope and intercepts according to the calculations disclosed above.

In state **A8** the system calculates the predicted fluorescent signal GP based on the reference glucose measurement Gluref, where [GP = alpt + (blpt * Gluref)/ (clpt + Gluref)], and alpt, blpt, and clpt are coefficients, using for example the fluorescent signal prediction module. The pH contribution result calculated at **A6** and the temperature contribution result calculated at **A7,** and additionally one or more coefficients, which can include the Michaelis-Menten coefficients, can be referenced in state **A8.** In certain embodiments, the coefficients are predetermined at a factory, while in other embodiments, the coefficients are determined during the calibration process. In a preferred embodiment, the Michaelis-Menten coefficients are determined at manufacturing and adjusted via in vivo calibration.

In state **A9,** the system determined whether only the Green2 signal is being measured by, for example, a determination module. In certain embodiments, G2 is a compiler switch referring to Green2 signal. In one embodiment, the G2 Flag is set at compile time and never changed. For backward compatibility, the G2 Flag can be left in the system until a determination is made which equation to use, Green2 only, or Green2/Blue2 ratio. If the determination module determines that only Green2 signal is being measured, then only Green2 signal is used to perform the calculations and a calculation **A10** is executed by a calculation module to calculate the correction factor K according to Equation 18. If the determination module determines that not only G2 is being measured, the ratio between Green2 signal to Blue2 signal is used for calculations and a calculation **A11** is executed by a calculation module to calculate the correction factor K according to Equation 19. $K = GM / G 2 ⁢ I 0 * GP$ $K = \left(\left(GM*B2{I}_{0}/G2{I}_{0}*B2\right)\right) / GP$
GM = analyte measurement value entered by user
G2 = green signal detected by a second green detector
I₀ = value of G2 captured during a prior calibration (*e.g.* ex vivo calibration at 42°C)
B2 = blue signal detected by a second blue detector
GP = predicted fluorescent signal

After calculating the correction factor K, another determination is made at state **A12,** where the same or different determination module determines whether the correction factor K is zero or not. If the determination module determines K is not zero, it carries out calculation **A13** to calculate the modified Michaelis-Menten parameters using Equation 20 and Equation 21 below. In certain embodiments, each analyte sensor has specific Michaelis-Menten coefficients MM_A, MM_B, and MM_C. Of these coefficients, MM_A, and MM_B may be changed when a user performs a one point *in vivo* calibration. Once the *in vivo* calibiration is done, a correction factor (K), is calculated, which can be used to modify the factory set coeffecients of MM_A, and MM_B to obtain the 1pt_MM_A, and 1pt_MM_B. Thereafter, the system can use 1pt_MM_A, and 1pt_MM_B instead of MM_A, and MM_B to measure Glucose. If the determination module determines K to be zero, it selects 1.0 to be the correction value in the same calculation. The values determined by **A13** are subsequently saved to a memory at state **A14.** $1 ⁢ pt _ MM _ A = MM _ B * K$ $1 ⁢ pt _ MM _ B = MM _ B * K$
1pt_MM_A = modified Michaelis-Menten parameter A
1pt_MM_B = modified Michaelis-Menten parameter B
MM_A = Michaelis-Menten parameter A optionally programmed into sensor memory during manufacturing
MM_B = Michaelis-Menten parameter B optionally programmed into sensor memory during manufacturing

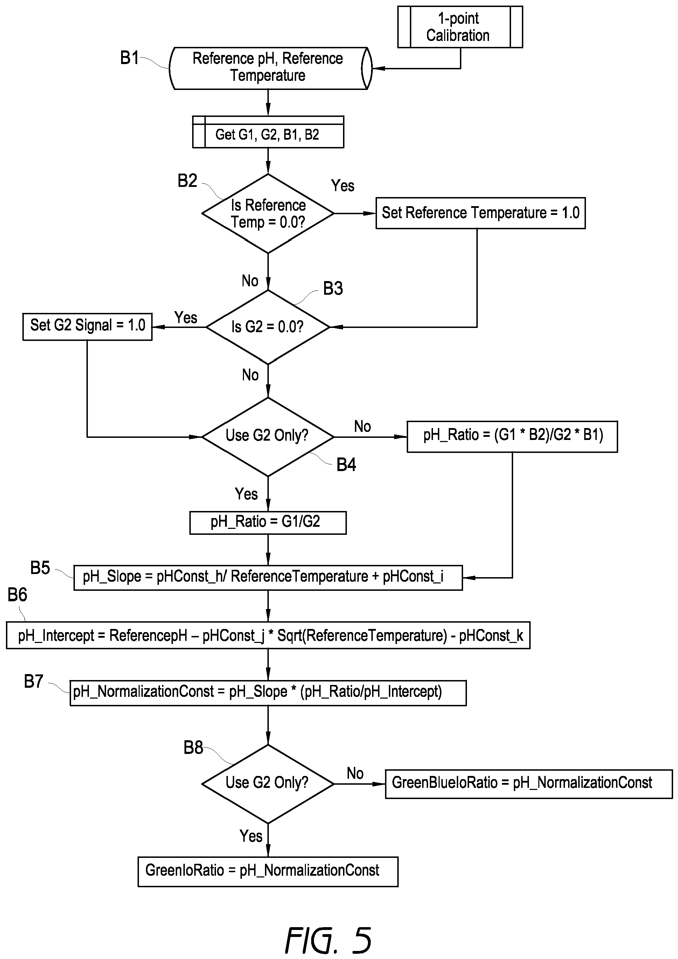

**FIG. 5** is a flow chart showing a pH calibration routine in accordance with an embodiment of the invention. For example, this pH calibration routine is carried out upon initiation or completion of the one point calibration described above. After obtaining or receiving the reference pH signal and reference temperature signal, coefficients are calculated at state **B1** according to the equations and algorithms disclosed above. The coefficients include the current from a first Green detector (G1), a second Green detector (G2), a first Blue detector (B1), and a second Blue detector (B2). The determination module determines whether the reference temperature is 0.0 or not at state **B2.** If the reference temperature is not 0.0, the system proceeds to state **B3** where the determination module determines whether G2 is 0.0. If the reference temperature is 0.0, a reference temperature of 1.0 is selected for the determination at state **B3.** If G2 is not 0.0 the system proceeds to state **B4** where the determination module determines whether to use G2 only. If G2 is 0.0, a G2 signal of 1.0 is selected for the determination at state **B4.** If only G2 is used, a selection module selects a first ratio equation, for example Equation 22, whereas if not only G2 is used, the selection module selects a second ratio equation, for example Equation 23. The calculation module thereafter calculates the pH ratio based on Equation 22 or 23. $pH _ Ratio = G 1 / G 2$ $pH _ Ratio = \left(G1*B2\right) / \left(G2*B1\right)$

Then at state **B5,** the calculation module calculates the pH slope based on an equation, for example Equation 24. At state **B6,** the calculation module calculates the pH intercept based on another equation, for example Equation 25. The various calculations of slopes, ratios, intercepts can be simultaneous or in any other order. At state **B7,** the calculation module calculates the pH normalization constant by correlating the pH slope, the pH ratio, and the pH intercept determined at the previous steps. Equation 26 is an exemplary equation for calculating the pH normalization constant. Other equations and algorithms referred to herein can also be used to determine these coefficients. In state **B8,** the determination module determines whether G2 is only being used. In other embodiments, the same determination at state **B4** is used instead. If only G2 is being used, a green I₀ ratio is calculated based on the pH normalization constant, whereas if not only G2 is being used, a green-blue I₀ ratio is calculated based on the pH normalization constant. $pH _ Slope = pHConst _ h / ReferenceTemperature + pHConst _ i$ $pH _ Intercept = ReferencepH - pHConst _ j*Sqrt \left(ReferenceTemperature\right) - pHConst _ k$ $pH _ NormalizationConst = pH _ Slope * \left(pH_Ratio/pH_Intercept\right)$

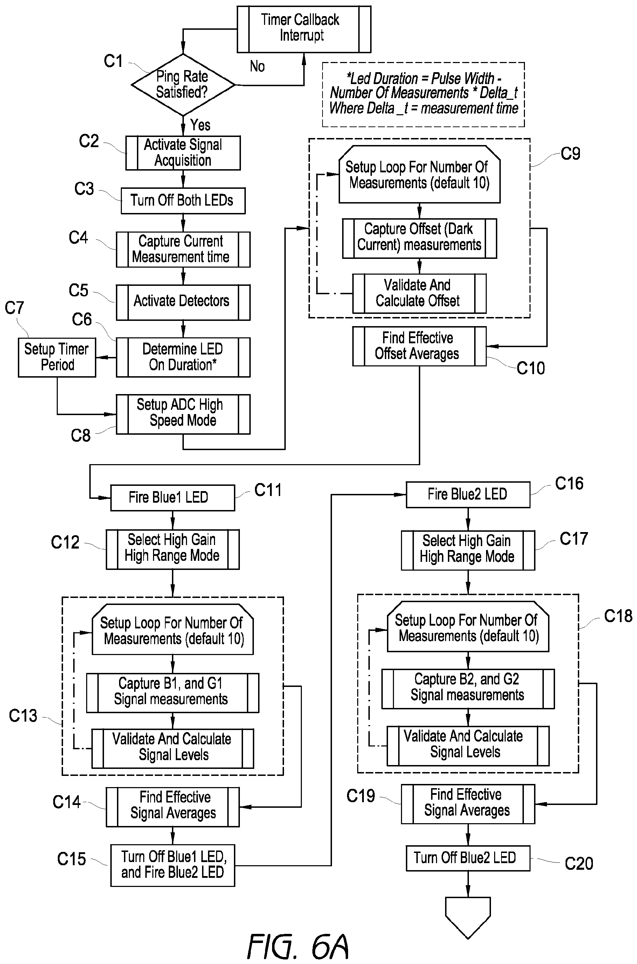

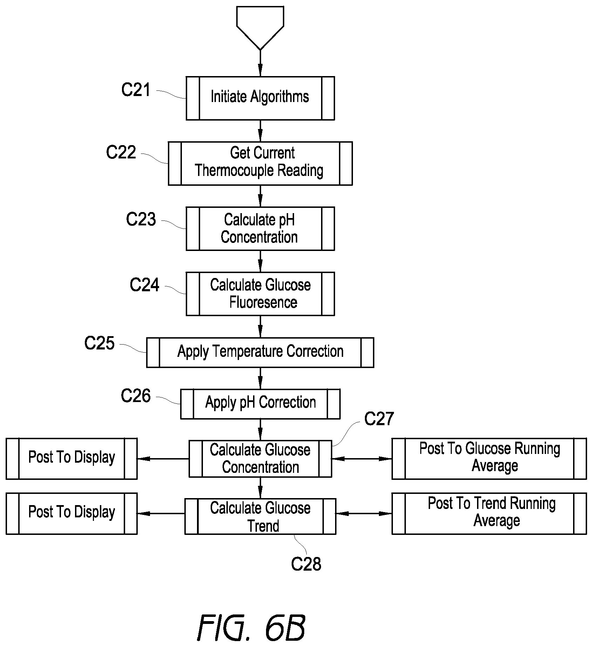

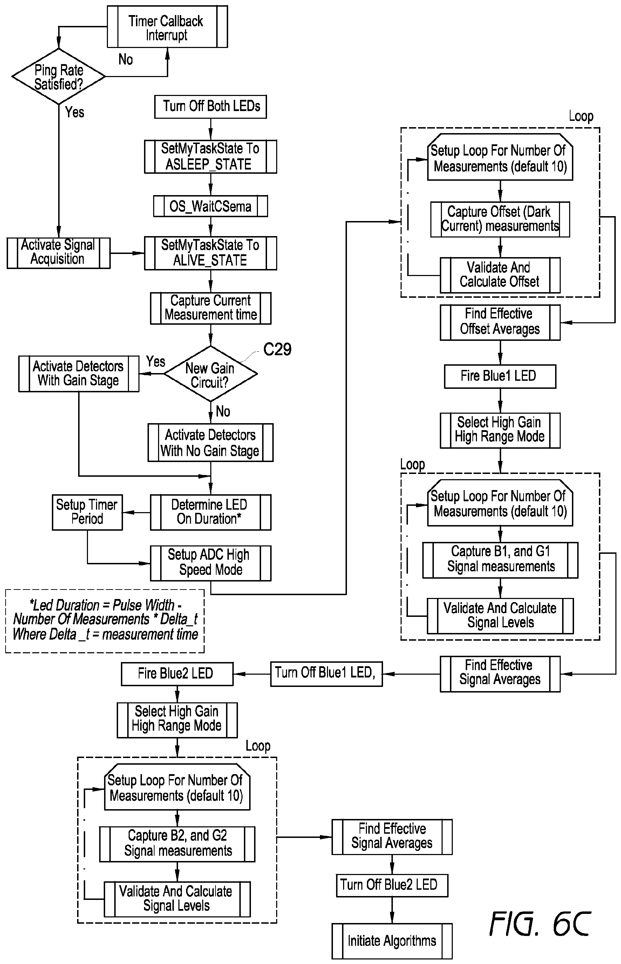

**FIG. 6A****,** **6B****, and** **6C** is a flow chart showing a signal acquisition routine in accordance with an embodiment of the invention. At state **C1** as shown in FIG. 6A, the system verifies whether the ping rate has been satisfied. If the system detects the ping rate is not satisfied, it will interrupt the timer callback. State **C1** can also include running quality checks on the system or monitor. Once the ping rate is verified, the signal acquisition is activated at **C2.** At **C3,** the one or more LEDs are turned off. In a preferred embodiment, two LEDs are present and both turned off. At **C4,** the current measurement time is captured and at **C5** the one or more detectors are activated. Then at **C6,** a determination module determines the LED duration, for example by suing Equation 27. A timer period can be setup accordingly at **C7** and the analogue to digital converter (ADC) can be set to high speed mode at **C8.** $LED Duration = Pulse Width - Number of Measurements*Delta _ t$ Delta_t = measurement time

The system then carries out a sequence of events in state **C9.** The system sets up a loop for a number of measurements. In one embodiment, the number of measurements is between 2 and 20. In a preferred embodiment, the number of measurement is 10. The system then detects the dark current, or the signal response produced by the detector without the emission source turned on. This dark current is captured as the offset and the system validates and calculates the offset amount. This process is repeated for the selected number of times. At state **C10,** the system calculates the effective offset average.

Next, in continued reference to **FIG. 6A****,** the system initiates the first emission source in state **C11.** In one embodiment, a first blue LED is fired at this state. In state **C12,** the system selects the high gain high range mode. In one embodiment, the system can have two or more gain settings, for example 10.09 and 27.7. There can also be two or more range settings for the Analog to Digital Converter (ADC), for example 1.024 volts and 0.256 volts. Thus, selecting the high gain and the high range can include choosing the maximum ADC range of 1.024 volts, and the amplifier gain of 27.7. In this embodiment, the system can determine based on this measurement, if it is necessary to make obtain measurements, or not. If signal levels are saturated with these settings, then either the gain, and/or the range can be lowered, thus another measurement can be obtained.

The system then carries out a sequence of events in state **C13.** The system sets up a loop for a number of measurements. In one embodiment, the number of measurements is between 2 and 20. In a preferred embodiment, the number of measurement is 10. The system then captures the signal measurements for B1 and G1. This system then validates and calculates the signal levels. This process is repeated for the selected number of times. At state **C14,** the system calculates the effective signal averages. The first emission source is turned off at **C15.**

Next, the system initiates the first emission source in state **C11.** In one embodiment, a first blue LED is fired at this state. In state **C12,** the system selects the high gain high range mode. The system then carries out a sequence of events in state **C13.** The system sets up a loop for a number of measurements. In one embodiment, the number of measurements is between 2 and 20. In a preferred embodiment, the number of measurement is 10. The system then captures the signal measurements for B1 and G1. This system then validates and calculates the signal levels. This process is repeated for the selected number of times. At state **C14,** the system calculates the effective signal averages. The first emission source is turned off at **C15.**

Next, the system initiates the second emission source in state **C16.** In one embodiment, a second blue LED is fired at this state. In state **C17,** the system selects the high gain high range mode. The system then carries out a sequence of events in state **C18.** The system sets up a loop for a number of measurements. In one embodiment, the number of measurements is between 2 and 20. In a preferred embodiment, the number of measurement is 10. The system then captures the signal measurements for B2 and G2. This system then validates and calculates the signal levels. This process is repeated for the selected number of times. At state **C19,** the system calculates the effective signal averages. The second emission source is turned off at **C20.**

In certain embodiments, the system comprises a third emission source, such as an LED at a different emission profile than the first and second LEDs. A similar gain and range adjustment followed by the measurement and validation cycle can be carried out to acquire the effective signal averages. In yet other embodiments, the system comprises a fourth or more emission sources, thus increasing the complexity of the system and improving the accuracy of the measurement system.

Next, in reference to **FIG. 6B****,** the system initiates the algorithms **C21** to calculate the analyte concentration. The algorithms include the various embodiments disclosed above, including the equations involving the Michaelis-Menten equations. In state **C22** the signals indicative of the temperature are acquired through the current thermocouple readings. Then at **C23,** the calculation module calculates the pH concentration according to the one or more pH measurement and calculation embodiments described herein. At **C24,** the calculation module calculates the analyte fluorescence. The temperature correction and pH correction are carried out at **C25** and **C26.** The calculation module then calculates the analyte concentration at state **C27** and displays the calculated value. Simultaneous the system can accumulate the analyte concentration measurement and calculate or post the running average. In one embodiment, the running average is obtained by an averaging filter, which takes a new value, and outputs the new calculated average. The averaging filter can use several window sizes for running an averaging module, for example three values for determining the average. Next, at state **C28,** the calculation module calculates the running average concentration trend so the operator can evaluate any trending of the glucose concentration in the subject and can take actions to stop or reverse trends that can be harmful to the subject. The calculated trend can also be displayed on the display screen. Simultaneously the system can accumulate the analyte trend information and calculate or post the running average. In one embodiment, the calculated pH or Glucose are first sent to an averaging filter, for example to calculate a three point running average, and the result of the three sample average is sent back to the calling routine. Then the three sample average is sent for displaying. Thus a glucose concentration is calculated, the glucose concentration is sent to the three point running average to get the average, the glucose average is sent to the display for viewing, a glucose trend is calculated and is sent to a six point running average to get the average, and the glucose trend is sent to the display for viewing.

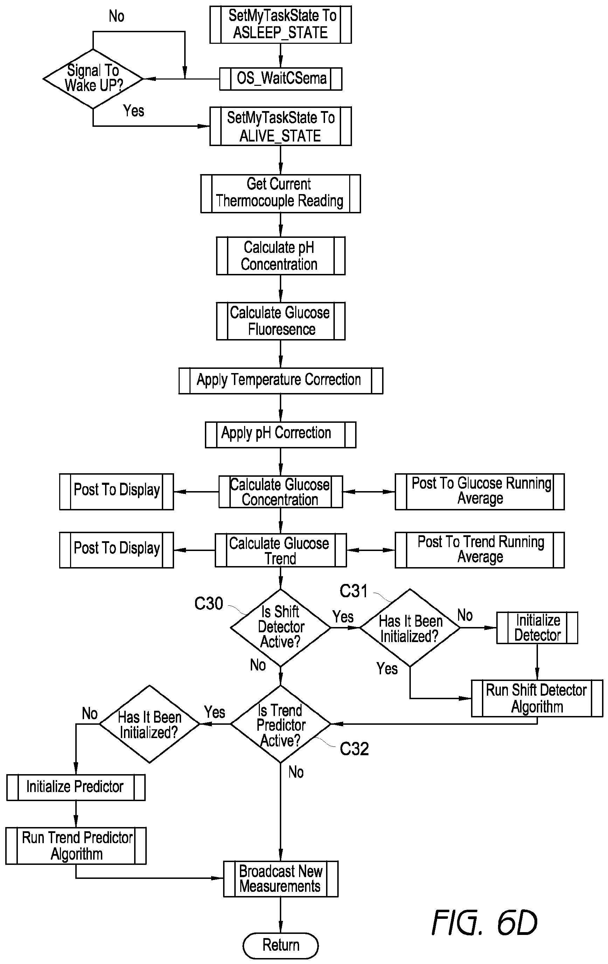

**FIG. 6C** and **6D** also show the signal acquisition routine in accordance with another embodiment of the invention. In this embodiment, the LEDs can be turned off and the monitor can be put into a sleep state while the system awaits a ping command or activation of the LED. When the signal acquisition is activated, the monitor is put into an alive state. After the current measurement time is captured, the monitor can determine whether a new gain circuit is applicable at state **C29.** Based on the determination, the system can activate the detectors with a gain stage, or without a gain stage. The system can also determine whether the shift detector is active at state **C30.** If the shift detector is active, the system can verify the shift detector is initialized at state **C31,** thereafter initialize the shift detector as needed and run the shift detector algorithm. The system can also determine whether the trend predictor is active at state **C32** and initialize the predictor as well as run the trend predictor algorithm.

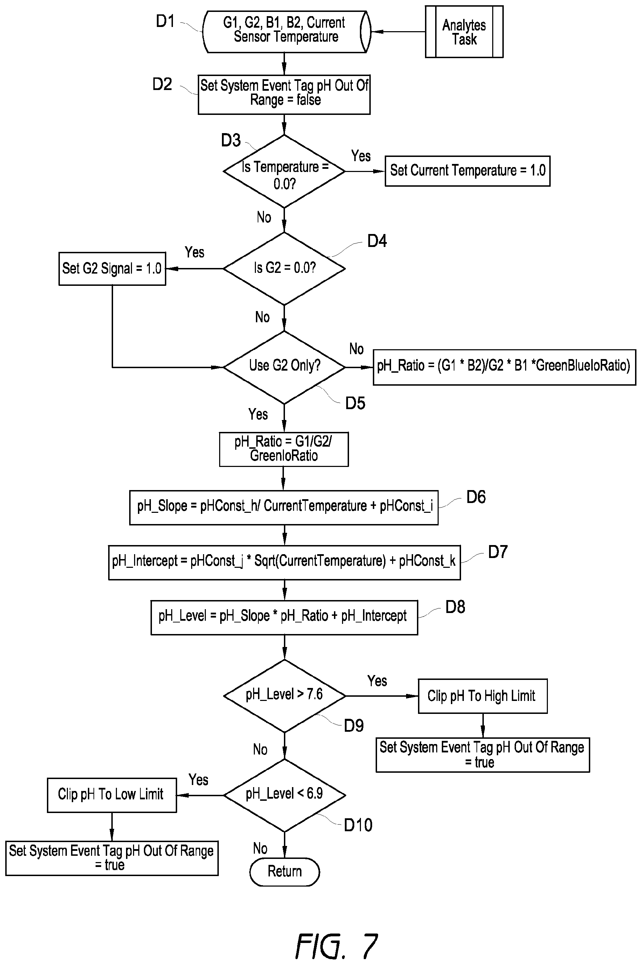

**FIG. 7** is a flow chart showing a pH calculation processing routine in accordance with an embodiment of the invention. In state **D1,** the system receives from an earlier process information including G1, G2, B1, B2, and current sensor temperature. Then in state **D2,** an event tag that indicates "pH out of range = false" is set. In state **D3,** the determination module decides whether the temperature is 0.0, and if yes the system sets the current temperature as 1.0. Next, in state **D4,** the G2 the determination module decides whether the G2 is 0.0, and if yes the system sets the G2 signal as 1.0.

In state **D5,** the determination module determines whether only G2 is used. If only G2 is used, the determination module selects a first ratio equation, for example Equation 28, whereas if not, the selection module selects a second ratio equation, for example Equation 29. The calculation module thereafter calculates the pH ratio based on Equation 28 or 29. $pH _ Ratio = G 1 / G 2 / GreenIoRatio$ $pH _ Ratio = \left(G1*B2\right) / G 2 * B 1 * GreenBlueIoRatio )$

Then at state **D6,** the calculation module calculates the pH slope based on an equation, for example Equation 30. At state **D7,** the calculation module calculates the pH intercept based on another equation, for example Equation 31. The various calculations of slopes, ratios, intercepts can be simultaneous or in any other order. At state **D8,** the calculation module calculates the pH level by correlating the pH slope, the pH ratio, and the pH intercept determined at the previous steps. Equation 32 is an exemplary equation for calculating the pH normalization constant. Other equations and algorithms referred to herein can also be used to determine these coefficients. In state **D9** and **D10,** the determination module determines whether the pH level is greater than 7.6 or less than 6.9. This can indicate that the subject has a blood pH level that is outside a safe or ideal physiological state. If it is greater than 7.6 or less than 6.9, it will process a signal indicating the pH is above the high limit or below the low limit, respectively, and thereafter set the system event tag indicating the pH is out of range. This feature can also have the effect of indicating to the user, the accuracy of the chemical indicator system of the analyte sensor may be affected if the analyte sensor is exposed to this pH level for extended periods. For example, the chemical indicator system may be bleached quicker or slower as compared to an optional compensation algorithm depending on the pH. In other embodiments, the threshold pH levels are a tighter range, for example 7.1 and 7.4. In yet other embodiments, the threshold pH levels are a wider range, for example 7.8 and 6.7. $pH _ Slope = pHConst _ h / CurrentTemperature + pHConst _ i$ $pH _ Intercept = pHConst _ j * Sqrt \left(CurrentTemperature\right) + pHConst _ k$ $pH _ Level = pH _ Slope*pH _ Ratio + pH _ Intercept$

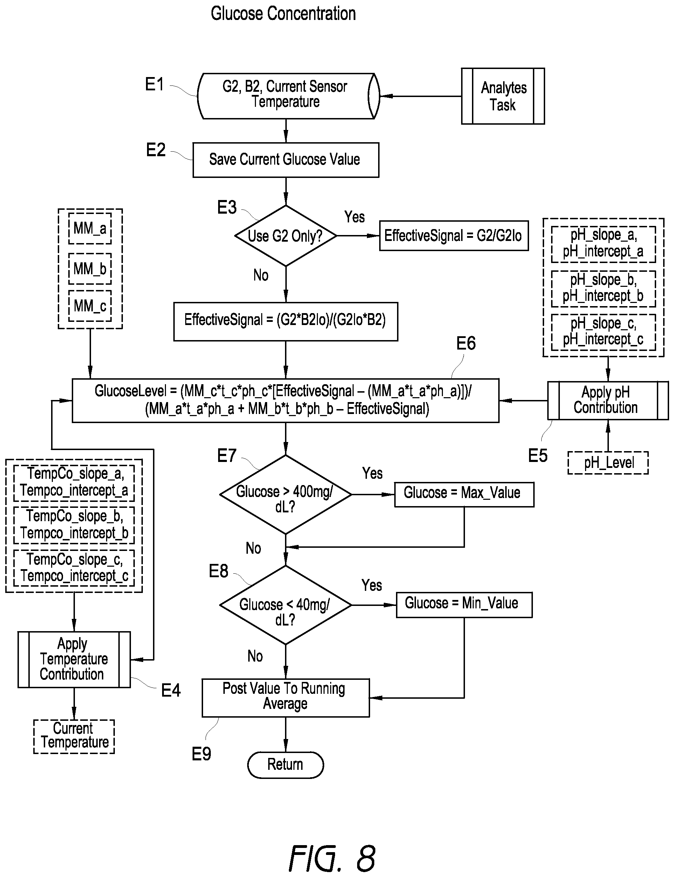

**FIGS. 8****,** **9****,** and **10** is a flow chart showing a processing routine in accordance with an embodiment of the invention. In state **E1** of **FIG. 8****,** the system receives from earlier process information including G2, B2, and current sensor temperature. Then in state **E2,** the current glucose value is saved. In state **E3,** the determination module determines whether only G2 is used. If only G2 is used, the determination module selects an effective signal according to an equation, for example Equation 33, whereas if not, the selection module selects another equation, for example Equation 34. The calculation module thereafter calculates the effective signal based on Equation 33 or 34. $EffectiveSignal = G 2 / G 2 ⁢ Io$ $EffectiveSignal = \left(G2*B2Io\right) / \left(G2Io*B2\right)$

Then at state **E4,** the calculation module calculates the temperature contribution using the current temperature and the temperature slope and intercept. At state **E5,** the calculation module calculates the pH contribution using the pH measurement and the pH slope and intercept. At state **E6,** the temperature contribution and pH contribution are used to calculate the glucose level according to another equation, for example Equation 35. The calculation module can carry out these calculations. At state **E6,** the calculation module can also receive the Michaelis-Menten coefficients represented as MM_A, MM_B, and MM_C. These Michaelis-Menten coefficients can be programmed into the sensor memory in manufacturing, or at run time. These values can be stored in the processor flash memory and recalled when needed. MM_A and MM_a refer to the same Michaelis-Menten coefficient "a". ph_a referrers to the pH correction that can be applied to MM_a. Similarly, t_a is the temperature correction that can be applied to MM_a. Similarly, ph_b and t_b, and ph_c, and t_c can be applied to MM_b, and MM_c respectively. The coefficients t and ph are representative of the temperature and pH contribution to the analyte concentration measurement, because temperature and pH can affect the analyte concentration measurement. Each parameter's contribution is reflected by changing the Michaelis-Menten coefficients respectively. So t_c can imply temperature contribution on Michelis-Menten coefficient C, (MM_c), and ph_c can imply the pH contribution to MM_c. Other equations and algorithms referred to herein can also be used to determine these coefficients and values. $GlucoseLevel = \left(MM_C*t_c*ph_c*\left[EffectiveSignal-\left(MM_A*t_a*ph_a\right)\right]\right) / \left(MM_A*t_a*ph_a+MM_B*t_b*ph_b-EffectiveSignal\right)$

At state **E7,** the determination module determines whether the glucose concentration is greater than a high threshold amount, for example 400 mg/dL. At state **E8,** the determination module determines whether the glucose concentration is below the low threshold amount, for example 40 mg/dL. If the glucose concentration exceeds the high or low threshold amounts, the system can set an event tag indicating the glucose concentration is out of range. This can cause an alarm to sound or cause the display to indicate it is out of range, for example by flashing the value, changing colors to red, or displaying a warning sign. In one embodiment, the system can display that the glucose is at the max value or the minimum value. This can indicate that the subject has a blood glucose level that is outside a safe or ideal physiological state. In other embodiments, the threshold glucose levels are a tighter range, for example a low threshold of 70 mg/dL and a high threshold of 180 mg/dL. In yet other embodiments, the high threshold glucose level is between 270 and 360 mg/dL. In yet still other embodiments, the low threshold glucose level is between 50 to 60 mg/dL. In state **E9,** the value is accumulated or posted the running average glucose concentration measurements.

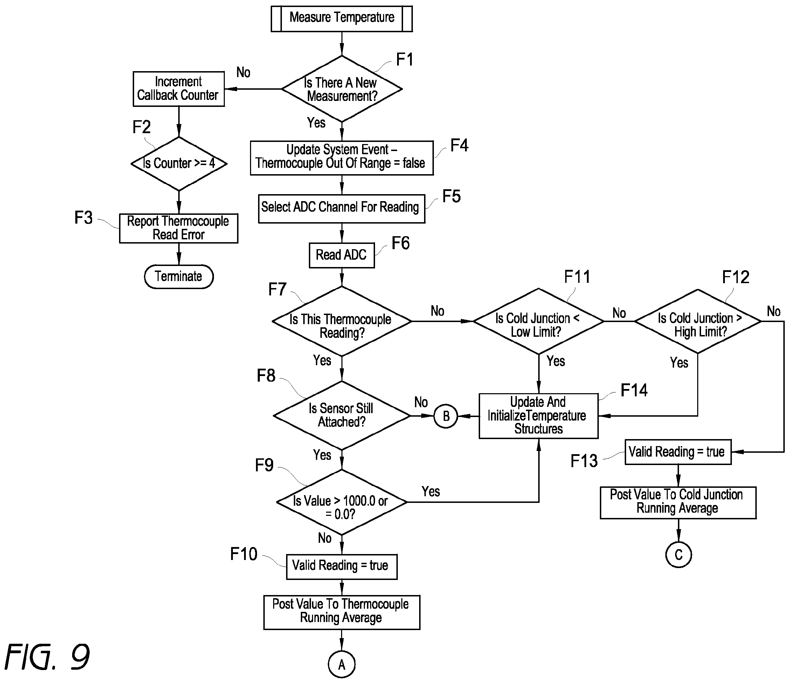

In **FIG. 9****,** the temperature can be measured in accordance with the methods and systems described herein. At state **F1,** the determination module determines whether there is a new temperature measurement. If not, at state **F2,** the determination module receives the signal from the increment callback counter and if the counter number is greater than or equal to four, a thermocouple read error is reported at **F3** and terminates the routine. If the determination module determines there is a new measurement, at state **F4** the system event is updated so that it shows the thermocouple is not out of range. In state **F5,** the system selects the analog to digital converter (ADC) channel for reading, and at state **F6,** the signal from the ADC is read.

Next, in continued reference to **FIG. 9****,** the determination module determines at state **F7** whether the ADC reading is the thermocouple reading. If it is the thermocouple reading, the determination module next determines at state **F8** whether the sensor is still attached. If the sensor is attached, the determination module next determines at state **F9** whether the ADC reading is greater than 1000 or greater, or is equal to zero. If neither is true, the ADC reading is considered a valid reading at **F10** and the value is posted to the thermocouple reading data set, for example to calculate, store, and/or display running averages. However, if at state **F7** the determination module determines the ADC reading not to be a thermocouple reading, it next determines at state **F11** whether the cold junction is less than a lower limit. If the cold junction is not less than the lower limit, then it next determines at state **F12** whether the cold junction is higher than the upper limit. If the cold junction is not higher than the upper limit, the ADC reading is considered a valid reading at **F13** and the value is posted to the cold junction reading data set, for example to calculate, store, and/or display running averages. If on the other hand, the ADC reading is determined to be greater than 1000 or equal to zero (**F9**), or the cold junction is below the lower limit or above the higher limit (**F11**, **F12**), then at state **F15,** the system updates the temperature structures.

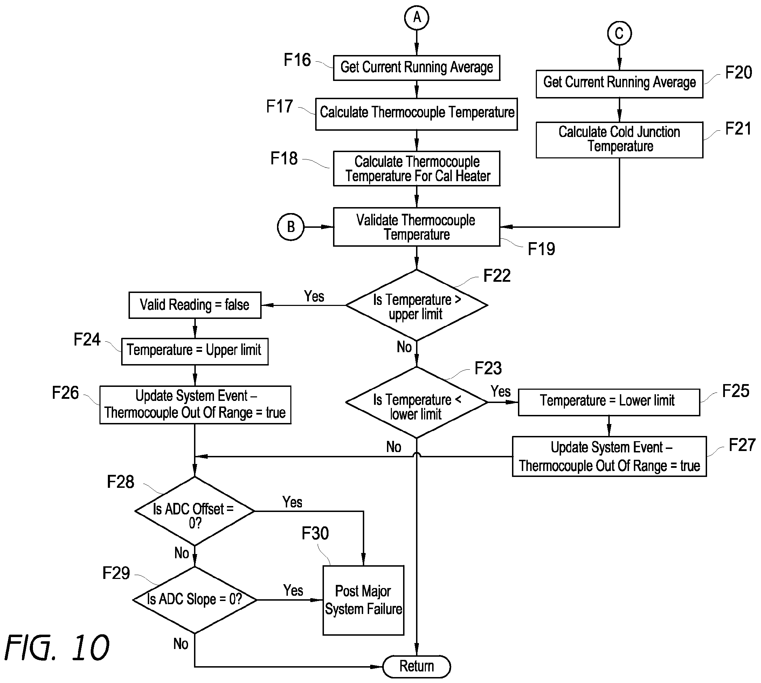

**FIG. 10** is a flow chart showing the temperature calculation routine in accordance with an embodiment of the invention. In one embodiment, this routine is carried out as an extension of the temperature calculation routine of **FIG. 9****.** The point of initiation of the second temperature calibration routine can depend on the result of the routine per **FIG. 9****.** For example, if the earlier temperature calculation routine ends with the posting of the thermocouple value (**A**), then on the second temperature calibration routine, the system starts at state **F16** by receiving or acquiring the current ADC reading running average. Then at state **F17,** the thermocouple temperature is calculated according to the methods disclosed herein. Then at state **F18,** the thermocouple temperature for the calibration heater is calculated. Thereafter, at state **F19** the thermocouple temperature is validated. In the alternate, if during the earlier temperature calculation routine of **FIG. 9** ends with the posting of the cold junction value (**C**), then on the second temperature calibration routine, the system starts at state **F20** by receiving or acquiring the current ADC reading running average and then at state **F21** calculating the cold junction temperature, followed by validating the thermocouple temperature at state **F19.** In yet another alternate, if the earlier temperature calculation routine of **FIG. 9** ends with the updating of the temperature structures or with a determination that the sensor is no longer attached (**B**), then on the second temperature calibration routine, the system starts at state **F19,** to validated the thermocouple temperature.

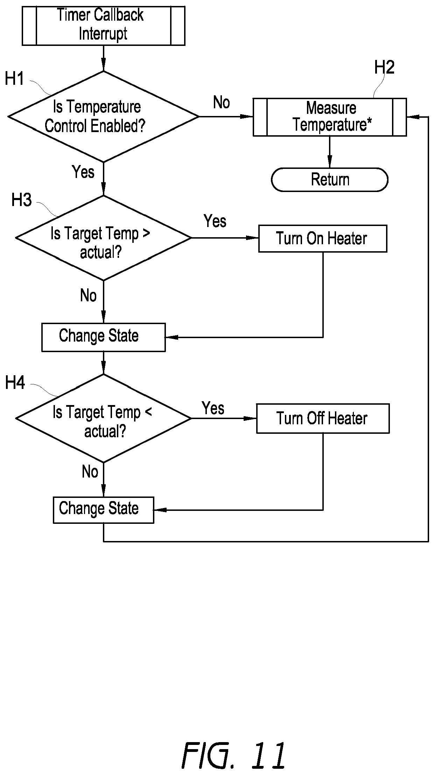

In continued reference to **FIG. 10****,** after validating the thermocouple temperature, at state **F22** the determination module determines if the temperature is greater than the upper limit. If the temperature is not greater than the upper limit, then at state **F23** the determination module determines if the temperature is less than the lower limit. If the temperature is not less than the lower limit, it returns the caller (*e.g.* a state machine) as shown in FIG. 11. The state machine can be called every 100ms to modulate the heater, and then it calls Measure Temperature in FIG. 9. This can be a function call, and when the function is done measuring temperature, it can return from activity to an idle state, until it is called again. If the determination module determines the temperature to be above the upper limit or below the lower limit at states **F22** and **F23,** the system generates a signal indicating the readings are no longer valid and that the temperature is above the upper limit (**F24**) or below the lower limit (**F25**). In state **F26** or **F27,** the system also updates the system event to indicate the thermocouple is out of range. If the determination module determines the ADC offset is not zero (**F28**) and the slope is not zero (**F29**), it will return to the state machine. If either the ADC offset or the ADC slope is determined to be zero, then at state **F30** a signal indicative of a system failure is stored or posted.

**FIG. 11** is a flow chart showing a temperature sensor control routine in accordance with an embodiment of the invention. A timer callback interrupt is carried out. In state **H1,** the determination module determines whether the temperature control is enabled. If the temperature control is not enabled, the temperature is measured at state **H2** and is returned to the state machine. The temperature measurement can be a function which is called within the state machine, and when the function completes its tasks, it can return to its caller. If on the other hand the temperature control is enabled, next in state **H3** the determination module determines if the target temperature is greater than the actual temperature. If the target temperature is greater than the actual temperature, the system turns on the heater. Next, in state **H4,** the determination module determines if the target temperature is less than the actual temperature, and if it is, the system turns the heater off. These steps also involve the system changing the state of the state machine. The temperature control state machine can have two or more states within it, for example one to turn the heater on, and the other to turn the heater off. The states can be changed from one to the other every time the state machine is called via a 100 ms timer interrupt. At power up, the state is set to turn on the heater, so when the state machine is invoked by the 100 ms timer, it will have a place to start from. From there on, the state machine can itself modify its own state to the next valid one. Then, the system can measure the temperature according to state **H2.**

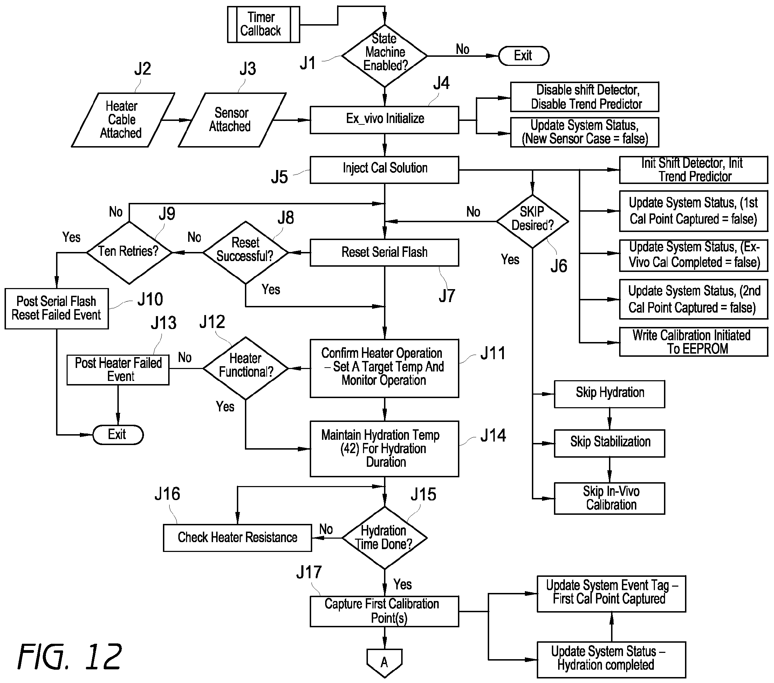

**FIG. 12** is a flow chart showing an ex vivo calibration routine in accordance with an embodiment of the invention. After the timer callback, at state **J1** if the determination module determines the state machine is not enabled, it does not proceed to executing the ex vivo calibration. The state machine can be a routine that maintains a finite number of states, or functionalities that it can perform. Each specific state can define the exact steps the routine will execute. For example the temperature state machine can have two states. In one state it turns on the heater if temperature is less than a target, and in the other state, it turns off the heater if it is over the target. Assume temperature state machine states are A, and B. Inside state A, temperature is tested against the target, and if it is less than the target temperature, then heater is turned on, and the future state is set to B. This means that the next time the state machine runs, it will execute state B. In state B, the temperature is checked against the target, but if it is higher than the target, heater is turned off, and future state of the state machine is set to A. Note that even if the temperature matches the target, and heater is not turned on or off, the future state is always changed from A to B inside state A, and from B to A inside state B. An optional heater cable is attached to the calibration area (**J2**), and the sensor is attached to the monitor system (**J3**). At state **J4,** the user or the system based on a predetermined condition initializes the ex vivo calibration. This can cause the shift detector and the trend predictor to be disabled, as well as updating the system status. Then, in state **J5,** the calibration solution, for example a sterile phosphate buffer or carbonate buffer, is injected into the calibration chamber. This can cause the shift detector to be initiated and writing to the analyte sensor memory that the calibration has been initiated, as well as updating the system status for the calibration process. In state **J6** the system then determines whether to skip certain steps, such as the hydration of the analyte sensor, stabilization of the signals of the analyte sensor, and the in vivo calibration routine.

Thereafter, in sate **J7** the serial flash or other memory module is reset and if the rest is not successful (**J8**) after several tries (**J9**) the system notes that the serial flash reset has failed (**J10**) and will exit the calibration routine. After the search flash is successfully reset, in state **J11**, the system confirms heater operation by setting a target temperature and monitoring the operation. The system checks the heater is functions (**J12**) and if it is determined to be not functioning, the system will note the post heater has failed (**J13**) and will exit the calibration routine. If the heater is functional, in state **J14** the system will maintain the hydration temperature, for example 42°C, for the hydration duration, for example 10 minutes. In other embodiments, the hydration temperature is between 37°C and 50°C. In yet other embodiments, the hydration temperature is between 22°C and 35°C. In still other embodiments, the hydration duration is between 1 and 8 minutes. In yet still other embodiments, the hydration duration is more than 30 minutes. Next, in state **J15** the determination module periodically checks whether the hydration time is done. If the hydration time is not done, the system checks the heater resistance (**J16**) and continues the hydration cycle. If the hydration time is done, at state **J17** the one or more of the first calibration points is captured. This can also cause the system to update the system event tags to indicate the first calibration points are captured and that the hydration is complete.

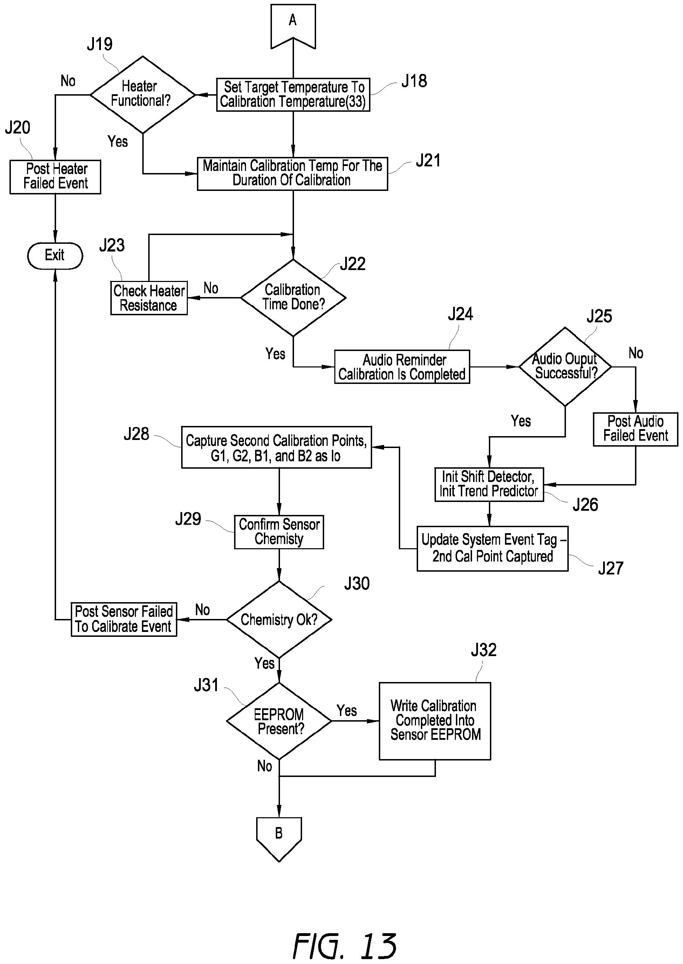

**FIG. 13** is a continued flow chart showing the second sequence of the ex vivo calibration routine. After capturing the first calibration points, in state **J18** the system sets the target temperature to the calibration temperature, for example 33°C. The system optionally checks whether the heater is functional (**J19**) and if it is determined to be not functioning, the system notes the heater failed (**J20**) and exits the calibration routine. Next, in sate **J21,** the system maintains the calibration temperature for the duration of the calibration process. The determination module then periodically checks at state **J22** if the calibration time is done. If the calibration time is not done, the system checks the heater resistance (**J23**) and continues the calibration cycle. If the calibration time is done, in state **J24** an audio reminder or alarm is initiated to indicate the calibration is completed. An option audio output checker verifies the audio reminder was successful, and if it determines it was not successful, notes an audio failure event to the monitor. Next, in state **J26,** the system initiates the shift detector and initiates the trend predictor (**J26**) and updates the system event tag to show the second calibration point is captured (**J27**).

Next, in state **J28,** the second calibration points are captured as G1, G2, B1, and B2 as I₀, or the intercept of the sensor intensity. Then in state **J29,** the system confirms the sensor chemistry is properly functioning, for example by verifying the raw intensity, the I₀, or the slope. If the determination module determines the chemistry to be compromised at state **J30,** the system notes the sensor failed calibration and terminates the calibration routine. In another embodiment, the user has the option to repeat the entire, or a portion of the calibration sequence. If the chemistry is to be found acceptable, the system verifies if a memory is present at state **J31** and then writes the calibration information at state **J32,** including any calibration coefficients and the I₀ and slope, into the memory. The memory can include an EEPROM or other nonvolatile memory.

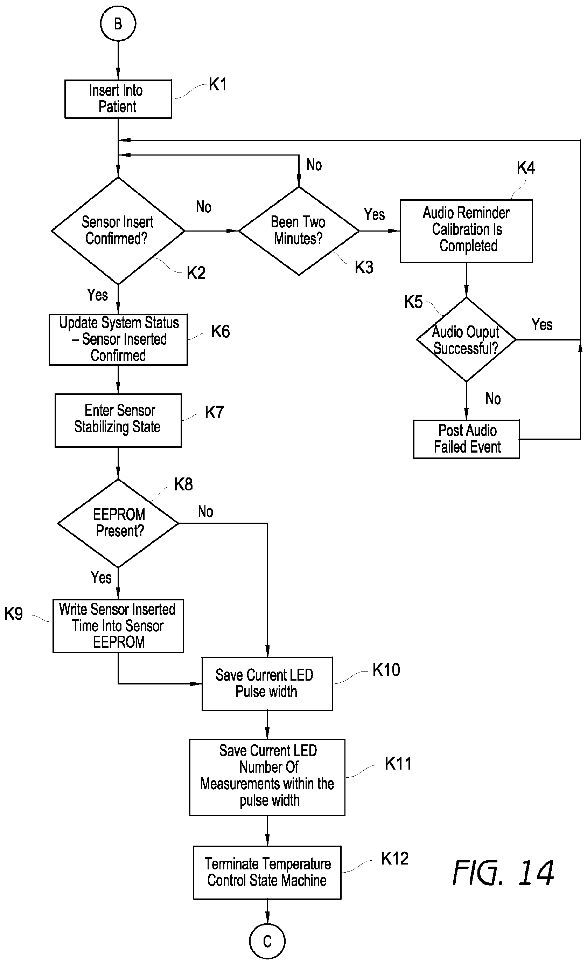

**FIG. 14** is another continued flow chart showing a third sequence of the ex vivo calibration routine. After verifying the chemistry is acceptable and writing information to the memory, the analyte sensor is taken out of the calibration fluid, for example the calibration chamber is removed, or the calibration solution is simply drained from the analyte sensor deployment device that comprises a calibration solution holding portion. Various deployment devices are described in US Patent Publication No. 2009/0264719, U.S. Application No. 13/095,748 filed April 27, 2011, U.S. Provisional Application No. 61/378,728 filed Aug. 31, 2010 and 61/328,590 filed April 27, 2010. Then in **K1** the sensor is inserted into the patient, such as through a cannula in the subjects arm. Next in state **K2** the determination module determines whether the sensor is inserted. In one embodiment, the user confirms the sensor is inserted by pushing a button or activating a command. In another embodiment, a determination module monitors the change in temperature, pH, or analyte concentration, or the signals indicative of such parameters, and based on the rate or direction of change, confirms that the sensor is inserted. In yet another embodiment, the determination module monitors the temperature, pH, or analyte concentration, or the signals indicative of such parameters, and if the value or signal level comes within a predetermined range, it confirms that the sensor is inserted. When the sensor insertion is not confirmed in **K2** for more than two minutes per a second determination step **K3,** the audio reminder again is activated (**K4**) to indicate the calibration is complete, followed by a verification step that the audio output was successful (**K5**). One the sensor insertion is confirmed, in state **K6** the system updates the status to indicate the insertion is confirmed.

Next, in state **K7** the system enters a sensor stabilizing state to allow the analyte sensor readings, the temperature readings, the pH readings, as well as other parameters and also the mechanical aspect of the analyte sensor, all to stabilize. The system also checks if the analyte sensor memory is present in state **K8** and if it is present, writes the sensor insertion time to the memory (**K9**). Then, in state **K10** the system saves the current LED pulse width, followed by saving the current number of measurements within the pulse width at state **K11.** In state **K12,** the temperature controller or machine is also terminated.

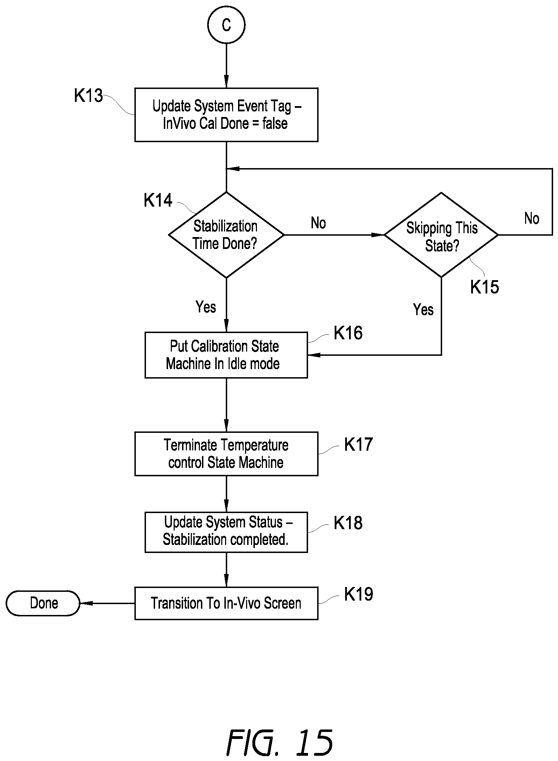

**FIG. 15** is another continued flow chart showing a fourth sequence of the ex vivo calibration routine. After the temperature control or machine is terminated, in state **K13** the system updates the system event tag. For example, the system notes that the in vivo calibration is not done. In state **K14,** the determination module determines if the stabilization time is completed, and if not, it allows the user to select or based on predetermined conditions to skip this step per step **K15.** Next, in state **K16** the calibration apparatus is put in idle mode (**K16**), the temperature controller or machine is terminated if it has not been earlier (**K17**), and the system status is updated to indicate the stabilization process is completed (**K18**). Thereafter, in state **K19** the system transitions to the in vivo operation system.

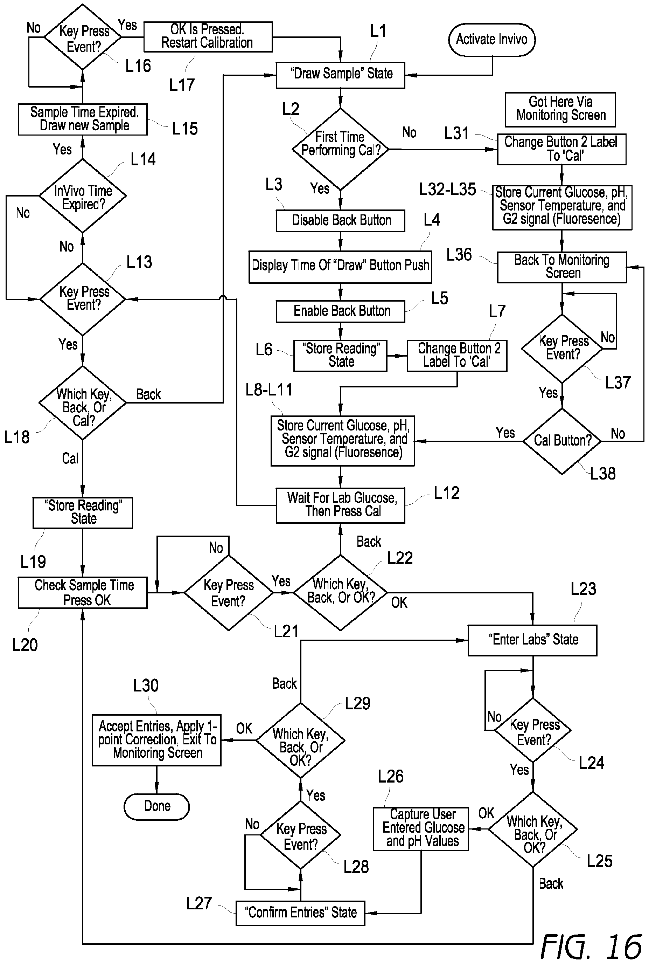

**FIG. 16A** is a flow chart showing an in vivo state routine in accordance with an embodiment of the invention. This routine can be used to calibrate the analyte sensor once it is inserted into the patient and an independent analyte measuring system is available to provide comparative data. After activating the in vivo operation, for example by pressing a button or based on an automated feature that detects the insertion of the analyte sensor into the subject's blood vessel, the user can draw a sample from the subject at state **L1,** such as blood. This sample can be measured by a reference device, for example a laboratory glucose measurement system. The user can also push the button, for example on the user interface of the monitor, to indicate a sample has been drawn. The user interface can have several buttons to carry out different functions, or in some embodiments the same button can have multiple functions depending on the routine and/or status.

Next, in state **L2** the determination module determines whether this is the first time performing the calibration. In this step, the user can be prompted to select Yes or No, or the system has a memory that has information indicating whether a calibration has been previously performed on the analyte sensor or not that is communicated to the determination module. The analyte sensor can have a memory that stores information indicating whether it has been previously put through an in vivo calibration routine. If it is the first time performing calibration, the button for returning or going back is disabled (**L3**), and the time the draw button was pushed is displayed on the display screen (**L4**). The time the draw button was pushed can also be saved to the monitor as well as the memory of the analyte sensor. Likewise, the time the activate button was pushed can also be saved. The button for returning or going back can be enabled at this state (**L5**). The user can, for example, press the button to return to an earlier state to draw a new sample from the subject. The system is then placed in the reading state and the reading state can also be stored or saved (**L6**). The reading state can mean where the monitor will read the internal signals such as the Blue 2 detector signal, and the Green 2 detector signal, current thermocouple temperature, and time of the readings. This information can be used in conjunction with the blood Glucose, and pH values entered by the user to determine the 1-point calibration factor. The resulting calibration factor can used to modify the Michaelis-Menten coefficients.

Next, in state **L7,** the button that was used for indicating a sample has been drawn changes label to "Cal" for calibration. In another embodiment, a different button for calibration is activated. The current glucose value (**L8**), the current pH concentration (**L9**), the current sensor temperature (**L10**), the raw fluorescent signal (**L11**) are stored to a memory. When the reference device measurement is received, for example the lab glucose values, the user can press the calibration button (**L12**). The system will determine whether the button was been pressed or not (**L13**) and if the user does not press the calibration button for a predetermined time, such that the in vivo calibration time is determined to be expired (**L14**), the system will time out and will require the user to draw a new sample (**L15**). If the system times out, once the user pushes a button to proceed (**L16**), the system will restart the calibration (**L17**) by returning to the step of drawing a sample (**L1**). The time out function prevents the user from performing the in vivo calibration based on a defective sample measurement, for example when the blood sample glucose level has changed from glycolysis due to high white or red blood cell counts.

If system will determines the button was been pressed (**L13**), the determination module next determines whether the back button or calibration button was pressed (**L18**). If the user pressed the back button within the specified time period, the system will return to state **L1** for drawing a sample. If the user pressed the calibration button within the specified time period, the system will store or save the readings state (**L19**). Thus, the Blue 2 detector signal, Green 2 detector signal, temperature, and time are saved internally for later use if the user pushes the calibration button within a specified time period, for example 90 minutes. Otherwise these values are discarded, and the user is asked to redraw blood and repeat the calibration process and the monitor will also capture a new set of detector signals, temperature, and time. The system then can prompt the user to verify the sample was drawn and tested within sufficient time (**L20**). In other embodiments, the system can automatically determine based on the stored time of pressing the draw button and the stored time of pressing the calibration button. At state **L21** determination module determines which button was pressed by the user and at state **L22** the system directs the system to return to state **L12** if the back button is pressed, or carry forward if the user confirms the sample was drawn and tested within sufficient time.

Once the user verifies the sample was drawn and tested within sufficient time, the system enters the laboratory state **L23.** The user then is prompted to press a button (**L24**) and the determination module determines whether the back button or the proceed button is pressed (**L25**). If the back button is pressed, it returns to an earlier state, for example **L20.** If the proceed button is pressed, the user can enter the measurements received from the reference device into the monitor or system (**L26**). For example, the analyte concentration including glucose values and other measurements such as pH values can be entered. In another embodiment, the system receives these data from an external device. The user is then required to confirm the entry of the data (**L27**) and press a button (**L28**). In state **L29,** the determination module determines if the user pressed the button to return to an earlier state, for example state **L23.** If the user pressed the button to proceed, then in state **L30** the system will accept and save the values entered by the user or received from the external device. The values will be applied to carry out the one point correction of the analyte concentration value according to the several equations and algorithms disclosed herein. The monitor is thereafter returned to the measurement status and the monitoring screen is displayed showing the various measurements and any additional trend or diagnostic information thereto. By allowing the system to correct or match the analyte concentration measurement with a reference device, a user can have the option to correlate the analyte sensor measurements to a device the user is accustomed to using for making diagnostic decisions. Also, the user can check and/or correct for any measurement drifting that may have occurred due to extensive use of the analyte sensor. For example, the ability to correct for drift can be advantageous for fluorescent based systems that can experience photodegradation or other chemical or non-chemical indicator systems that experience drifts in the measurement due to structural or chemical wear over use.

In continued reference to **FIG. 16****,** the user can repeat the in vivo calibration several times over the life of the sensor. Thus, if the in vivo calibration is activated for a second or more time, the determination module will determine that it is not the first time the sensor has had the calibration performed (**L2**). Then, in state **L31,** the button that was used for indicating a sample has been drawn changes label to calibration. In another embodiment, a different button for calibration is activated. The current glucose value (**L32**), the current pH concentration (**L33**), the current sensor temperature (**L34**), the raw fluorescent signal (**L35**) are stored to a memory. The system then returns to the monitoring screen (**L36**) displaying the measurements and other diagnostic events. When the user presses the calibration button (**L38**), the system then can move to state **L8-L11 or** prompt the user to verify the sample was drawn and tested within sufficient time. In other embodiments, the system can automatically determine based on the stored time of pressing the draw button and the stored time of pressing the calibration button. Thereafter, the system can carry out the same in vivo calibration sequence as a first time calibration. Thus the user then is prompted to press a button (**L24**) and the determination module determines whether the back button or the proceed button is pressed (**L25**). If the back button is pressed, it returns to an earlier state, for example **L20.** In another embodiment, when the back button is pressed, the system returs to an earlier state associated with the calibration sequence that is not the first time of calibration, for example to state **L36** and showing the monitoring screen. The user then can enter the measurements received from the reference device into the monitor or system (**L26**), confirm the entry of the data (**L27**) and after pressing a button (**L28**), the system will accept and save the values (**L30**).

### Sensor Memory

The sensor assembly may further comprise a memory. The memory is configured to record, store, and/or transmit information, and includes but is not limited to EEPROM, SEEPROM, flash memory, RAM, DRAM, memory chip, and other memory storage elements. In a preferred embodiment, a serial EEPROM is utilized to record, store, and transmit a variety of parameters. In one embodiment, the memory is within the sensor. In one embodiment, the memory is within a connector that attaches the sensor to the controller. In another embodiment, the memory is externally connected to the sensor or the connector. In one embodiment, the memory or housing thereof, is detachable by an operator, such as by a snap-off or readily pliable mechanism such as friction from a press fitting or a locking mechanism.

The memory of the present invention can be associated to the sensor and controller system in various ways. The memory can be attached to the connector that connects the sensor and controller, to the controller, or to the sensor. The connector may comprise of various components that will allow for association with a memory. In one embodiment, the connector comprises an internal structure that allows for placing the memory internally, for example by an over-molding or an insert-molding, or by a cavity with an accessible port. In another embodiment, the connector comprises an external structure that allows for placing the memory externally, which optionally further comprises a protective cover to avoid any contamination from external objects, such as fluids. In one embodiment, the connector comprises optical and/or electrical components to connect the sensor and controller, such as an optical-electrical cable. In one embodiment, the connector comprises a sensor that is permanently engaged. In another embodiment, the connector is detachable from the sensor. In another embodiment, the memory is associated directly to the sensor, by being attached internally or externally by similar components disclosed above.

In one embodiment, the memory is detachable from the sensor assembly and is not in contact with the patient. By having the memory detachable from the potentially contaminated portion, end users can transfer the sensor usage information by shipping the memory unit without having to ship the entire biologically contaminated sensor assembly. In one embodiment, the sensor or connector comprising the memory further comprises a wireless communication device, such as a radio or infrared communication device. In another embodiment, the sensor or connector further comprises a data access port for easier access to the memory unit. By having wireless or easy data access to the sensor memory, end users can access the sensor memory by a device, such as a computer or smart phone, and transfer the memory data digitally, for example by email or via the internet.

The memory can be configured to be used both at the time of manufacture of the sensor to record a variety of QC and factory calibration information, and likewise, at the time of use of the monitor. Such information include, but are not limited to information related to model number or sensor types, date of manufacture, lot number or other identifying manufacturing information, factory determined calibration coefficients, slope and offset pertaining to thermocouple characteristics, algorithms for calibration or operation, and other chemistry-dependent information. Other applicable information include monitor or controller ID, *in vitro* calibration data including hydration time and cycle data, general signal levels and quality of signals, *in vivo* calibration data, date and initial time of use, period and duration of use, error codes that occurred during use, disconnects and/or reconnects and any new *in vivo* calibration data, alarm data, glucose measurements, pH measurements, temperature measurements, alarm parameters, voltage readings, patient information, specific operating parameters when adjusted by user.

Information recorded, stored, and transmitted by the memory has various uses. In certain embodiments, the controller or sensor can disconnect and reconnect times and re-calibration operations, detect an attempted "re-use" of a used sensor, detect elapsed time exceeding prescribed use limits, detect or prevent *in vivo* or *in vitro* calibration outside the prescribed limits, detect expired sensors, detect time of insertion into vessel or tissue either by an algorithm or manually, report back to factory any issues or problems related to a specific use, determine whether recalibration is required or necessary, etc.

### Glucose Activity and Glycemic Control

While clinicians have used insulin for decades to regulate glucose levels in diabetics and critically ill patients, determining precise dosages remains a problem. Insulin reduces circulating glucose levels through a series of complex interactions involving a number of hormones and cell types. Dosage protocols for insulin attempt to replicate the physiologic secretion of the hormone by the pancreas. However, administering according to fixed times and algorithms based on blood glucose measurements can only crudely approximate the ability of a healthy individual to continuously adjust insulin production in response to the amount of bioavailable glucose and the needs of the body. Thus, to determine the precise amount of insulin that should be administered to maintain a patient's blood glucose at an appropriate level, it is necessary to have near real-time, accurate measurements of the amount of bioavailable glucose circulating in blood.

Unfortunately, most existing methods for determining blood glucose concentrations fail to provide near real-time, accurate measurements of the amount of bioavailable glucose. Clinicians and diabetic patients typically rely on point-of-care testing that seems to measure glucose concentration in plasma, e.g., using glucometers to read test strips that filter separate plasma from cells in a drop of whole blood. While the results can be available quickly, they vary depending on the patient's hematocrit, plasma protein and lipid profiles, etc., and can often be falsely elevated (See e.g., Chakravarthy et al., 2005 "Glucose determination from different vascular compartments by point-of-care testing in critically ill patients" Chest 128(4) October, 2005 Supplement: 220S-221S). More accurate determinations can be obtained by first separating the cellular components of whole blood. However, this requires separation of the plasma from the cellular components of blood, e.g., by centrifugation. Subsequently, isolated plasma must be stored and/or transported and/or diluted prior to analysis. Storage and processing conditions, e.g., temperature, dilution, etc., will almost certainly perturb the *in vivo* equilibrium between the bound and free (bioavailable) glucose. Consequently, regardless of the technology subsequently employed for measuring plasma glucose concentration (e.g., glucose oxidase, mass spectrometry, etc.), the measured glucose concentration is likely no longer reflective of the amount of bioavailable glucose *in vivo.* Therefore, it is not feasible to use plasma glucose measurements for near real-time monitoring and adjustment of a patient's glucose level.

Accordingly, in certain embodiments, the preferred glucose sensors described herein measure glucose "activity" as opposed to glucose concentration. More precisely, glucose activity refers to the amount of free glucose per kilogram of water. In some embodiments, glucose activity can be measured directly using glucose sensors, such as the equilibrium, non-consuming optical glucose measurement systems discussed above, which employ a chemical indicator system to quantify the amount of free, bioavailable glucose, which is in equilibrium between the water compartment of blood (i.e., not associated with cells, proteins or lipids, etc.) and the glucose binding moiety/quencher. The discussion of the sensors that follow will often refer to the physical quantity to be measured as an "analyte concentration", "glucose concentration" or simply a "concentration." However, it is to be understood that "concentration" as used herein, refers to both "analyte concentration" as that phrase would be ordinarily used and also to "activity" (in some cases "glucose activity") as that phrase is described above.

### Analyte Sensor Manufacturing Process

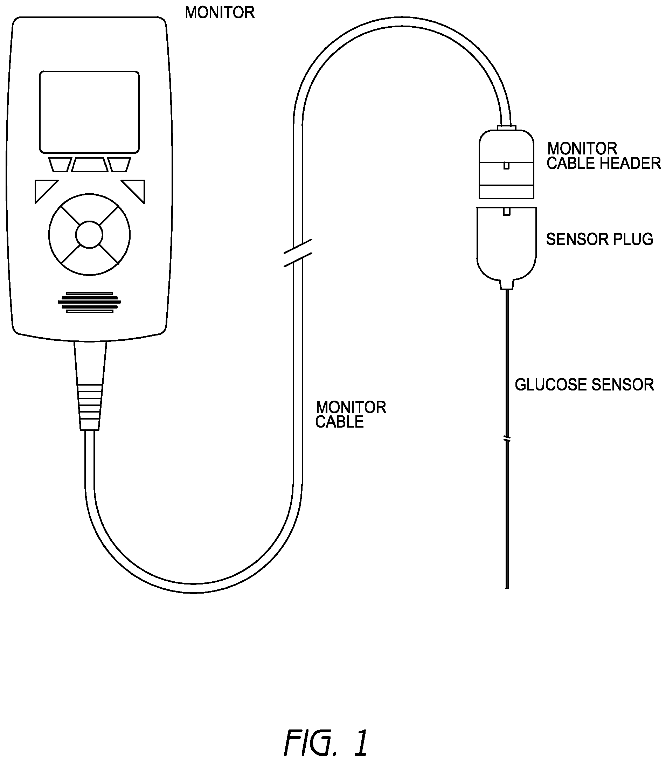

Various embodiments are described herein with regards to methods of storing and/or retrieving information in the sensor memory during manufacturing of the sensor device. **FIG. 1** illustrates an analyte monitoring system with a monitor and a separate disposable intravascular or interstitial analyte sensor. The monitor has a cable with a cable header at the end that engages the sensor plug of the disposable analyte sensor. The sensor plug can also include the memory module, such as a SEEPROM (Serial Electrically Erasable Programmable Read-Only Memory) component, which can store information. In a preferred embodiment, the SEEPROM component can contain 64 Kbit storage, PC serial interface circuit, ISO 15693 RF protocol circuit, and be dual ported such that it can interface through the RF link and also through the I²C serial interface (*e.g.* ST part number M24LR64-R). The sensor plug can further include an antenna that can communicate, and also receive and transfer power from an external programmer unit. Power can also be provided by the monitor when the I²C serial interface is in use. For example, the memory module can store certain factory information for the monitor to read during sensor use by using the electrical wires and terminals implemented in the sensor plug housing. Also, the monitor can write sensor usage information to the memory module, which can be later accessed by the user or manufacturer for informational or investigational purposes.

In certain embodiments, the analyte sensors are assembled in batches of multiple sensors. The assembly process can include formulating the chemical compounds that is used for a chemical indicator system that is imbedded in the tip portion of the analyte sensor. The assembly process can also include placing optical fibers inside of a cable and connecting the one end to the sensor plug, which can include the optical ferrule for the optical fibers to connect to. The assembly process can also include forming the chemical indicator system on the tip portion of the analyte sensor and placing a temperature sensor also on the tip portion. Electrical wires and connectors can also be placed onto the sensor plug, including placing the memory module inside the sensor plug, and connecting the temperature sensor to the electrical wires. After the analyte sensor is assembled to an operational state, the manufacturing process can include quality check procedures, for example verifying the electrical components including the temperature sensor operate properly, and verifying the chemical indicator system and optical components operate as desired by conducing a test process that simulates a sensor use or a calibration. The test process can be conducted on a sample of analyte sensors that will be discarded after testing, or can be conducted on some or all of the analyte sensors that will be later packaged for use.

Once the analyte sensors are assembled, each individual analyte sensor is packaged into individual sensor packages. The sensor package can be made of any suitable material, such as Tyvek® material, aluminum foil, ethylene-vinyl alcohol copolymer, polyvinylidenechloride, polyethylene terephthalate, nylon, polypropylene, polyester, polyethylene, aluminum oxide coatings, silica oxide coatings, polyvinyl alcohol, and any combinations thereof. The sensor package can include a plastic tray for the analyte sensor, along with other optional support plumbing and components, to be securely placed in. The sensor package can be a pouch or a tray assembly.

After placing the assembled analyte sensor inside the package, the package is sealed. The analyte sensor can be cleaned prior to packaging, such as by wiping with alcohol or other disinfectants or detergents. After packaging, the packaged analyte sensors can be sterilized, for example by placing the packaged analyte sensor in a field of radiation that can penetrate the package, such as gamma radiation, x-rays, or high-energy electrons. The sealed package can keep the analyte sensor sterile until it is opened by the user. Other sterilization techniques include beta radiation, ethylene oxide, steam, and/or autoclaving.

In certain embodiments, after the analyte sensor is packaged and sterilized, a sample of analyte sensors is acquired from the batch of analyte sensors and tested. The testing can include calibrating the sensor to determine the calibration coefficients for the particular sensor. The testing can also include a clinical simulation process where the analyte sensors are put through extensive tests that simulate clinical use. The testing can also verify the temperature sensor and electrical components are properly operating. The testing can also verify the chemical indicator system has the proper amount of fluorescent emission for the tested analyte concentration, as well as a proper analyte response characteristic and sensitivity to analyte concentration change, pH change, temperature change, etc. Based on the test results, a determination is made whether the batch of sterilized analyte sensors is acceptable for use based on a number of quality criteria. The tested samples may be discarded because they are no longer sterile.

Also based on the test results, the calibration coefficients are calculated for the sterilization batch, the analyte sensor assembly batch, and/or the chemical indicator system subassembly batch. In a preferred embodiment, the calibration coefficients are determined for each sterilization batch to account for any variation that may be introduced by the difference in sterilization conditions, for example the amount of gamma irradiation that is applied to the sterilization batch, for example the amount of gamma irradiation that is applied to the sterilization batch. In one embodiment, the calibration coefficients are determined for each analyte sensor assembly batch, because the sensors are not later sterilized, or because the sterilization does not introduce significant changes to the analyte sensor response characteristic or quality. In one embodiment, the calibration coefficients are determined for chemical indicator system subassembly batch to account for the different analyte sensor response characteristic or quality from each batch of the chemical indicator system subassembly.

In the embodiment where a sample set of sterilized analyte sensors are tested to determine the calibration coefficients for the associated sterilization batch, once the sample quality and functionality are verified, the calibration coefficients can be calculated based on the test measurements. The calibration coefficients and other information can be programmed into the memory module for each individual sterilized analyte sensor. In certain embodiments, a physical contact with the analyte sensor may not be possible for programming the analyte sensor in order to maintain the sterility of the analyte sensor. In certain embodiments using a passive RFID sensors, the analyte sensor does not have a powering device, such as a battery, to power the memory module to an operational state.

Radio Frequency Identification (RFID) technology can be used to power the memory module on the sterilized analyte sensor and provide for communication capability to program the memory module. The RFID technology that can be used includes passive RFID (using no battery), active RFID (with an on-board battery that always broadcasts or beacons its signal) or battery assisted passive RFID (BAP) which has a small battery on board that can be activated when the storage device communicates with the RFID reader. In a preferred embodiment, the RFID technology can be defined in the ISO 16963 standard as passive RFID technology operating at 13.65 MHz. Use of RFID technology allows for the reading and writing without the need for physical contact with the sensor. Also, because a battery may be omitted from the analyte sensor, it can be made more compact and also avoid any issues relating to the sterilization process damaging the battery, and also the concern of battery life being potentially shorter than the shelf life of the sterilized analyte sensor.

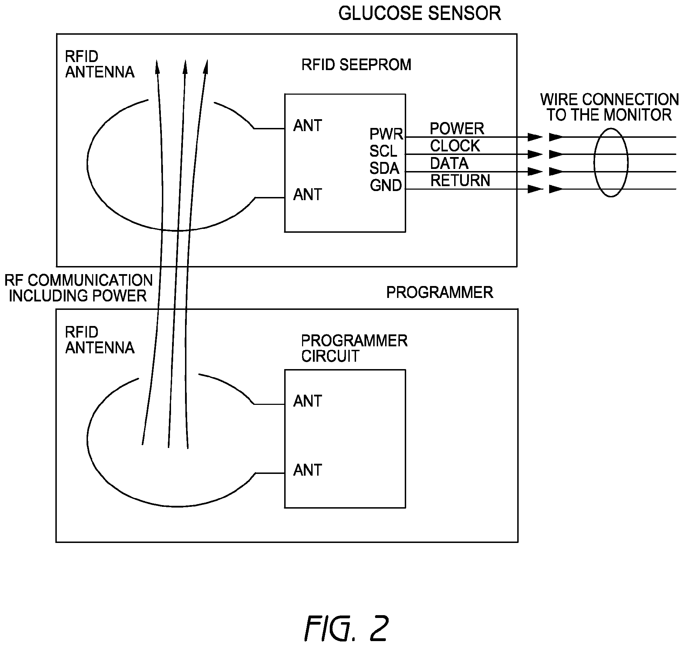

**FIG. 2** illustrates an analyte sensor being programmed by an external programmer through an RFID system. The SEEPROM is on the analyte sensor and inside the package. The SEEPROM is connected to an RFID antenna. The SEEPROM is also connected to the electrical connections on the sensor plug portion, which connects to the monitor cable. When the analyte sensor is used in clinical settings, the SEEPROM portion becomes electrically connected to the monitor through the connections and cables, allowing the monitor to access the individual analyte sensor information such as the calibration coefficients. An external programmer also has an RFID antenna that is connected to a programmer circuit. The external programmer can powered by an external source or by a battery.

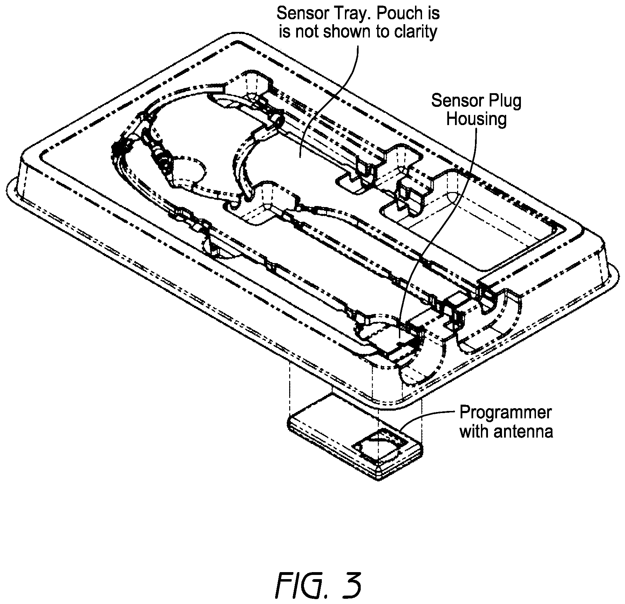

**FIG. 3** illustrates the analyte sensor placement, while in a tray, relative to the external programmer. In this embodiment, the sterilized analyte sensor is shown placed on a tray and inside a pouch (pouch not shown). In this embodiment, the analyte sensor's antenna is placed inside the sensor plug housing. The external programmer has an antenna, which is placed near the antenna of the analyte sensor. The required distance between a external programmer antenna and analyte sensor antenna depend on the system being used as well as the antenna size and the RF transmission power. For a passive RFID technology, a short distance between the external programmer antenna and the analyte sensor antenna is typically required. In one embodiment, the analyte sensor plug housing has small physical size, thus the RFID antenna size in the analyte sensor is limited and thus the supported distance between the external programmer antenna and the analyte sensor antenna is limited between 2∼10 cm. In a preferred embodiment, the supported distance is about 5 cm.

When the external programmer antenna is in close enough proximity to the analyte sensor antenna, the programming sequence is initiated so that the external programmer programs the SEEPROM while providing power to the SEEPROM. This programming sequence can be performed in the factory as part of the manufacturing process of the analyte sensor. In this embodiment, the information, such as the calibration coefficients, to be stored in each analyte sensor unit are passed to the external programmer unit. The sterilized analyte sensor inside the packing is placed in close proximity with the external programmer unit. Custom tooling can be used that allows the analyte sensor assembly to automatically be in close enough proximity to the external programmer unit when the packaging is placed in a certain holder or tray assembly. In certain embodiments, a conveyer system can be used wherein the analyte sensors are placed on holders or trays along a conveyer belt, wherein the conveyer moves at a predetermined rate and/or predetermined sequences, such that each analyte sensor will pass over, under, or across from the external programmer by a predetermined time for the programming sequence to be completed.

The user or the system automatically initiates the programming operation. In certain embodiments, when the analyte sensor antenna is in close proximity to the external programmer antenna, the external programmer detects the analyte sensor antenna and automatically initiates the programming operation. After initiating the programming operation, the external programmer communicates with the analyte sensor through the respective antennas, providing power to the SEEPROM of the analyte sensor via the antenna, and then programs the information into the SEEPROM.

### Optical Glucose Sensor Configurations

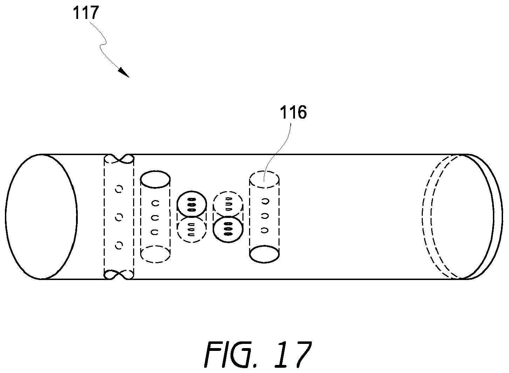

**0236]** Various glucose sensor configurations are possible as disclosed in U.S. 2009 Patent Publication No. 2011/0105866. With reference to **FIG. 17****,** certain prior art embodiments (see US Patent Publication No. 2008/0188725) are illustrated. The glucose sensor **117** in **FIG. 17** is an optical fiber with a series holes **116** drilled straight through the sides of the optical fiber. In certain embodiments, the holes **116** are filled with one or more glucose-sensing chemical indicator systems. These holes may be covered with a selectively permeable membrane, wherein the permeability is selected such that the molecules of the chemical indicator system (e.g., fluorophore and quencher) are retained within the cavities, whereas glucose is freely permeable. In certain embodiments, the series of holes **116** that are drilled through the glucose sensor **117** are evenly spaced horizontally and evenly rotated around the sides of the glucose sensor **117** to form a spiral or helical configuration. In certain embodiments, the series of holes are drilled through the diameter of the glucose sensor.

In certain embodiments, the glucose sensor **117** is a solid optical fiber with a series of holes drilled through the sides of the fiber at an angle. In certain embodiments, the optical fiber comprises a groove along the length of the optical fiber, wherein the groove is filled with hydrogel/chemical indicator system. In certain embodiments, the glucose sensor **117** is a solid optical fiber with triangular wedges cut from the fiber.

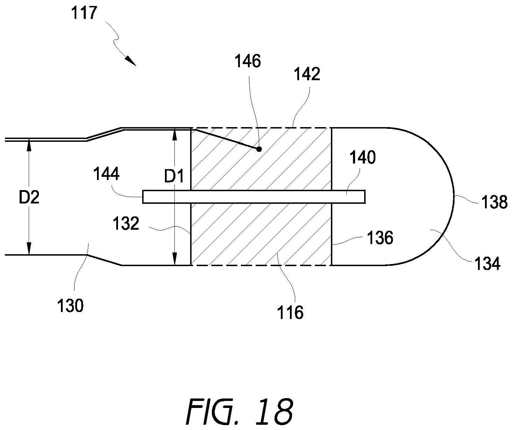

In certain embodiments, as illustrated in **FIGS. 18-19****,** the glucose sensor **117** comprises an optical fiber **130** having a distal end **132,** an atraumatic tip portion **134** having a proximal end **136** and a distal end **138,** a void or cavity **116** between the distal end **132** of the optical fiber **130** and the proximal end **136** of the atraumatic tip portion **134,** and a rod **140** connecting the distal end **132** of the optical fiber **130** to the proximal end **136** of the atraumatic tip portion **134,** wherein the rod traverses the void or cavity. In preferred embodiments, molecules of a chemical indicator system are disposed within the void or cavity **116** and immobilized (by covalent bonding or non-covalent interaction) or otherwise associated within hydrogel matrices. See e.g., the chemical indicator systems disclosed in US Patent Nos. 7,417,164 and 7,470,420. The cavity **116** may be loaded with hydrogel/chemical indicator system by any methods known in the art. In preferred embodiments, the cavity **116** is filled with hydrogel/chemical indicator system in a liquid state. The hydrogel/chemical indicator systems are preferably polymerized *in situ,* as detailed in co-pending US Patent Appl. 12/026,396 (published as 2008/0187655).

In some embodiments, the proximal surface of the rod **144** is reflective so that a portion of the excitation light signal (or reference light signal) is reflected proximally down the optical fiber **130** to a detector (not shown). The term rod is used herein to refer to any elongate structural member, regardless of its geometry, configured to connect the atraumatic tip portion to the optical fiber. The rod may be centered coaxially (as illustrated) or off-centered with regard to the cross-section of the fiber and atraumatic tip portion. In some embodiments, there may be more than one rod extending between the fiber and the atraumatic tip portion. Where more than one rod is employed, the rods may be arranged symmetrically or asymmetrically with respect to the cross-section of the fiber and atraumatic tip portion.

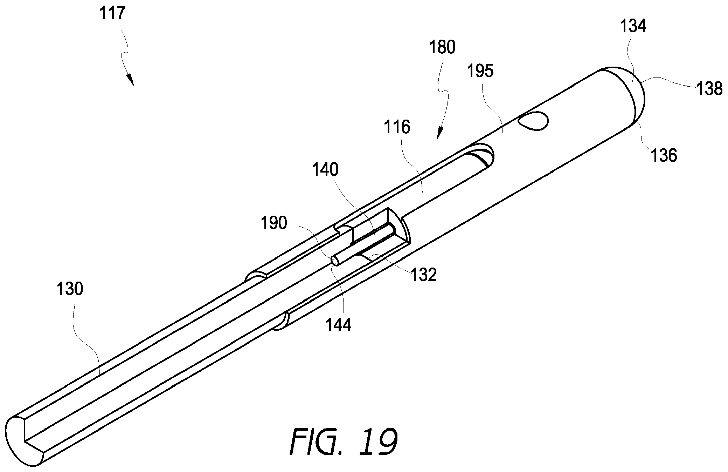

In certain embodiments, as illustrated in FIG. **19****,** a reference material **190** may be attached to the proximal surface of the rod **144.** The reference material **190** may be reflective (e.g., a mirror) and functions similar to embodiments in which the proximal surface of the rod **144** reflects at least a portion of the excitation light signal (or reference light signal) down the optical fiber **130** to a detector (not shown). In other embodiments, the reference material **190** comprises a separate dye indicator system, such as for example a glucose-insensitve fluorescent dye. The excitation light from the optical fiber **130** causes the glucose-insensitive fluorophore to emit a fluorescent light back to a detector (not shown) in order to reference the emission signal from the hydrogel/chemical indicator system. In certain embodiments, the separate dye indicator system is formed of a plastic material, such as for example polycarbonate, polyethylene, or polystyrene, infused with a fluorescent dye configured to emit a separate glucose-insensitive signal.

As illustrated in FIG. **18****,** the hydrogel and glucose-sensing chemical indicator system is disposed within the cavity **116.** In preferred embodiments, the hydrogel/chemical indicator system filled cavity **116** is covered by a selectively permeable membrane **142** that allows passage of glucose into and out of the hydrogel/chemical indicator system. Although these embodiments are described using a glucose sensor **117,** it should be understood by a person of ordinary skill in the art that the sensor **117** can be modified to measure other analytes by changing, for example, the sensing chemistry, and if necessary, the selectively permeable membrane **142.**

In some embodiments, as illustrated in **FIG. 18****,** the sensor **117** comprises a distal portion and a proximal portion. The distal portion of the sensor **117** comprises the atraumatic tip portion **134,** the hydrogel/chemical indicator system filled cavity **116,** the rod **140,** at least the portion of the selectively permeable membrane **142** that covers the cavity **116** and the distal end **132** of the optical fiber **130.** The proximal portion of the sensor **117** comprises the proximal portion of the optical fiber **130.** In some embodiments, the diameter, **D1,** of the distal portion of the sensor **117** is greater than the diameter, **D2,** of the proximal portion of the sensor **117.** For example, the diameter **D1** of the distal portion of the sensor **117** can be between about 0.0080 inches and 0.020 inches, while the diameter **D2** of the proximal portion of the sensor **117** can be between about 0.005 inches to 0.015 inches. In some embodiments, the diameter **D1** of the distal portion of the sensor **117** is about 0.012 inches, while the diameter **D2** of the proximal portion of the sensor **117** is about 0.010 inches.

The rod **140** in **FIG. 18** adds mechanical stability to the distal portion of the sensor **117.** In some embodiments, the rod **140** also adds flexibility to the distal portion of the sensor **117,** allowing the atraumatic tip portion **134** to flex back and forth relative to the orientation of the optical fiber **130.** The flexibility of the rod **140,** and thus the degree which the atraumatic tip portion **134** can flex, can be increased or decreased by decreasing or increasing the diameter of the rod **140.** In addition, the flexibility of the rod **140** can be altered by making the rod **140** from a stiff or flexible material.

In some embodiments as illustrated in **FIG. 18****,** a reflective surface **144** is disposed on the proximal end of the rod **140,** which is inserted into the optical fiber **130.** The reflective surface **144** is capable of reflecting back at least a portion of either reference light or excitation light emitted from the light source. The other end of the rod **140** is inserted into the atraumatic tip portion **134.** In certain embodiments, the atraumatic tip portion may be made from a non-reflective material, for example polyethylene (e.g., black polyethylene) or polypropylene. The reference or excitation light that passes though the optical fiber **130** in the region corresponding to the diameter of the rod **140** is reflected off the reflective surface **144** without entering into the hydrogel filled cavity **116;** the amount of light entering the hydrogel/chemical indicator system can be controlled by varying the diameter/cross-sectional area of the rod and/or by attaching a mirror or other reflective member **190** (illustrated in **FIG. 19**) having a selected cross-sectional area to the proximal end of the rod. The hydrogel filled cavity **116** is preferably covered by a selectively permeable membrane **142,** which is at least permeable to glucose. Therefore, the reflected reference or excitation light and the ratio between the reflected and emitted light is independent of the temperature, pH, glucose concentration, and chemistry formulation of the hydrogel filled cavity **116.** The ratio between the reflected and emitted light is dependent, however, on the diameter of the rod and the ratio of the diameter of the rod to the area of the sensor. In certain embodiments, the rod **140** is sufficiently stiff to keep the hydrogel filled cavity **116** in a fixed orientation relative to the optical fiber **130** so that any light that is transmitted through the hydrogel cavity **116** is not reflected back to the optical fiber **130.**

In some embodiments, as illustrated in **FIG. 19****,** the glucose sensor **117** includes a cage **195,** as an outer shell, connecting the atraumatic tip **134** with the optical fiber **130.** The cage **195** can add mechanical stability to the distal portion of the sensor **117.** In some embodiments, the cage **195** also adds flexibility to the distal portion of the sensor **117,** allowing the atraumatic tip portion **134** to flex back and forth relative to the orientation of the optical fiber **130.** The flexibility of the cage **195,** and thus the degree which the atraumatic tip portion **134** can flex, can be increased or decreased by decreasing or increasing the thickness of the cage **195** walls. In addition, the flexibility of the cage **195** can be altered by making the cage **195** from a stiff or flexible material. In certain embodiments, the thickness of the cage **195** walls is about 0.001 inches, about 0.002 inches, or about 0.004 inches. In some embodiments, the thickness of the cage **195** walls may be less than about 0.001 inches. In some embodiments, the thickness of the cage **195** walls may be greater than about 0.010 inches.

In some embodiments, the diameter of the optical fiber **130** may be smaller than the diameter of the interior of the cage **195,** allowing the optical fiber **130** to fit within the interior of the cage **195** and abut the void or cavity **116.** For example, the diameter of the optical fiber **130** may be between about 0.005 inches and about 0.020 inches, between about 0.008 inches and about 0.014 inches, or between about 0.010 inches and about 0.012 inches. The diameter of the interior of the cage **195** may be about 0.001 inches larger.

In certain embodiments, the cage **195** has a window or opening **180,** covered by a selectively permeable membrane **142** (not shown), which allows for at least the transmission of analytes, such a glucose, into the void or cavity **116.** In certain preferred embodiments, the void or cavity **116** is filled with a hydrogel/chemical indicator system. A rod **140** may be positioned within the glucose sensor **117** having a reference material **190.** As discussed above, the reference material **190** may be a mirror for reflecting excitation light from the optical fiber **130** back to a detector (not shown) or a glucose-insensitive fluorescent dye for emitting a glucose-insensitive reference signal back to a detector (not shown). The combination of the cage **195** and the rod **140** may provide a sufficiently rigid structure such that the excitation light which enters the void or cavity **116** remains separate from the light that enters the reference material **190.**

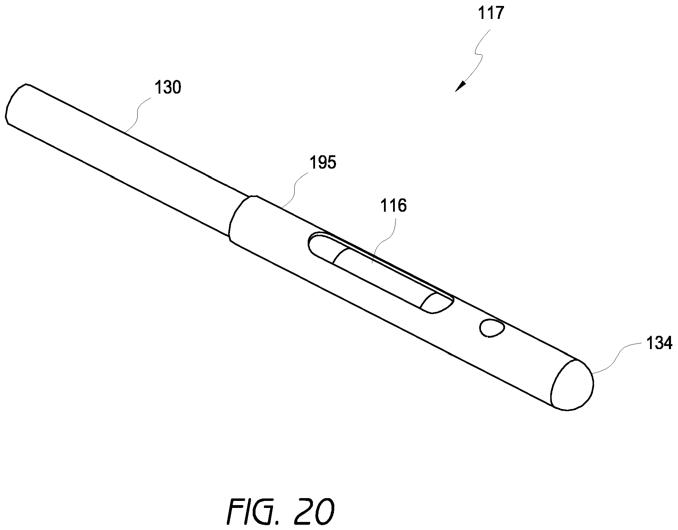

**FIGS. 20-21** illustrate certain embodiments having different configurations for the reference material. As discussed previously, the reference material **190** in each of these embodiments may either comprise reflective material to return at least a portion of the excitation light back to a detector (not shown) or a separate dye indicator system to return an emission signal back to a detector (not shown). Similar to previous embodiments, the excitation or reference light is reflected off the reflective surface without entering the cavity **116,** which is filled with the hydrogel/chemical indicator system. Likewise, the emitted or reference light from the separate dye indicator system is independent of the glucose concentration. Therefore, the reference light and the ratio between the reference light and emitted glucose concentration dependent lights are independent of the temperature, pH, glucose concentration, and hydrogel chemistry. The surface area, shape, and configuration of the reference material can be varied to control the amount of excitation light that enters the hydrogel/chemical indicator system filed cavity **116.** The distal end of the rod or hypotube **140,** as in previous embodiments, or reference material may have a non-reflective surface, such as a black plug made of polyethylene so that light entering the hydrogel/chemical indicator system filled cavity **116** is not reflected back into the optical fiber **130.**

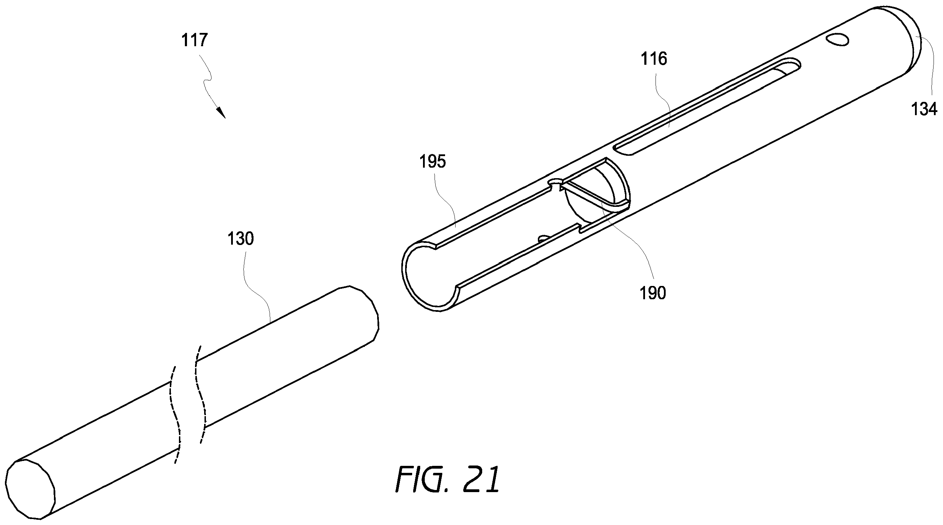

In **FIG. 20****,** the reference material abuts the void or cavity **116** beneath the cage **195** and extends to and comprises the atraumatic tip **134.** In certain embodiments, the atraumatic tip **134** is formed of a glucose-insensitive red dye plastic material. In **FIG. 21****,** the reference material is a reflective strip that spans the diameter of the hydrogel-filled cavity **116.** The term reflective strip is used herein to refer to any elongate member, regardless of its geometry, width, or thickness that spans at least a cross-section of the glucose sensor **117.** The reflective strip may be centered at the diameter of the glucose sensor **117** (as illustrated) or off-centered with regard to the cross-section of the cage **195** or optical fiber **130.** In some embodiments, there may be more than one reflective strip in one or more locations within the glucose sensor **117.** Where more than one reflective strip is employed, the reflective strip may be arranged symmetrically or asymmetrically with respect to the cross-section of the glucose sensor **117.** In certain embodiments, the reflective strip **190** may be between about 0.001 inches and about 0.005 inches wide and between about 0.001 inches and about 0.005 inches thick.

In one embodiment, the reference material is disposed within the cage **195** as a hypotube containing the hydrogel-filled cavity **116** and having a reflective surface or annular mirror at the proximal surface of the hypotube. In certain embodiments, the hypotube has an outer diameter equal to the outer diameter of the optical fiber **130.** The reference material may be a reflective strip and placed within a hole drilled in the optical fiber **130.** In one embodiment, the reference material is located in the cavity **116** and is in a side-by-side configuration with the hydrogel/chemical indicator system.

In certain embodiments, a reference material comprises a translucent material. In certain embodiments, this translucent material comprises a red dye, such as the glucose-insensitive fluorescent dye discussed previously. The red dye may allow some of the excitation light to be transmitted to the hydrogel-filled cavity **116,** may reflect some of the excitation light back to a detector (not shown) before the excitation light reaches the hydrogel-filled cavity **116,** and may emit a separate glucose-insensitive signal to a detector (not shown).

A person skilled in the art would readily understand that the above described embodiments, or components of the above described embodiments, may be combined. For example, a glucose sensor may contain one or more structural elements, such as a cage, a hypotube, and/or a rod. In addition, a glucose sensor may contain one or more reference materials, functioning as a reflective surface and/or as a separate dye indicator system, in different locations and configurations.

As shown in **FIG. 19****,** in some embodiments, the glucose sensor **117** comprises an atraumatic tip portion **134.** The atraumatic tip portion **134** has a distal end **138** that is curved and substantially free of sharp edges. In addition, the atraumatic tip portion **134** can be flexible and deformable. The distal end **138** of the atraumatic tip portion **134** can be hemispherical, parabolic, elliptical or curved in any other suitable shape that is reduces the risk of injury to the patient. The atraumatic tip portion **134** can be made from a variety of materials, such as plastics, polymers, gels, metals and composites of the above.

In some embodiments, the glucose sensor **117** includes a temperature sensor or probe **146,** such as thermocouple or thermistor (See e.g., **FIG. 18**). In another embodiment, the thermocouple has a diameter that is substantially the same diameter D2 as the optical fiber 130 and is disposed adjacent to the optical fiber or sensor area. The temperature sensor **146** can measure the temperature of the hydrogel and glucose sensing chemistry system, and/or the blood when disposed intravascularly. The temperature sensor **146** is particularly preferred when the glucose-sensing chemistry is affected by temperature. For example, in some embodiments, the fluorescence intensity emitted by the fluorophore system is dependent on the temperature of the fluorophore system. By measuring the temperature of the fluorophore system, temperature induced variations in fluorophore fluorescence intensity can be accounted for, allowing for more accurate determination of glucose concentration.

In certain embodiments, the temperature sensor can be a thermistor (as described above with regard to **FIG. 18****,** reference numeral **146,** a platinum resistance temperature device ("RTD"), another RTD, a thermocouple, an infrared-based temperature detector, a fluorescence-based temperature sensing element, or other temperature sensing elements with determinable temperature-dependent characteristics.

Devices such as thermistors, platinum RTDs, and other RTDs generally require one or more conductors, such as wires, to conduct the output of the sensor to a receiving unit which converts the output to a temperature signal. The conductors can be bundled with the optical fiber of fluorescence-based glucose sensors, such as those discussed above, or they can be routed separately. In one embodiment, the temperature sensor is placed inside the body, and the receiver is placed outside the body. In another embodiment, the temperature sensor is placed inside the body, and a transmitter, signal processor, etc. is also placed inside the body and is connected to or is a part of the temperature sensor. In preferred embodiments, the temperature sensing element is located at or near the glucose sensing moiety.

In another embodiment, a fluorescence-based temperature sensing technique can be used. Fluorescence-based temperature sensing techniques include those based on fluorescence decay, such as where an excitation light is provided to a phosphor, the excitation light is stopped, and the fluorescence is monitored versus time, with the rate of decrease in fluorescence being related to the temperature of the phosphor. Various techniques, can also include phase measurement and phase angle analysis.

Methods for performing fluorescence-based temperature measurement have been described. See for example, LumaSense Technologies, Inc. (Santa Clara, CA), "Fluoroptic Temperature Monitoring," http://www.lumasenseinc.com/technology/fluoroptic_ thermometry.html. Fluorescent materials that can be used in fluorescence-based temperature measurement are known to, or readily identified by those having skill in the art.

In some embodiments, the fluorescent material can be surrounded by material which prevents or inhibits chemical interaction between the fluorescent material and blood components. Suitable materials include glass (for example, borosilicate, lime-soda, or other types including those used for fiberoptic cables), polymers (for example, Teflon, fluoropolymers, silicone, latex, polyolefins, polyisoprene, and other rigid and nonrigid polymeric materials), metals (for example, 300 series stainless steel, 400 series stainless steel, nickel, nickel alloys, chromium steels, zirconium and its alloys, titanium and its alloys, as well as other corrosion resistant metals and alloys including exotic metals and alloys), ceramics (for example, ceramic materials related to aluminum oxide, silica and oxide, zirconium, carbides, etc.), and combinations of these.

In some embodiments, the temperature sensor can be positioned within the glucose sensor, or near it. While in one preferred embodiment, the temperature sensor can be positioned as close as possible to (e.g., within) the glucose-sensing chemical indicator system of the glucose sensor, positions some distance away can also be successfully utilized, including those locations where the temperature measured provides an indication of the temperature at the glucose-sensing site(s) within an acceptable error for the use for which the temperature measurement is being made.

In some embodiments, the temperature sensor and/or the leads to the sensor can be isolated from the physiological environment, such as by coating, covering, or encasing the various parts with a material that prevents or inhibits chemical or physical interaction between the temperature sensor and/or its leads and blood components. Chemical interactions that are preferably avoided include corrosion, leaching of chemical species, generation of additional signals (e.g. optical, electrical, etc.) and take-up by the body of materials present in the sensor or leads, whether present from manufacture, corrosion or other means, such as compounds, metals, or ions causing a physiological response in some patients including copper, silver, organic compounds, organometallic compounds, etc.

Physical interactions can include breakage and physical separation (e.g. disconnection and potential loss), signal leakage (e.g. optical; electrical, etc.), signal degradation (including resistance, stray signal detection, noise, capacitance, electrochemical effects, induced voltages, ground loops, etc.). Suitable materials include glass (e.g.,_borosilicate, lime-soda, as well as other types of glass, such as those used in production of optic fibers), polymers (e.g., Teflon, fluoropolymers, silicone, latex, polyolefins, polyisoprene, acrylics, polycarbonates, and other rigid and nonrigid polymeric materials), metals (e.g., 300 series stainless steel, 400 series stainless steel, nickel, nickel alloys, chromium steels, zirconium and its alloys, titanium and its alloys, as well as other corrosion resistant metals and alloys including exotic metals and alloys), ceramics (e.g., ceramic materials related to aluminum oxide, silica and oxide, zirconium, carbides, etc.), and combinations of these.

Suitable methods for applying for isolating material to the temperature sensor or leads can include any appropriate method, including casting, painting, dipping, gluing, reacting, drawing, depositing, mechanically adhering, encapsulating, etc.

In some embodiments, suitable sizes for temperature sensors that will be incorporated into the glucose sensor include those temperature sensing elements resulting in an overall glucose sensor of between about 0.005 inches and 0.020 inches.

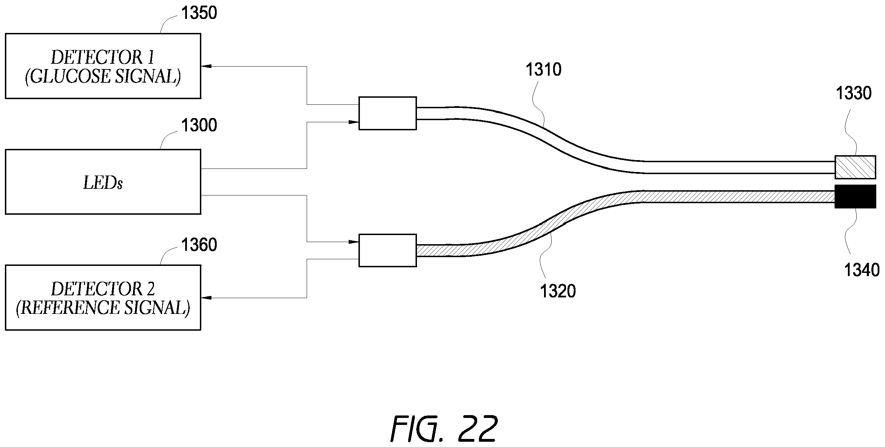

**FIG. 22** illustrates another embodiment for measuring the glucose concentration in comparison to a reference signal. In this embodiment, a LED light source 1300 sends an excitation signal down two separate adjacent optical fibers **1310, 1320.** The first optical fiber, or the glucose fiber **1310,** has a proximal tip and a distal tip. The distal tip has a glucose sensing hydrogel **1330** which contains a fluorophore or dye, a quencher, and glucose binding receptors. The second optical fiber, or the reference fiber **1320,** also has a proximal tip and a distal tip. The distal tip of the reference fiber has a reference material **1340.** In certain embodiments, the reference material **1340** contains the same or a different fluorophore or dye, may or may not contain the quencher, but does not contain glucose receptors. In other embodiments, the reference material **1340** has the same exact glucose sensing hydrogel, but it is encased in a glucose impermeable membrane. In both of these embodiments, the reference fiber **1320** emits a fluorescent return signal independent of the glucose concentration. In yet other embodiments, the reference material **1340** comprises a reflective element, such as a mirror or metallic plate, and the reference fiber **1320** transmits the excitation signal that is reflected by the reference material **1340.**

After the excitation light passes through the glucose fiber **1310** and the reference fiber **1320,** the glucose sensing hydrogel **1330** and the reference material **1340** emit fluorescent signals, or reflect the excitation signals, back to two separate detectors, a glucose signal detector **1350** and a reference signal detector **1360,** for ratiometric processing. The benefit of the dual fiber configuration is that both fibers **1310, 1320** experience the same external pressure, bending, temperature, and other external factors. In addition, both fibers **1310, 1320** contain substantially the same material in the glucose sensing hydrogel **1330** and reference material **1340.** As a result, the ratio of the intensities between the two fibers **1310, 1320,** as measured by the detectors **1350, 1360,** produce a calibrated glucose signal that removes, *inter alia,* the effect of the fluctuations in the LED output or altered transmission along the optical fiber, and thereby increase the accuracy in the measurement of the glucose concentration.

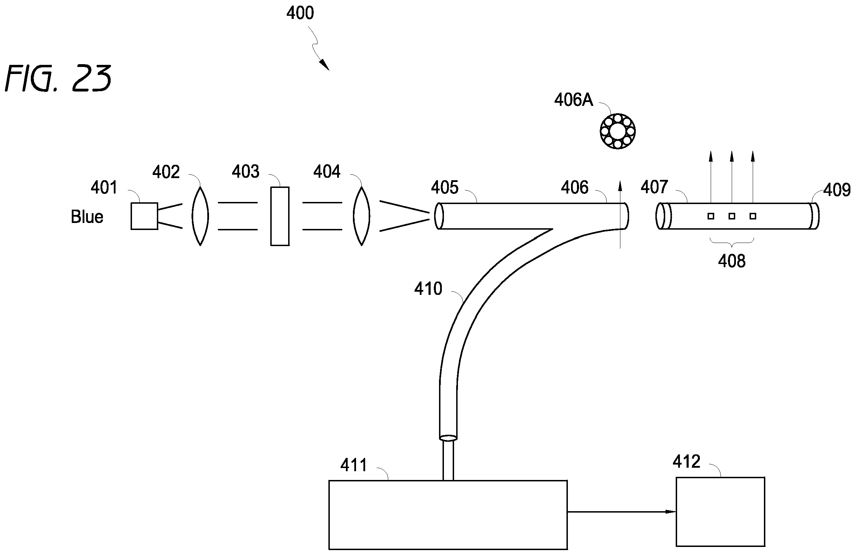

With reference to **FIG. 23****,** in certain embodiments, the light generated by the single light source **401** is transmitted through a optical module comprising a collimator lens **402,** an interference filter **403,** and/or a focusing lens **404** as described above. The resulting light can be filtered through an interference filter **403.** The resulting light can be focused by a focusing lens **404** into an optical fiber **405,** which may be a single fiber or a bundle of fibers. The optical fiber **405** can surround optical fiber **410** as both fiber optic lines connect to the first end of the glucose sensor **407.** In certain embodiments, a mirror or reflective surface **409** is attached to the second end of the glucose sensor **407.** The optical fiber **410** may be a single fiber or a bundle of fibers. The glucose sensor can comprise hydrogels that further comprise a fluorophore system that produces two emission wavelengths, a first emission wavelength and a second emission wavelength. In certain embodiments, the fluorophore system is excited by the light generated by light source **401.** In certain embodiments, the optical fiber **410** is connected to a light sensitive module comprising a micro spectrometer **411** that measures the entire spectrum of light in the glucose measurement system **400.** Data from the micro spectrometer **411** can be transmitted to computer **412** for processing. The micro spectrometer **411** can allow system **400** to simultaneously measure the excitation light intensity as well as both emission light intensities. Ratiometric calculations may be employed to substantially eliminate or reduce non-glucose related factors affecting the intensity of the measured emission light and measured excitation light (as detailed in US Patent Publication No. 2008/0188725). The measured emission light can be divided by the measured excitation light, wherein such calculations substantially eliminate or reduce non-glucose related factors affecting the intensity of the lights.

In certain preferred embodiments, the fluorophore dye may be selected such that it exists in distinguishable acid and base conformations, each of which emit at a distinct wavelength, and wherein the relative proportion of acid and base forms depend on the pH. The ratio of intensities of the acid and base emissions can be used to determine the pH of the blood (as detailed in US Patent Publication No. 2008/0188722). The ratio of the acid or base emission intensity over the excitation light can be used to determine the level of glucose in the blood. Of course in a variation to this single exciter-dual emitter fluorophore system, one could employ a single exciter-single emitter for detection of glucose concentration without simultaneous ratiometric determination of pH. Indeed, a great variety of design options are available (see e.g., US Patent Publication Nos. 2008/0188725 and 2008/0188722), wherein the chemical indicator and optical systems may be selected based on the preferred use.

### Glucose-Sensing Chemical Indicator Systems

In certain embodiments, the hydrogels are associated with a plurality of fluorophore systems. In certain embodiments, the fluorophore systems comprise a quencher with a glucose receptor site. In certain embodiments, when there is no glucose present to bind with the glucose receptor, the quencher prevents the fluorophore system from emitting light when the dye is excited by an excitation light. In certain embodiments, when there is glucose present to bind with the glucose receptor, the quencher allows the fluorophore system to emit light when the dye is excited by an excitation light.

In certain embodiments, the emission produced by the fluorophore system may vary with the pH (as well as the temperature) of the solution (for example, blood), such that different excitation wavelengths (one exciting the acid form of the fluorophore and the other the base form of the fluorophore) produce different emissions signals. In preferred embodiments, the ratio of the emission signal from the acid form of the fluorophore over the emission signal from the base form of the fluorophore is related to the pH level of the blood. In certain embodiments, an interference filter is employed to ensure that the two excitation lights are exciting only one form (the acid form or the base form) of the fluorophore. Chemical indicator systems, hardware configurations and methods for determining both pH and glucose based on ratiometric determination are described in detail in co-pending U.S. Application Nos. 11/671,880 (published as 2008/0188722) and 12/027,158 (published as 2008/0188725).

The indicator system (also referred to herein as a fluorophore system) can comprise a fluorophore operably coupled to a quencher. In certain embodiments, the fluorophore system comprises a polymer matrix comprising a fluorophore susceptible to quenching by a viologen, a viologen quencher with quenching efficacy dependent on glucose concentration, and a glucose permeable polymer, wherein said matrix is in contact with blood in vivo. Preferably the fluorophore is a fluorescent organic dye, the quencher is a boronic acid functionalized viologen, and the matrix is a hydrogel.

"Fluorophore" refers to a substance that when illuminated by light at a particular wavelength emits light at a longer wavelength; i.e. it fluoresces. Fluorophores include but are not limited to organic dyes, organometallic compounds, metal chelates, fluorescent conjugated polymers, quantum dots or nanoparticles and combinations of the above. Fluorophores may be discrete moieties or substituents attached to a polymer.

Fluorophores that may be used in preferred embodiments are capable of being excited by light of wavelength at or greater than about 400 nm, with a Stokes shift large enough that the excitation and emission wavelengths are separable by at least 10 nm. In some embodiments, the separation between the excitation and emission wavelengths may be equal to or greater than about 30 nm. These fluorophores are preferably susceptible to quenching by electron acceptor molecules, such as viologens, and are resistant to photo-bleaching. They are also preferably stable against photooxidation, hydrolysis and biodegradation.

In some embodiments, the fluorophore may be a discrete compound.

In some embodiments, the fluorophore may be a pendant group or a chain unit in a water-soluble or water-dispersible polymer having molecular weight of about 10,000 daltons or greater, forming a dye-polymer unit. In one embodiment, such dye-polymer unit may also be non-covalently associated with a water-insoluble polymer matrix M¹ and is physically immobilized within the polymer matrix M¹, wherein M¹ is permeable to or in contact with an analyte solution. In another embodiment, the dye on the dye-polymer unit may be negatively charged, and the dye-polymer unit may be immobilized as a complex with a cationic water-soluble polymer, wherein said complex is permeable to or in contact with the analyte solution. In one embodiment, the dye may be one of the polymeric derivatives of hydroxypyrene trisulfonic acid. The polymeric dyes may be water-soluble, water-swellable or dispersible in water. In some embodiments, the polymeric dyes may also be cross-linked. In preferred embodiments, the dye has a negative charge.

In other embodiments, the dye molecule may be covalently bonded to the water-insoluble polymer matrix M¹, wherein said M¹ is permeable to or in contact with the analyte solution. The dye molecule bonded to M¹ may form a structure M¹-L¹-Dye. L¹ is a hydrolytically stable covalent linker that covalently connects the sensing moiety to the polymer or matrix. Examples of L¹ include lower alkylene (e.g., C₁-C₈ alkylene), optionally terminated with or interrupted by one or more divalent connecting groups selected from sulfonamide (--SO₂NH--), amide --(C=O)N--, ester --(C=O)--O--, ether.--O--, sulfide --S--, sulfone (--SO₂--), phenylene --C₆H₄--, urethane --NH(C=O)--O--, urea --NH(C=O)NH--, thiourea --NH(C=S)--NH--, amide --(C=O)NH--, amine --NR-(where R is defined as alkyl having 1 to 6 carbon atoms) and the like, or a combination thereof. In one embodiment, the dye is bonded to a polymer matrix through the sulfonamide functional groups.

In one preferred embodiment, the fluorophore may be HPTS-CysMA (structure illustrated below); see U.S. Patent No. 7,417,164.

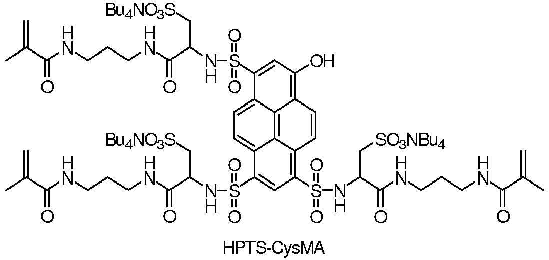

Of course, in some embodiments, substitutions other than Cys-MA on the HPTS core are consistent with aspects of the present invention, as long as the substitutions are negatively charged and have a polymerizable group. Either L or D stereoisomers of cysteine may be used. In some embodiments, only one or two of the sulfonic acids may be substituted. Likewise, in variations to HPTS-CysMA shown above, other counterions besides NBu₄⁺ may be used, including positively charged metals, e.g., Na⁺. In other variations, the sulfonic acid groups may be replaced with e.g., phosphoric, carboxylic, etc. functional groups.

Fluorescent dyes, including HPTS and its derivatives are known and many have been used in analyte detection. See e.g., U.S. Pat. Nos. 6,653,141, 6,627,177, 5,512,246, 5,137,833, 6,800,451, 6,794,195, 6,804,544, 6,002,954, 6,319,540, 6,766,183, 5,503,770, and 5,763,238.

In accordance with broad aspects of the present invention, the analyte binding moiety provides the at least dual functionality of being able to bind analyte and being able to modulate the apparent concentration of the fluorophore (e.g., detected as a change in emission signal intensity) in a manner related to the amount of analyte binding. In preferred embodiments, the analyte binding moiety is associated with a quencher. "Quencher" refers to a compound that reduces the emission of a fluorophore when in its presence. Quencher (Q) is selected from a discrete compound, a reactive intermediate which is convertible to a second discrete compound or to a polymerizable compound or Q is a pendant group or chain unit in a polymer prepared from said reactive intermediate or polymerizable compound, which polymer is water-soluble or dispersible or is an insoluble polymer, said polymer is optionally crosslinked.

In one example, the moiety that provides glucose recognition in the embodiments is an aromatic boronic acid. The boronic acid is covalently bonded to a conjugated nitrogen-containing heterocyclic aromatic bis-onium structure (e.g., a viologen). "Viologen" refers generally to compounds having the basic structure of a nitrogen containing conjugated N-substituted heterocyclic aromatic bis-onium salt, such as 2,2'-, 3,3'- or 4,4'-N,N' bis-(benzyl) bipyridium dihalide (i.e., dichloride, bromide chloride), etc. Viologen also includes the substituted phenanthroline compounds. The boronic acid substituted quencher preferably has a pKa of between about 4 and 9, and reacts reversibly with glucose in aqueous media at a pH from about 6.8 to 7.8 to form boronate esters. The extent of reaction is related to glucose concentration in the medium. Formation of a boronate ester diminishes quenching of the fluorphore by the viologen resulting in an increase in fluorescence dependent on glucose concentration. A useful bis-onium salt is compatible with the analyte solution and capable of producing a detectable change in the fluorescent emission of the dye in the presence of the analyte to be detected.

Bis-onium salts in the embodiments of this invention are prepared from conjugated heterocyclic aromatic di-nitrogen compounds. The conjugated heterocyclic aromatic di-nitrogen compounds are selected from dipyridyls, dipyridyl ethylenes, dipyridyl phenylenes, phenanthrolines, and diazafluorenes, wherein the nitrogen atoms are in a different aromatic ring and are able to form an onium salt. It is understood that all isomers of said conjugated heterocyclic aromatic di-nitrogen compounds in which both nitrogens can be substituted are useful in this invention. In one embodiment, the quencher may be one of the bis-onium salts derived from 3,3'-dipyridyl, 4,4'-dipyridyl and 4,7-phenanthroline.

In some embodiments, the viologen-boronic acid adduct may be a discrete compound having a molecular weight of about 400 daltons or greater. In other embodiments, it may also be a pendant group or a chain unit of a water-soluble or water-dispersible polymer with a molecular weight greater than about 10,000 daltons. In one embodiment, the quencher-polymer unit may be non-covalently associated with a polymer matrix and is physically immobilized therein. In yet another embodiment, the quencher-polymer unit may be immobilized as a complex with a negatively charge water-soluble polymer.

In other embodiments, the viologen-boronic acid moiety may be a pendant group or a chain unit in a crosslinked, hydrophilic polymer or hydrogel sufficiently permeable to the analyte (e.g., glucose) to allow equilibrium to be established.

In other embodiments, the quencher may be covalently bonded to a second water-insoluble polymer matrix M², which can be represented by the structure M²-L²-Q. L² is a linker selected from the group consisting of a lower alkylene (e.g., C₁-C₈ alkylene), sulfonamide, amide, quaternary ammonium, pyridinium, ester, ether, sulfide, sulfone, phenylene, urea, thiourea, urethane, amine, and a combination thereof. The quencher may be linked to M² at one or two sites in some embodiments.

In certain embodiments, at least one quencher precursor is used to attach the quenching moiety to at least one polymer. For example, aromatic groups may be used to functionalize a viologen with combinations of boronic acid groups and reactive groups. In certain embodiments, this process includes attaching an aromatic group to each of the two nitrogens in the dipyridyl core of the viologen. At least one boronic acid group, a reactive group, or a combination of the two are then attached to each aromatic group, such that the groups attached to each of the two nitrogens on the dipyridyl core of the viologen may either be the same or different. Certain combinations of the functionalized viologen quenching moiety are described as follows:
a) a first aromatic group having a pendent reactive group is attached to the first nitrogen and a second aromatic group having at least one pendent boronic group is attached to the second nitrogen;
b) one or more boronic acid groups are attached to a first aromatic group, which is attached to the first nitrogen, and one boronic acid group and a reactive group are attached to a second aromatic group, which second aromatic group is attached to the second nitrogen;
c) one boronic acid group and a reactive group are attached to a first aromatic group, which first aromatic group is attached to the first nitrogen, and one boronic acid group and a reactive group are attached to a second aromatic group, which is attached to the second nitrogen; and
d) one boronic acid group is attached to an aromatic group, which aromatic group is attached to each of the two nitrogens, and a reactive group is attached to a carbon in a heteroaromatic ring in the heteroaromatic centrally located group.

Preferred embodiments comprise two boronic acid moieties and one polymerizable group or coupling group wherein the aromatic group is a benzyl substituent bonded to the nitrogen and the boronic acid groups are attached to the benzyl ring and may be in the ortho- meta- or para- positions.

In one preferred embodiment, the quencher precursor (before incorporation into a hydrogel) may be 3,3'-oBBV (structure illustrated below); see U.S. Patent No. 7,470,420.

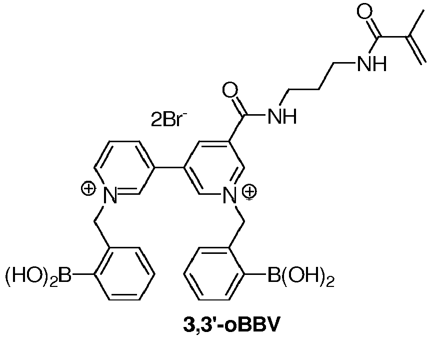

The quencher precursor 3,3'-oBBV may be used with HPTS-CysMA to make hydrogels in accordance with preferred aspects of the invention.

Other indicator chemistries, such as those disclosed in U.S. Patent Nos. 5,176,882 to Gray et al. and 5,137,833 to Russell, can also be used in accordance with embodiments of the present invention; both of which are incorporated herein in their entireties by reference thereto. In some embodiments, an indicator system may comprise an analyte binding protein operably coupled to a fluorophore, such as the indicator systems and glucose binding proteins disclosed in U.S. Patent Nos. 6,197,534, 6,227,627, 6,521,447, 6,855,556, 7,064,103, 7,316,909, 7,326,538, 7,345,160, and 7,496,392, U.S. Patent Application Publication Nos. 2003/0232383, 2005/0059097, 2005/0282225, 2009/0104714, 2008/0311675, 2008/0261255, 2007/0136825, 2007/0207498, and 2009/0048430, and PCT International Publication Nos. WO 2009/021052, WO 2009/036070, WO 2009/021026, WO 2009/021039, WO 2003/060464, and WO 2008/072338.

For *in vivo* applications, the sensor is used in a moving stream of physiological fluid which contains one or more polyhydroxyl organic compounds or is implanted in tissue such as muscle which contains said compounds. Therefore, it is preferred that none of the sensing moieties escape from the sensor assembly. Thus, for use *in vivo,* the sensing components are preferably part of an organic polymer sensing assembly. Soluble dyes and quenchers can be confined by a selectively permeable membrane that allows passage of the analyte but blocks passage of the sensing moieties. This can be realized by using as sensing moieties soluble molecules that are substantially larger than the analyte molecules (molecular weight of at least twice that of the analyte or greater than 1000 preferably greater than 5000); and employing a selective semipermeable membrane such as a dialysis or an ultrafiltration membrane with a specific molecular weight cutoff between the two so that the sensing moieties are quantitatively retained.

Preferably the sensing moieties are immobilized in an insoluble polymer matrix, which is freely permeable to glucose. The polymer matrix is comprised of organic, inorganic or combinations of polymers thereof. The matrix may be composed of biocompatible materials. Alternatively, the matrix is coated with a second biocompatible polymer that is permeable to the analytes of interest.

The function of the polymer matrix is to hold together and immobilize the fluorophore and quencher moieties while at the same time allowing contact with the analyte, and binding of the analyte to the boronic acid. To achieve this effect, the matrix must be insoluble in the medium, and in close association with it by establishing a high surface area interface between matrix and analyte solution. For example, an ultra-thin film or microporous support matrix is used. Alternatively, the matrix is swellable in the analyte solution, e.g. a hydrogel matrix is used for aqueous systems. In some instances, the sensing polymers are bonded to a surface such as the surface of a light conduit, or impregnated in a microporous membrane. In all cases, the matrix must not interfere with transport of the analyte to the binding sites so that equilibrium can be established between the two phases. Techniques for preparing ultra-thin films, microporous polymers, microporous sol-gels, and hydrogels are established in the art. All useful matrices are defined as being analyte permeable.

Hydrogel polymers are used in some embodiments. The term, hydrogel, as used herein refers to a polymer that swells substantially, but does not dissolve in water. Such hydrogels may be linear, branched, or network polymers, or polyelectrolyte complexes, with the proviso that they contain no soluble or leachable fractions. Typically, hydrogel networks are prepared by a crosslinking step, which is performed on water-soluble polymers so that they swell but do not dissolve in aqueous media. Alternatively, the hydrogel polymers are prepared by copolymerizing a mixture of hydrophilic and crosslinking monomers to obtain a water swellable network polymer. Such polymers are formed either by addition or condensation polymerization, or by combination process. In these cases, the sensing moieties are incorporated into the polymer by copolymerization using monomeric derivatives in combination with network-forming monomers. Alternatively, reactive moieties are coupled to an already prepared matrix using a post polymerization reaction. Said sensing moieties are units in the polymer chain or pendant groups attached to the chain.

The hydrogels useful in this invention are also monolithic polymers, such as a single network to which both dye and quencher are covalently bonded, or multi-component hydrogels. Multi-component hydrogels include interpenetrating networks, polyelectrolyte complexes, and various other blends of two or more polymers to obtain a water swellable composite, which includes dispersions of a second polymer in a hydrogel matrix and alternating microlayer assemblies.

Monolithic hydrogels are typically formed by free radical copolymerization of a mixture of hydrophilic monomers, including but not limited to HEMA, PEGMA, methacrylic acid, hydroxyethyl acrylate, N-vinyl pyrrolidone, acrylamide, N,N'-dimethyl acrylamide, and the like; ionic monomers include methacryloylaminopropyl trimethylammonium chloride, diallyl dimethyl ammonium. chloride, vinyl benzyl trimethyl ammonium chloride, sodium sulfopropyl methacrylate, and the like; crosslinkers include ethylene dimethacrylate, PEGDMA, trimethylolpropane triacrylate, and the like. The ratios of monomers are chosen to optimize network properties including permeability, swelling index, and gel strength using principles well established in the art. In one embodiment, the dye moiety is derived from an ethylenically unsaturated derivative of a dye molecule, such as 8-acetoxypyrene-1,3,6-N, N', N"-tris(methacrylamidopropylsulfonamide), the quencher moiety is derived from an ethylenically unsaturated viologen such as 4-N-(benzyl-3-boronic acid)-4'-N'-(benzyl-4ethenyl)-dipyridinium dihalide (m-SBBV) and the matrix is made from HEMA and PEGDMA. The concentration of dye is chosen to optimize emission intensity. The ratio of quencher to dye is adjusted to provide sufficient quenching to produce the desired measurable signal.

In some embodiments, a monolithic hydrogel is formed by a condensation polymerization. For example, acetoxy pyrene trisulfonyl chloride is reacted with an excess of PEG diamine to obtain a tris-(amino PEG) adduct dissolved in the unreacted diamine. A solution of excess trimesoyl chloride and an acid acceptor is reacted with 4-N-(benzyl-3-boronic acid)-4'-N'-(2 hydroxyethyl) bipyridinium dihalide to obtain an acid chloride functional ester of the viologen. The two reactive mixtures are brought into contact with each other and allowed to react to form the hydrogel, e.g. by casting a thin film of one mixture and dipping it into the other.

In other embodiments, multi-component hydrogels wherein the dye is incorporated in one component and the quencher in another are preferred for making the sensor. Further, these systems are optionally molecularly imprinted to enhance interaction between components and to provide selectivity for glucose over other polyhydroxy analytes. Preferably, the multicomponent system is an interpenetrating polymer network (IPN) or a semi-interpenetrating polymer network (semi-IPN).

The IPN polymers are typically made by sequential polymerization. First, a network comprising the quencher is formed. The network is then swollen with a mixture of monomers including the dye monomer and a second polymerization is carried out to obtain the IPN hydrogel.

The semi-IPN hydrogel is formed by dissolving a soluble polymer containing dye moieties in a mixture of monomers including a quencher monomer and through complex formation with the fluorophore. In some embodiments, the sensing moieties are immobilized by an insoluble polymer matrix which is freely permeable to polyhydroxyl compounds. Additional details on hydrogel systems have been disclosed in US Patent Publications Nos. US2004/0028612, and 2006/0083688.

The polymer matrix is comprised of organic, inorganic or combinations of polymers thereof. The matrix may be composed of biocompatible materials. Alternatively, the matrix is coated with a second biocompatible polymer that is permeable to the analytes of interest. The function of the polymer matrix is to hold together and immobilize the fluorescent dye and quencher moieties while at the same time allowing contact with the analytes (e.g., polyhydroxyl compounds, H⁺ and OH⁻), and binding of the polyhydroxyl compounds to the boronic acid. Therefore, the matrix is insoluble in the medium and in close association with it by establishing a high surface area interface between matrix and analyte solution. The matrix also does not interfere with transport of the analyte to the binding sites so that equilibrium can be established between the two phases. In one embodiment, an ultra-thin film or microporous support matrix may be used. In another embodiment, the matrix that is swellable in the analyte solution (e.g. a hydrogel matrix) can be used for aqueous systems. In some embodiments, the sensing polymers are bonded to a surface such as the surface of a light conduit, or impregnated in a microporous membrane. Techniques for preparing ultra-thin films, microporous polymers, microporous sol-gels, and hydrogels have been established in the prior art.

In one preferred embodiment, the boronic acid substituted viologen may be covalently bonded to a fluorescent dye. The adduct may be a polymerizable compound or a unit in a polymer. One such adduct for example may be prepared by first forming an unsymmetrical viologen from 4,4'-dipyridyl by attaching a benzyl-3-boronic acid group to one nitrogen and an aminoethyl group to the other nitrogen atom. The viologen is condensed sequentially first with 8-acetoxy-pyrene-1,3,6-trisulfonyl chloride in a 1:1 mole ratio followed by reaction with excess PEG diamine to obtain a prepolymer mixture. An acid acceptor is included in both steps to scavange the byproduct acid. The prepolymer mixture is crosslinked by reaction with a polyisocyanate to obtain a hydrogel. The product is treated with base to remove the acetoxy blocking group. Incomplete reaction products and unreacted starting materials are leached out of the hydrogel by exhaustive extraction with deionized water before further use. The product is responsive to glucose when used as the sensing component as described herein.

Alternatively, such adducts are ethylenically unsaturated monomer derivatives. For example, dimethyl bis-bromomethyl benzene boronate is reacted with excess 4,4'-dipyridyl to form a half viologen adduct. After removing the excess dipyridyl, the adduct is further reacted with an excess of bromoethylamine hydrochloride to form the bis-viologen adduct. This adduct is coupled to a pyranine dye by reaction with the 8-acetoxypyrene-tris sulfonyl chloride in a 1:1 mole ratio in the presence of an acid acceptor followed by reaction with excess aminopropylmethacrylamide. Finally, any residual amino groups may be reacted with methacrylol chloride. After purification, the dye/viologen monomer may be copolymerized with HEMA and PEGDMA to obtain a hydrogel.

### Solution Example

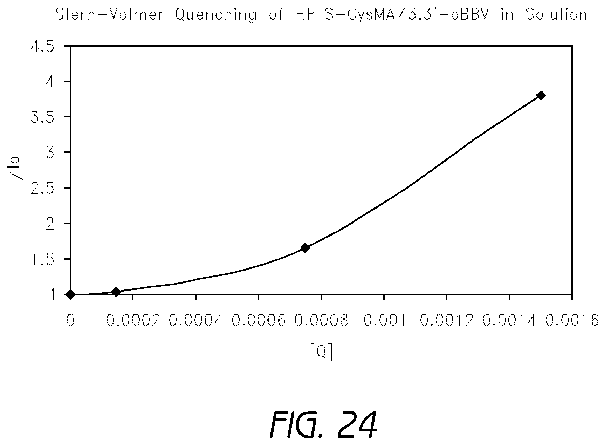

To a solution of HPTS-CysMA (1×10⁻⁵ M in pH 7.4 PBS) was added increasing amounts of 3,3'-oBBV (30 mM in MeOH) and the fluorescence emission measured after each addition. **FIG. 24** gives the relative emission change (Stern-Volmer curve) upon addition of 3,3'-oBBV (Q) indicating the quenching of HPTS-CysMA with 3,3'-oBBV. The fluorimeter settings were as follows: 1% attenuation, ex slit 8 nm, em slit 12 nm, 486 nm ex λ, 537 nm em λ.

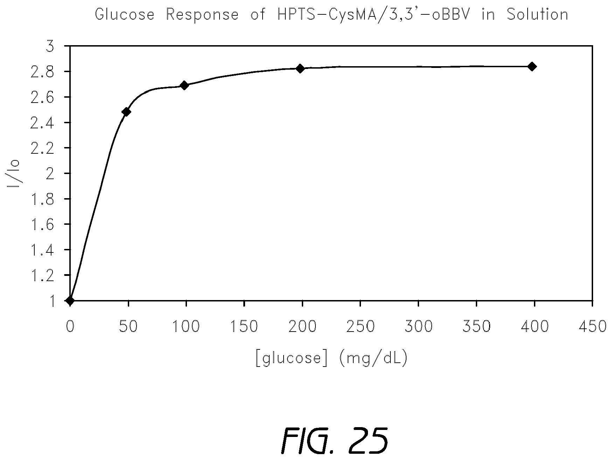

HPTS-CysMA (1 × 10-5 M) and 3,3'-oBBV (3 × 10-3 M) were titrated with a stock solution of glucose (31250 mg/dL) in pH 7.4 PBS and the fluorescence emission measured after each addition of glucose. The relative change upon addition of glucose is given in **FIG. 25****.**

### Hydrogel Example

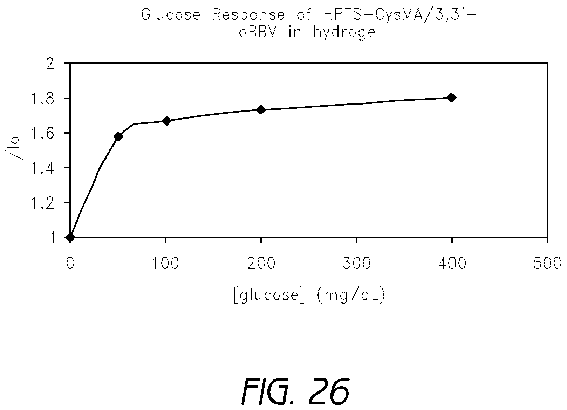

HPTS-CysMA (2 mg), 3,3'-oBBV (15 mg), N,N'-dimethylacrylamide (400 mg), N,N'-methylenebisacrylamide (8 mg), HCl (10 µL of 1 M solution), and VA-044 (1 mg) were dissolved in water and diluted to 1 mL in a volumetric flask. The solution was freeze-pump-thawed (3×), injected into a mold containing a 0.005" polyimide spacer and polymerized at 55 °C for 16 h. The resultant film was placed in pH 7.4 phosphate buffer and was tested in a flow cell configuration with increasing amounts of glucose (0, 50, 100, 200, 400 mg/dL). The relative fluorescence change upon addition of glucose is given in **FIG. 26****.** The fluorimeter settings were as follows: ex slit 8 nm, em slit 3.5 nm, 515 nm cutoff filter, 486 nm ex λ, 532 nm em λ.

### Sterilization and RFID Programming Example

A glucose sensor assembly with an optical fiber and a fluorescent based chemical sensor placed at the tip of the optical fiber is manufactured according to one of thee methods described above. Each glucose sensor also has electrical components such as a memory coupled to an antenna and electrical cables. A sample of glucose sensor assemblies is put through a simulation test to determine parameters and coefficients related to the chemical sensor operation. Each glucose sensor assembly is quality tested for basic functionality and after passing the quality testing, each glucose sensor assembly is placed into plastic tray that secures the glucose sensor assembly. Other components that the user can use, such as the calibration chamber and syringe are also placed into the plastic tray. Each plastic tray is placed into a Tyvek® breathable pouch and sealed. The remaining glucose sensor assemblies are packaged into the plastic tray and pouch similarly. The packages of glucose sensor assemblies are organized for sterilization in batches.

Several batches of packaged glucose sensor assemblies are sterilized by ethylene oxide. One batch is sterilized by ethylene oxide by a nominal amount. Another batch is sterilized by a higher level of ethylene oxide, such as longer exposure, higher concentration, and/or higher temperature. Anther batch is sterilized by a lower level of ethylene oxide.

Several other batches of packaged glucose sensor assemblies are sterilized by gamma irradiation. One batch is sterilized by a nominal amount of gamma irradiation. Another batch is sterilized by a higher amount of gamma irradiation. Anther batch is sterilized by a lower amount of gamma irradiation. Further batches are also sterilized by different methods like electron beam, x-ray, steam, and dry heat, each run at different parameters.

A number of samples are taken for each batch of sterilized glucose sensor assemblies. The sampled glucose sensor assemblies are taken out of the packaging. The samples are put through a through a simulation test to determine parameters and coefficients related to the chemical sensor operation. The determined parameters and coefficients, such as the calibration parameters, are compared to each other, and to the sample of sensor assemblies that are tested prior to packaging/sterilization.

A comparison of the data will show that the parameters and coefficients related to the chemical sensor operation will differ between pre-sterilization and post-sterilization. They will also differ based on the sterilization method used, such as ethylene oxide, gamma, electron beam, x-ray, steam, dry heat. They will also differ based on the sterilization parameter, such as the irradiation amount or processing temperature.

After determining the calibration coefficients for each batch, the calibration coefficients are stored to a programming module along with other information such as lot numbers and expiration information. The remaining sterilized sensor assemblies that are still in their packaging are placed into a programming operation line, where each sterilized sensor assembly is programmed. An RFID system is used where a programmer with an antenna is connected to the programming module. Radio frequency is communicated from the antenna connected to the programming module to the antenna connected to the memory of the sensor assembly. The radio frequency powers the memory and communicates the information to the memory. The packaging is not opened at all during programming, thus maintaining the sterility of the sensor.

When the sensor is used in clinical settings, the package is opened and the sensor's electrical wire is connected to a monitor system. When the electrical wire is connected and the monitor is configured to initiate the calibration process or operation, the monitor acquires the lot and expiration information, or other use information. The monitor then determines whether the sensor may be used or not, by verifying the expiration date has not passed. The monitor also reads the calibration parameters from the sensor's memory and executes the calibration operation. After calibration of the sensor, it is placed into the subject for continuous real-time glucose monitoring. Because the calibration parameters are determined with the effect from the sterilization factored in, the sensor accurately measures the patient's glucose level. Thus, the clinician is able to monitor the subject's glucose level more accurately.

Although the foregoing invention has been described in terms of certain embodiments and examples, other embodiments will be apparent to those of ordinary skill in the art from the disclosure herein. Moreover, the described embodiments have been presented by way of example only, and the extent of protection is covered by the scope of claims.

## Claims

1. A method of manufacturing a sterilized optical analyte sensor comprising an optical fiber sensor with a chemical indicator system, a non-volatile memory, and a RFID element, the method comprising:
providing a batch of optical analyte sensors;
packaging each optical analyte sensor in a sealed package for sterilization;
receiving the batch of optical analyte sensors that have been sterilized;
testing several samples of the optical analyte sensors from the batch of optical analyte sensors;
calculating parameters representative of the functionality of the batch of optical analyte sensors; and
programming the optical analyte sensors that have not been tested,
wherein programming the optical analyte sensors comprises:
powering the non-volatile memory by applying radio frequency to the RFID element, and
writing the parameters representative of the functionality to the non-volatile memory.

2. The method of Claim 1, wherein powering the non-volatile memory and writing the parameters representative of the functionality to the non-volatile memory is done without opening the sealed package for sterilization.

3. The method of any one of Claims 1 and 2, wherein packaging each optical analyte sensor in a sealed package for sterilization further comprises placing each optical analyte sensor in a plastic tray comprising compartments configured to accommodate the optical analyte sensor.

4. The method of any one of Claims 1 - 3, wherein testing several samples of the optical analyte sensors comprises:
contacting the optical analyte sensor with a first calibration buffer;
generating a signal indicative of a first analyte concentration of the first calibration buffer; and
generating a first signal indicative of a first temperature of the first calibration buffer.

5. The method of Claim 4, wherein calculating parameters representative of the functionality comprises:
transforming the signal indicative of the first analyte concentration using a modified Michaelis-Menten equation comprising Michaelis-Menten parameters, wherein the Michaelis-Menten parameters are set based on data comprising:
temperature calibration data; and
the first signal indicative of a first temperature.

6. The method of any one of Claims 4 and 5, wherein testing several samples of the optical analyte sensors further comprises:
contacting the optical analyte sensor with the a second calibration buffer;
generating a second signal indicative of a second analyte concentration of the second calibration buffer;
generating a second signal indicative of a second temperature of the second calibration buffer.

7. The method of any one of Claims 1 - 6, wherein programming the optical analyte sensors further comprises checking whether the non-volatile memory is operational.

## Patentansprüche

1. Verfahren zur Herstellung eines sterilisierten optischen Analytsensors, der einen optischen Fasersensor mit einem chemischen Indikatorsystem, einem nichtflüchtigen Speicher, und einem RFID-Element umfasst, wobei das Verfahren Folgendes umfasst:
Bereitstellen einer Charge optischer Analytsensoren;
Verpacken jedes optischen Analytsensors in einer versiegelten Verpackung zur Sterilisation;
Empfangen der Charge optischer Analytsensoren, die bereits sterilisiert wurden;
Testen mehrerer Proben der optischen Analytsensoren aus der Charge optischer Analytsensoren;
Berechnen von Parametern, die für die Funktionalität der Charge optischer Analytsensoren repräsentativ sind; und
Programmierung der optischen Analytsensoren, die nicht getestet wurden, wobei die Programmierung der optischen Analytsensoren Folgendes umfasst:
Anregen des nichtflüchtigen Speichers durch Anlegen von Radiofrequenz an das RFID-Element; und
Beschreiben des nichtflüchtigen Speichers mit den Parametern, die für die Funktionalität repräsentativ sind.

2. Verfahren nach Anspruch 1, wobei das Anregen des nichtflüchtigen Speichers und das Beschreiben des nichtflüchtigen Speichers mit den Parametern, die für die Funktionalität repräsentativ sind, ohne das Öffnen der zur Sterilisierung versiegelten Verpackung geschieht.

3. Verfahren nach einem der Ansprüche 1 und 2, wobei das Verpacken jedes optischen Analytsensors in einer zur Sterilisierung versiegelten Verpackung ferner das Anordnen jedes optischen Analytsensors in einer Kunststoffschale umfasst, die Kammern umfasst, die konfiguriert sind, um den optischen Analytsensor aufzunehmen.

4. Verfahren nach einem der Ansprüche 1 bis 3, wobei das Testen verschiedener Proben des optischen Analytsensors Folgendes umfasst:
Inkontaktbringen des optischen Analytsensors mit einem ersten Kalibrierungspuffer;
Erzeugen eines Signals, das eine erste Analytkonzentration des ersten Kalibrierungspuffers anzeigt; und
Erzeugen eines ersten Signals, das eine erste Temperatur des ersten Kalibrierungspuffers anzeigt.

5. Verfahren nach Anspruch 4, wobei die Berechnung der Parameter, die für die Funktionalität repräsentativ sind, Folgendes umfasst:
Transformieren des Signals, welches die erste Analytkonzentration anzeigt, unter Verwendung einer modifizierten Michaelis-Menten-Gleichung, die Michaelis-Menten-Parameter umfasst, wobei die Michaelis-Menten-Parameter basierend auf Daten eingestellt werden, die Folgendes umfassen:
Temperaturkalibrierungsdaten; und
das erste Signal, das eine erste Temperatur anzeigt.

6. Verfahren nach einem der Ansprüche 4 und 5, wobei das Testen mehrerer Proben des optischen Analytsensors ferner Folgendes umfasst:
Inkontaktbringen des optischen Analytsensors mit dem einen zweiten Kalibrierungspuffer;
Erzeugen eines zweiten Signals, das eine zweite Analytkonzentration des zweiten Kalibrierungspuffers, anzeigt;
Erzeugen eines zweiten Signals, das eine zweite Temperatur des zweiten Kalibrierungspuffers anzeigt.

7. Verfahren nach einem der Ansprüche 1 bis 6, wobei die Programmierung der optischen Analytsensoren ferner das Testen, ob der nichtflüchtige Speicher betriebsbereit ist, umfasst.

## Revendications

1. Procédé de fabrication d'un capteur d'analytes optique stérilisé comprenant un capteur à fibre optique avec un système indicateur chimique, une mémoire non volatile, et un élément RFID, le procédé comprenant :
la fourniture d'un lot de capteurs d'analytes optiques ;
l'emballage de chaque capteur d'analytes optique dans un emballage scellé pour stérilisation ;
la réception du lot de capteurs d'analytes optiques qui ont été stérilisés ;
le test de plusieurs échantillons des capteurs d'analytes optiques parmi le lot de capteurs d'analytes optiques ;
le calcul des paramètres représentatifs de la fonctionnalité du lot de capteurs d'analytes optiques ; et
la programmation des capteurs d'analytes optiques qui n'ont pas été testés, dans laquelle la programmation des capteurs d'analytes optiques comprend :
l'alimentation de la mémoire non volatile en appliquant une radiofréquence à l'élément RFID, et
l'écriture des paramètres représentatifs de la fonctionnalité dans la mémoire non volatile.

2. Procédé selon la revendication 1, dans lequel l'alimentation de la mémoire non volatile et l'écriture des paramètres représentatifs de la fonctionnalité dans la mémoire non volatile sont effectuées sans ouvrir l'emballage scellé pour stérilisation.

3. Procédé selon l'une quelconque des revendications 1 et 2, dans lequel l'emballage de chaque capteur d'analytes optique dans un emballage scellé pour stérilisation comprend en outre le placement de chaque capteur d'analytes optique dans un plateau en plastique comprenant des compartiments configurés pour recevoir le capteur d'analytes optique.

4. Procédé selon l'une quelconque des revendications 1 à 3, dans lequel le test de plusieurs échantillons des capteurs d'analytes optiques comprend :
la mise en contact du capteur d'analytes optique avec un premier tampon d'étalonnage ;
la génération d'un signal indicatif d'une première concentration d'analytes du premier tampon d'étalonnage ; et
la génération d'un premier signal indicatif d'une première température du premier tampon d'étalonnage.

5. Procédé selon la revendication 4, dans lequel le calcul de paramètres représentatifs de la fonctionnalité comprend :
la transformation du signal indicatif de la première concentration d'analytes en utilisant une équation de Michaelis-Menten modifiée comprenant des paramètres de Michaelis-Menten, dans laquelle les paramètres de Michaelis-Menten sont réglés en fonction de données comprenant :
des données d'étalonnage de la température ; et
le premier signal indicatif d'une première température.

6. Procédé selon l'une quelconque des revendications 4 et 5, dans lequel le test de plusieurs échantillons des capteurs d'analytes optiques comprend en outre :
la mise en contact du capteur d'analytes optique avec un deuxième tampon d'étalonnage ;
la génération d'un deuxième signal indicatif d'une seconde concentration d'analytes du deuxième tampon d'étalonnage ;
la génération d'un deuxième signal indicatif d'une deuxième température du deuxième tampon d'étalonnage.

7. Procédé selon l'une quelconque des revendications 1 à 6, dans lequel la programmation des capteurs d'analytes optiques comprend en outre l'étape visant à vérifier si la mémoire non volatile est opérationnelle.
